(19) Europäisches Patentamt
European Patent Office
Office européen des brevets

(11) Publication number : 0 562 849 A2

(12) # EUROPEAN PATENT APPLICATION

(21) Application number : 93302261.8

(22) Date of filing : 24.03.93

(51) Int. Cl.$^5$ : **C07J 9/00,** C07J 41/00, C07C 21/00, C07J 1/00, C07J 51/00, C07J 17/00, C07J 3/00, C07J 43/00, A61K 31/56

(30) Priority : 27.03.92 US 858908
03.03.93 US 18985

(43) Date of publication of application :
29.09.93 Bulletin 93/39

(84) Designated Contracting States :
AT BE CH DE DK ES FR GB GR IE IT LI LU NL PT SE

(71) Applicant : ELI LILLY AND COMPANY
Lilly Corporate Center
Indianapolis Indiana 46285 (US)

(72) Inventor : Archer, Robert Allen
5563 North Pennsylvania
Indianapolis, Indiana 46220 (US)
Inventor : Beavers, Lisa Selsam
R. R. 1, Box 56
Trafalgar, Indiana 46181 (US)
Inventor : Gadski, Robert Alan
4431 North Illinois Street
Indianapolis, Indiana 46208 (US)
Inventor : Lin, Ho-Shen
8128 Trevellian Way
Indianapolis, Indiana 46217 (US)
Inventor : McClure, Don B.
7816 Mallard Way
Indianapolis, Indiana 46256 (US)
Inventor : McCowan, Jefferson Ray
2653 Crescent Hill Lane
Indianapolis, Indiana 46208 (US)
Inventor : Pawlak, Joseph Matthew
3607 Ivory Way
Indianapolis, Indiana 46227 (US)
Inventor : Rampersaud, Ashraff Ali
8375 North Central Avenue
Indianapolis, Indiana (US)
Inventor : Schmidt, Robert John
8453 Slippery Elm Court
Indianapolis, Indiana 46227 (US)
Inventor : Schreyer, Teri Ann
373 Measowlark Lane
Batesville, Indiana 47006 (US)
Inventor : Yu, Melvin Joseph
400 Brookside Drive
Andover, Massachusetts (US)

(74) Representative : Hudson, Christopher Mark et al
Erl Wood Manor
Windlesham Surrey GU20 6PH (GB)

(54) **Steroid derivatives.**

(57) This invention relates to steroid derivatives, pharmaceutical formulations containing those compounds alone or together with other cholesterol control agents, and methods of their use for upregulating LDL receptor gene expression, lowering serum LDL cholesterol and/or inhibiting atherosclerosis. Said steroid derivatives have the formula :

EP 0 562 849 A2

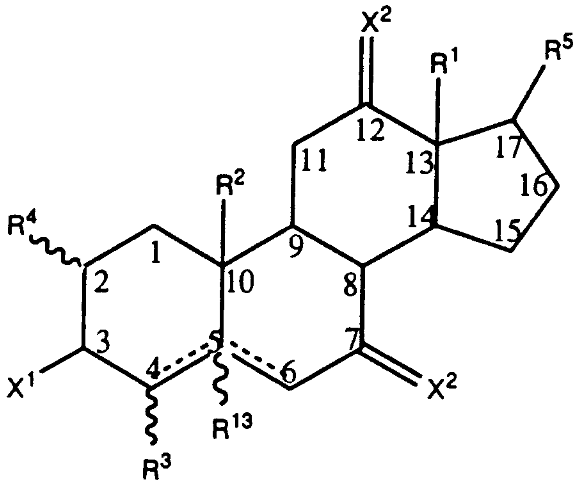

I

The present invention relates to steroid derivatives, to pharmaceutical compositions containing those compounds and to methods of their use.

All mammalian cells require cholesterol as a structural component of their cell membranes and for non-sterol end products. Cholesterol is also required for steroid hormone synthesis. The very property, however, that makes cholesterol useful in the cell membranes, its insolubility in water, also makes it potentially lethal. When cholesterol accumulates in the wrong place, for example within the wall of an artery, it cannot be readily mobilized and its presence leads to the development of an atherosclerotic plaque. Elevated concentrations of serum cholesterol associated with low density lipoproteins have been demonstrated to be a major contributing factor in the development and progression of atherosclerosis.

In mammals, serum lipoprotein is composed of cholesterol together with cholesteryl esters, triglycerides, phospholipids and apoproteins. Serum or plasma lipoprotein is comprised of several fractions. The major fractions or classes of plasma lipoproteins are very low density lipoprotein (VLDL),intermediate density lipoprotein (IDL), low density lipoprotein (LDL), and high density lipoprotein (HDL). These classes differ from one another in size and density in the relative proportions of triglycerides and cholesteryl esters in the core, and in the nature of the apoproteins on the surface.

In mammals, serum cholesterol is derived from exogenous dietary sources as well as through endogenous synthesis. Endogenous synthesis of cholesterol involves a complex set of enzyme-catalyzed reactions and regulatory mechanisms generally termed the mevalonate pathway. Cells face a complex problem in regulating mevalonate synthesis because cholesterol, the bulk end product of mevalonate metabolism, is derived from plasma low density lipoprotein which enters the cell by receptor-mediated endocytosis, as well as from synthesis within the cell. Each cell must balance these external and internal sources so as to sustain mevalonate synthesis while avoiding sterol over accumulation. This balance is achieved through feedback regulation of at least two sequential enzymes in mevalonate synthesis, 3-hydroxy-3-methylglutaryl coenzyme A (HMG-CoA) synthase and HMG-CoA reductase and also of LDL receptors. In the absence of LDL, mammalian cells maintain high activities of the two enzymes, thereby synthesizing mevalonate for production of cholesterol as well as the non-sterol products. When LDL is present, from exogenous sources, HMG-CoA synthase and reductase activity is repressed and the cells produce smaller amounts of mevalonate for the non-sterol end products.

Abundant evidence indicates that treatment of hyperlipoproteinemia will diminish or prevent atherosclerotic complications. In addition to a diet that maintains a normal body weight and minimizes concentrations of lipids in plasma, therapeutic strategies include elimination of factors that exacerbate hyperlipoproteinemia and the administration of therapeutic agents that lower plasma concentrations of lipoproteins, either by diminishing the production of lipoproteins or by enhancing the efficiency of their removal from plasma.

Presently there are no cholesterol lowering drugs which are known to operate at the level of gene expression.

The most promising class of drugs currently available for the treatment of hypercholesterolemia act by inhibiting HMG-CoA reductase, the rate-limiting enzyme of endogenous cholesterol synthesis. Drugs of this class competitively inhibit the activity of the enzyme. Eventually, this lowers the endogenous synthesis of cholesterol and, by normal homeostatic mechanisms, plasma cholesterol is taken up by LDL receptors to restore the intracellular cholesterol balance.

Relative to other cells in the body, liver cells play a critical role in maintaining serum cholesterol homeostasis by both releasing precursors of LDL and through receptor mediated LDL uptake from the serum. In both man and animal models an inverse correlation appears to exist between liver LDL receptors and LDL associated serum cholesterol levels. In general, higher hepatocyte receptor numbers result in lower LDL associated serum cholesterol levels. Cholesterol released into hepatocytes can be stored as cholesteryl esters, converted into bile acids and released into the bile duct, or enter into an oxycholesterol pool. It is this oxycholesterol pool that is believed to be involved in end product repression of both the genes of the LDL receptor and enzymes involved in the cholesterol synthetic pathway.

Transcription of the LDL receptor gene is known to be repressed when cells have an excess supply of cholesterol, probably in the form of oxycholesterol. A DNA sequence in the LDL receptor promoter region, known as the sterol response element, appears to confer this sterol end product repression. This element has been extensively studied (Brown, Goldstein and Russell, U.S. Patent Nos. 4,745,060 and 4,935,363) and appears to consist of a 16 base pair sequence that occurs 5' of the LDL receptor coding region. The sterol response element can be inserted into genes that normally do not respond to cholesterol, conferring sterol end product repression on the chimeric gene. The exact mechanism of this repression is not understood. There is, however, abundant evidence that polar intermediates in cholesterol biosynthesis and naturally occurring as well as synthetic hydroxysterols repress genes containing the sterol response element.

It is postulated that the number of LDL receptors synthesized by a cell is regulated by the amount of cholesterol in the cell. It has been suggested that a hydroxycholesterol binding protein serves as a receptor. When

the receptor is bound to an oxysterol it acts on the sterol response element to control transcription through a mechanism that is similar to the action of members of the steroid hormone receptor super gene family. Brown and Goldstein have disclosed methods for employing the sterol response element in a screen assay for drugs capable of stimulating cells to synthesize LDL receptors (U.S. Patent No. 4,935,363). The present invention describes methods and compounds that act to inhibit directly or indirectly the end product repression of the LDL receptor gene by interfering with the interaction of hydroxycholesterol receptors with its natural ligands. The interference can be either in the form of an antagonist or of a molecule that directly or indirectly lowers the concentration or availability of ligands involved in end product repression of the LDL receptor. This should result in induction of the LDL receptor on the surface of liver cells, facilitating LDL uptake, bile acid synthesis and secretion to remove cholesterol metabolites and the lowering of LDL associated serum cholesterol levels.

It would be most desirable if the synthesis of LDL receptors could be upregulated at the chromosomal level. The upregulation of LDL receptor synthesis at the chromosomal level offers the promise of resetting the level of serum cholesterol at a lower, and clinically more desirable, level.

Accordingly, it is one object of the present invention to provide compounds which directly or indirectly upregulate LDL receptor synthesis at the chromosomal level and are useful in the treatment of hypercholesterolemia.

A further object of the present invention is to provide therapeutic compositions for treating said condition.

Still further objects are to provide methods for upregulating LDL receptor synthesis, for lowering serum LDL cholesterol levels, and for inhibiting atherosclerosis.

Other objects, features and advantages will become apparent to those skilled in the art from the following description and claims.

The present invention provides novel steroid derivatives which upregulate LDL receptor gene expression.

More particularly, this invention relates to compounds having the formula (I) and pharmaceutically acceptable salts thereof;

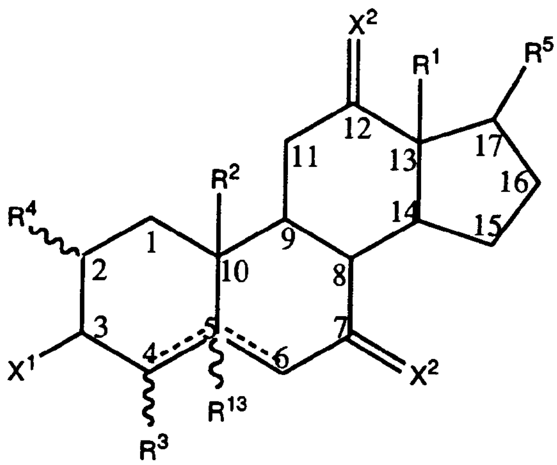

I

wherein the groups $R^1$, $R^5$, $X^2$, $R^{13}$, $R^3$, $X^1$, $R^4$, and $R^2$ are as hereinafter defined.

This invention also provides pharmaceutical compositions which comprise a compound of Formula (I) in association with a pharmaceutically acceptable carrier, diluent, or excipient.

This invention is also a multi-mode pharmaceutical composition comprising a compound of formula (I) together with other cholesterol control agents.

This invention is also an improved method of controlling cholesterol in a mammal by administering both the compounds of this invention and other cholesterol control agents.

A further embodiment of the present invention is a method for upregulating LDL receptor gene expression in mammals.

A further embodiment is a method for treating hypercholesterolemia in mammals.

A further embodiment is a method of inhibiting atherosclerosis.

These methods comprise administering to a mammal in need of LDL receptor upregulation, reduced serum LDL cholesterol levels, or atherosclerosis inhibition, an LDL receptor gene expression upregulating dose, a serum cholesterol lowering dose, or an atherosclerosis inhibiting dose, of a compound of Formula (I).

The term "alkyl" by itself or as part of another substituent means, unless otherwise stated, a straight or branched chain hydrocarbon radical having the stated number of carbon atoms and includes straight or branch chain groups such as methyl, ethyl, n-propyl, isopropyl, n-butyl, t-butyl, isobutyl, sec-butyl, and where indicated, higher homologs and isomers such as n-pentyl, n-hexyl, 2-methylpentyl, 1,5-dimethylhexyl, 1-methyl-4-isopropyl hexyl and the like. A divalent radical derived from an alkane is exemplified by $-CH_2CH_2CH_2CH_2-$. A divalent radical derived from an alkene is exemplified by $-CH=CH-CH_2-$. The term "amino" means a group $-NH_2$. The term, "substituted amino" means an amino group where one or both amino hydrogens are independently replaced by a $C_1$-$C_4$ alkyl, $C_2$-$C_4$ alkenyl, independently replaced by a $C_1$-$C_4$ alkyl, $C_2$-$C_4$ alkenyl, halo, $C_1$-$C_4$ alkoxy, -OH, -SH, or -S($C_1$-$C_4$ alkyl) group. The term "halo" means chloro, bromo, fluoro or iodo. The term "mercapto" means a group -SH. The term "acetamido" means a group $CH_3C(O)NH$-. The term "alkenyl", employed alone or in combination with other terms, means a straight chain or branched monounsaturated hydrocarbon group having the stated number of carbon atoms, such vinyl, propenyl (allyl), crotyl, isopentenyl, and the various butenyl isomers, and where indicated, higher homologs and isomers. The term "cycloalkenyl" means an unsubstituted or substituted monovalent monounsaturated cyclic hydrocarbon radical having the stated number of carbon atoms, including, various isomers of cyclopentenyl and cyclohexenyl. The substituents can be one or two of the same or different substituents selected from halo, hydroxy, cyano, mercapto, -S($C_1$-$C_4$ alkyl), amino, substituted amino, acetamido, carboxy, trifluoromethyl, $C_1$-$C_4$ alkoxy, ($C_1$-$C_4$ alkoxy)carbonyl and aminocarbonyl. The term "cycloalkadienyl" means a monovalent diunsaturated cyclic radical having the stated number of carbon atoms, including, the various isomers of cyclopentadienyl and cyclohexadienyl.

The dotted lines between the 4,5 and 5,6 positions represent the presence or absence of an additional bond; that is, an unsaturation. Only one unsaturation can be present at any one time. The 5-position hydrogen atom shown in Formula (I) as $R^{13}$ will, of course, be absent when an unsaturation is present.

The phenyl ring forming part of the benzyl group attached to the 4 position of the steroid ring is substituted in a manner represented by the formula:

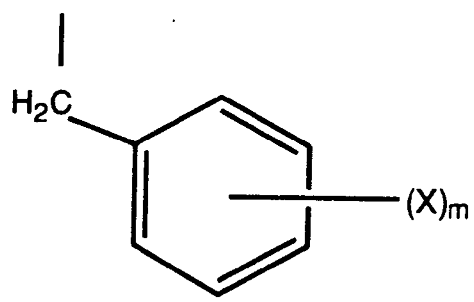

where X is a group selected from hydrogen, hydroxy, -SH, -S($C_1$-$C_4$ alkyl), hydroxyalkyl, halo, -COOH, ester, alkoxy, acetamido, alkoxyalkyl, alkoxyaryl, and wherein m is 1 to 3, with at least one X para to the carbon atom attached to the steroid nucleus.

Preferably the substitution is by a single X group (where m=1).

The term "heterocycle" means an unsubstituted or substituted stable 5- or 6-membered monocyclic heterocyclic ring which consists of carbon atoms and from one to three heteroatoms selected from the group consisting of N, O, S, and wherein the nitrogen and sulfur heteroatoms may optionally be oxidized, and the nitrogen heteroatom may optionally be quaternized. The heterocyclic ring may be attached, unless otherwise stated, at any heteroatom or carbon atom which affords a stable structure. The heterocycle is unsubstituted or substituted with 1 or 2 substituents independently selected from halo, -OH, -SH, -S($C_1$-$C_4$) alkyl), $C_1$-$C_5$ alkyl, $C_1$-$C_5$ alkoxy, carboxy, ($C_1$-$C_4$ alkoxy)carbonyl, aminocarbonyl, $C_1$-$C_4$ alkylaminocarbonyl, amino, acetamido, $C_1$-$C_4$ alkylamino, di($C_1$-$C_4$ alkyl)amino or a group $-(CH_2)_q$-R where q is 1, 2, 3, or 4 and R is hydroxy, $C_1$-$C_4$ alkoxy, carboxy, $C_1$-$C_4$ alkoxycarbonyl, amino, aminocarbonyl, $C_1$-$C_4$ alkylamino or di($C_1$-$C_4$alkyl)amino. Examples of such heterocycles include piperidinyl, piperazinyl, 2-oxopiperazinyl, 2-oxopiperidinyl, 2-oxo-pyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, , isothiazolidinyl, thiadiazolyl, furyl, tetrahydrofuryl, tetrahydropyranyl, thienyl, thiamorpholinyl, thiamorpholinylsulfoxide, thiamorpholinylsulfone, oxadiazolyl, and triazolyl.

The definition of $X^2$ describes a divalent oxo atom or two hydrogen atoms, one hydrogen and one hydroxy group, or one hydrogen and one mercapto group a hydrogen and a halo group and two halo groups.

Most broadly defined the compounds of this invention are compounds having the formula (I) and pharmaceutically acceptable salts thereof;

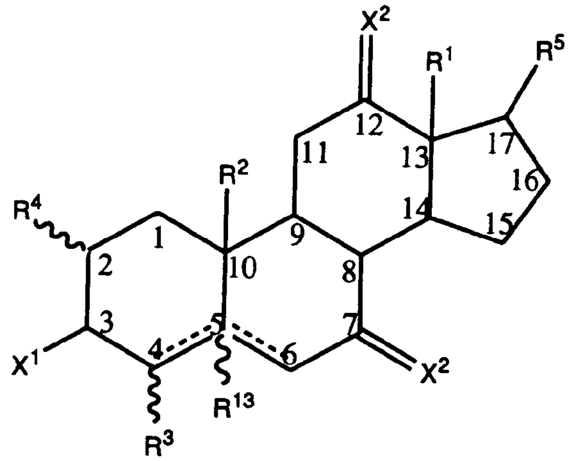

I

wherein the groups $R^1$, $R^5$ ,$X^2$, $R^{13}$, $R^3$, $X^1$, $R^4$, and $R^2$ are as hereinafter defined.

$R^1$ is a straight chain $C_1$-$C_4$ alkyl or $C_1$-$C_4$ halo alkyl;

$R^2$ is hydrogen, methyl, or halomethyl;

$R^3$ is hydrogen, $C_1$-$C_6$ alkyl, $C_1$-$C_6$ haloalkyl, or a group

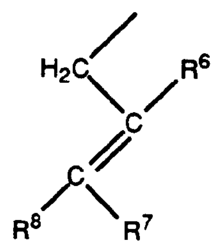

where, $R^6$ is hydrogen, halo, $C_1$-$C_4$ alkyl, $C_1$-$C_4$ haloalkyl, $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

$R^4$ is hydrogen, $-CH_2CH=C(X^4)_2$, substituted benzyl,or $-(CH_2)_n-X^4$ where n = 1 to 6, and $X^4$ is independently hydrogen, -OH, $C_1$-$C_6$ alkyl, $C_1$-$C_6$ haloalkyl, $C_1$-$C_6$ alkoxy, $C_1$-$C_6$ haloalkoxy, substituted or unsubstituted phenyl, substituted or unsubstituted phenoxy, substituted or unsubstituted benzyloxy, -SH, -S($C_1$-$C_4$ alkyl), or monocyclic heterocyclic ring;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

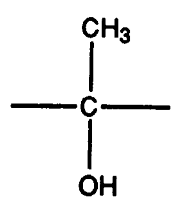

;

and

Z is a bond, -O-, $-CH_2-$, $-CH(CH_3)-$, $-CH(CH_2CH_3)-$, -CH(halo)-, $-C(halo)_2-$, or

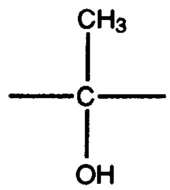 ;

provided that only one of A and Z are -O-, $-CH(CH_3)-$, $-CH(CH_2CH_3)-$,-CH(halo)-, $-C(halo)_2-$, or

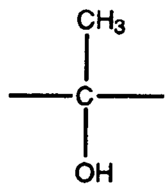 ;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl), -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl;

$R^{13}$ is hydrogen, provided the steroid nucleus is saturated, or $R^{13}$ is absent when the nucleus is unsaturated at the 4,5 or 5,6 position;

$X^1$ is hydroxy, acyloxy, amino, acetamido, substituted amino, mercapto, =O, or ($C_1$-$C_4$ alkoxy)carbonyloxy; and

each $X^2$ is independently oxygen; hydrogen, hydrogen; hydrogen, hydroxy; hydrogen, mercapto; halo, hydrogen; or halo, halo.

The compounds of the present invention, as will be appreciated by one skilled in the art, possess several potential chiral carbon atoms. As a consequence of these chiral centers, the compounds of the present invention occur as racemates, racemic mixtures, individual diastereomers and substantially pure isomers. All asymmetric forms, individual isomers, and combinations thereof, are within the scope of the present invention. A preferred class of compounds have the partial steric configuration shown in formula (II) as follows:

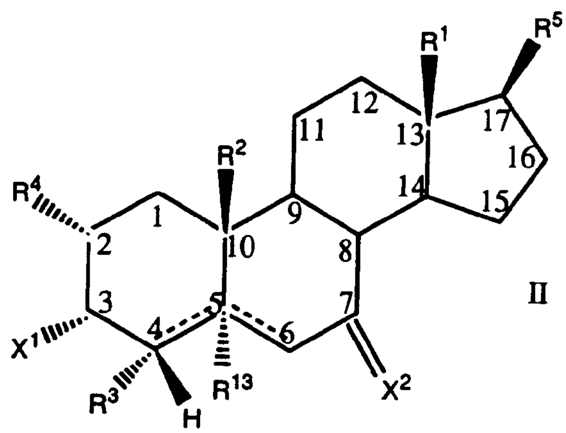

II

wherein, the substituents $R^1$, $R^5$, $X^2$, $R^{13}$, $R^3$, and $R^4$ and $R^2$ are as previously defined, for formula (I) and $X^1$ is selected from the group consisting of hydroxy, amino, acetamido, substituted amino, mercapto, ethylendioxy, and ($C_1$-$C_4$ alkoxy)carbonyloxy.

The group $R^3$ is preferably a 2-propenyl, 2-butenyl, 2-methyl-2-propenyl or 2-halo-2-propenyl group, where halo is preferably fluorine, chlorine or bromine.

Moreover, the group $R^5$ may have a variety of steric forms. Preferred forms of $R^5$ are those represented by the formulae XII, XIII, XIV, and XV set out below:

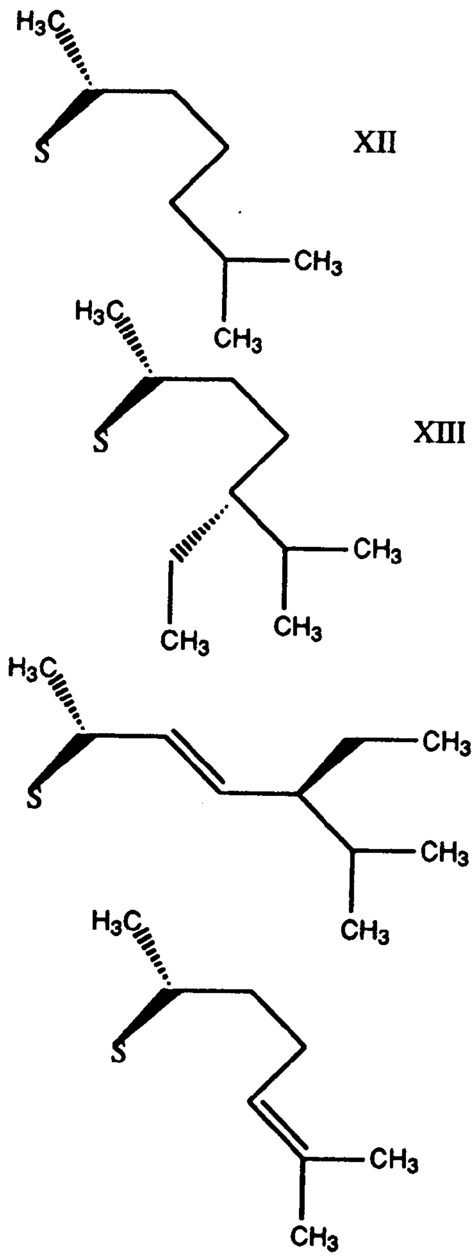

XII

XIII

XIV

XV

where s is the point of attachment of the group to the 17 position of the steroid ring of Formula II.

A particularly preferred class of compounds of this invention having active substituents at the 4 position of the steroid nucleus are the compounds of formula II having the following combinations (1) or (2) of substituents:

(1) $R^4$ is hydrogen; and

$R^3$ is $C_1$-$C_6$ alkyl, $C_1$-$C_6$ haloalkyl, a group

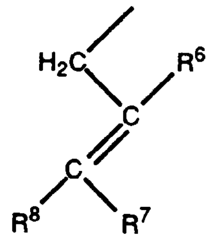

$R^6$ is hydrogen, halo, $C_1$-$C_4$ alkyl, $C_1$-$C_4$ haloalkyl, $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

or,

(2) $R^4$ is hydrogen; and

$R^3$ is a benzyl group represented by the formula:

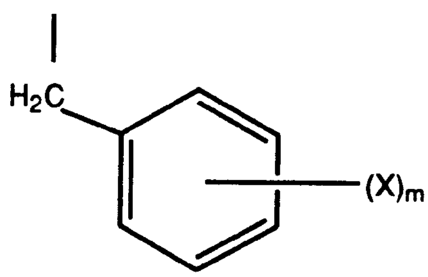

where X is a group selected from hydrogen, hydroxy, hydroxyalkyl, halo, -COOH, ester, alkoxy, alkoxyalkyl, alkoxyaryl, and wherein m is 1 to 3, with at least one X para to the carbon atom attached to the steroid nucleus.

A particularly preferred class of compounds of this invention having active substituents at the 2 position of the steroid nucleus are the compounds of formula II having the following combinations of substituents:

(1) $R^3$ is hydrogen; and

$R^4$ is $C_1$-$C_6$ alkyl, $C_1$-$C_6$ haloalkyl, or a group

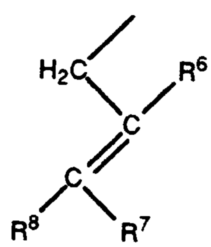

where, $R^6$ is hydrogen, halo, $C_1$-$C_4$ alkyl, $C_1$-$C_4$ haloalkyl, $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

or,

(2) $R^3$ is hydrogen; and

$R^4$ is a benzyl group represented by the formula:

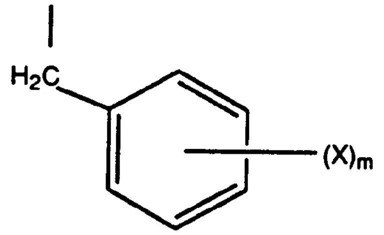

where X is a group selected from hydrogen, hydroxy, hydroxyalkyl, halo, -COOH, ester, alkoxy, alkoxyalkyl, alkoxyaryl, and wherein m is 1 to 3, with at least one X para to the carbon atom attached to the steroid nucleus.

Most preferred because of ease of preparation are steroid compounds with a saturated ring represented by formula III below:

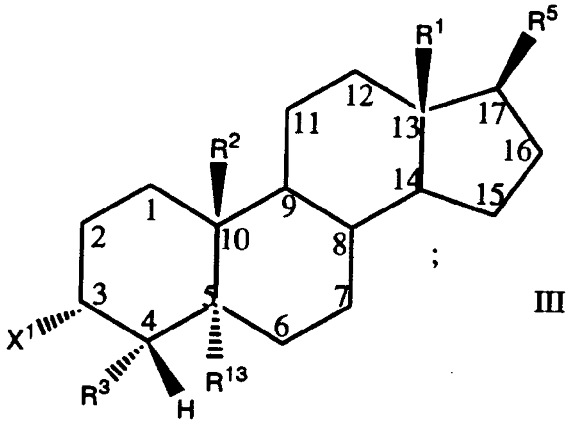

;   III

wherein;

$R^1$ is a straight chain $C_1$-$C_4$ alkyl or $C_1$-$C_4$ halo alkyl;

$R^2$ is hydrogen, methyl, or halomethyl;

$R^3$ is hydrogen, $C_1$-$C_6$ alkyl, $C_1$-$C_6$ haloalkyl, or a group

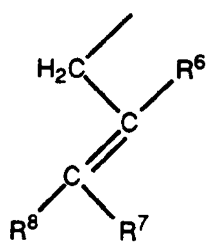

where, $R^6$ is hydrogen, halo, $C_1$-$C_4$ alkyl, $C_1$-$C_4$ haloalkyl, $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

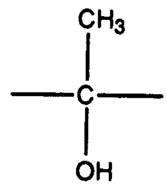 ;

and

Z is a bond, -O-, $-CH_2-$, $-CH(CH_3)-$, $-CH(CH_2CH_3)-$, -CH(halo)-, $-C(halo)_2-$, or

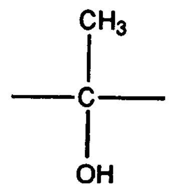 ;

provided that only one of A and Z are -O-, $-CH(CH_3)-$, $-CH(CH_2CH_3)-$,-CH(halo)-, $-C(halo)_2-$, or

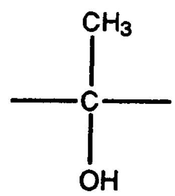 ;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl;

$X^1$ is hydroxy, acyloxy, amino, acetamido, substituted amino, mercapto, =O, ethylendioxy, or ($C_1$-$C_4$ alkoxy) carbonyloxy;

$R^{13}$ is hydrogen.

Illustrative of specific compounds having utility in the preparation of pharmaceutical compositions and practicing the methods of this invention are the following:

[4α(E),5α]-4-(2-butenyl)cholestan-3α -ol,
[4α5α]-4-(1-propenyl)cholestan-3α -ol ,
[4α(E),5α]-4-(2-butenyl)-25-hydroxycholestan-3α-ol ,
[4α,5α]-4-butylcholestan-3α -ol ,
[4α(E),5α]-4-(2-butenyl)-3α aminocholestane ,
[4α(E),5α]-4-(2-butenyl)-3α acetamidocholestane ,
[4α(E),5α]-4-(2-butenyl)-3b acetamidocholestane
4α-(4-fluorobenzyl)cholestan-3α-ol ,
4α-(4-bromobenzyl)cholestan-3α-ol,
4α-(4-iodobenzyl)cholestan-3α-ol,
4α-(4-trifluoromethylbenzyl)cholestan-3α-ol,
4α-(4-dichlorobenzyl)cholestan-3α-ol,

4α-(4-cyanobenzyl)cholestan-3α-ol,
4α-(4-methoxycarbonylbenzyl)cholestan-3α-ol,
4α-(4-trifluoromethoxybenzyl)cholestan-3α-ol,
4α-(4-chlorobenzyl)cholestan-3α-ol,
4α-(4-benzyloxybenzyl)cholestan-3α-ol,
4α-(4-hydroxymethylbenzyl)cholestan-3α-ol,
4α-(4-carboxybenzyl)cholestan-3α-ol,
4α-(4-hydroxybenzyl)cholestan-3α-ol,
4α-benzyl-4-cholestan-3α-ol,
4α-allyl-5-cholestan-3α-ol,
4α-allyl-cholan-24-N,N-dimethylamino-3α-ol,
3α, 12α-dihydroxy-25-azacoprostane,
3α-hydroxy-25-azacoprostane,
3α, 7α-dihydroxy-25-azacoprostane,
3α,7α, 12α-trihydroxy-25-azacoprostane,
3α,7α, 12α-dihydroxy-25-azacoprostane,
(3α,4α, 5α)-17-(pentyloxy)-4-(2-propenyl) androstan-3-ol ,
(3α,4α, 5α)-17-(octyloxy)-4-(2-propenyl) androstan-3-ol ,
(3α,4α)-17-[(4-methylpentyl)oxy]-4-(2-propenyl) androstan-3-ol ,
(3α,4α)-17-(3-phenylpropoxy)-4-(2-propenyl) androstan-3-ol ,
(3α,4α)-17-(phenylmethoxy)-4-(2-propenyl) androstan-3-ol ,
(3α,4α)-17-[(4,4-dimethylpentyl)oxy]-4-(2-propenyl) androstan-3-ol ,
2-(hydroxymethylene)-4α-(2-propenyl)cholestan -3-one,
(2α,3α,5α)-2-(2-propenyl)cholestan-3-ol
3β,4α,5α,20β)-4-(2-propenyl)cholestan-3-ol
(3α,4α,5α,20β)-4-(3,3-difluoro-2-propenyl) cholestan-3-ol
(3α,4α,5α,20β)-4-(2-propenyl)cholestan-3-amine
(3α,4α,5α,20β)-4-propylcholestan-3-ol
(3α,4α)-4-(2-methyl-2-propenyl)cholestan-3-ol
(3α,4α)-4-(2-chloro-2-propenyl)cholestan-3-ol
(3α,4α)-4-(2-bromo-2-propenyl)cholestan-3-ol,
(3α,4α,24R)-4-(2-propenyl)-24-(ethyl)cholestan-3- ol, and
(3α,4α,22E, 24R)-4-(2-propenyl)-24- (ethyl)cholest-22-en-3-ol.

Other highly preferred compounds are (3α,4α,5α)-4-(2-chloro-2 propenyl) cholestan-3-ol and (3α,4α,5α)-4-(2-bromo-2-propenyl)cholestan-3-ol represented by Formula VI; (3α,4α,5α)-4-(2-propenyl)stigmastan-3-ol represented by formula VII; and (3α,4α,5α,22E)-4-(2-propenyl)stigmast-22-en-3-ol represented by formula VIII as follows:

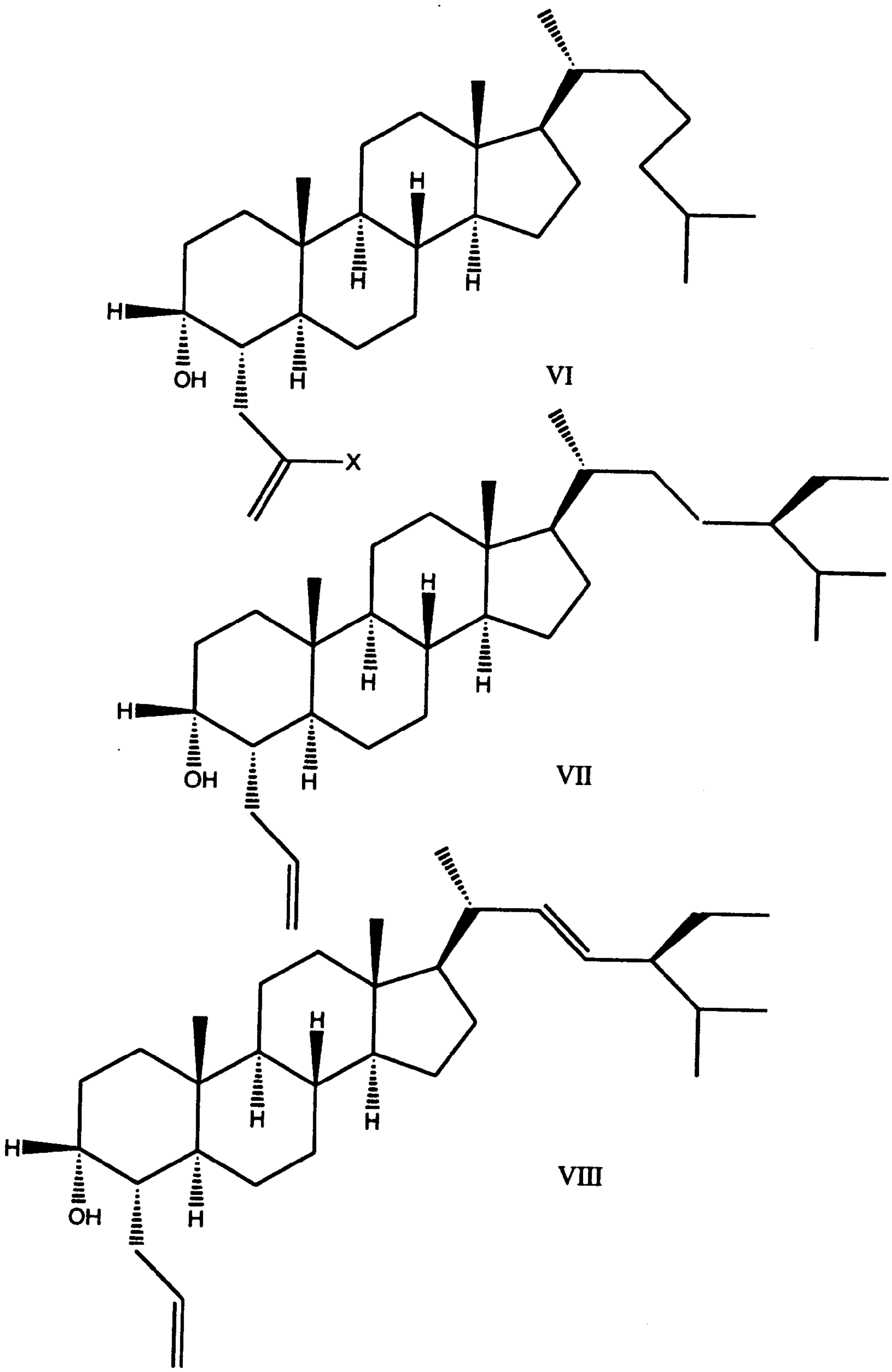

VI

VII

VIII

Most preferred are the following compounds:

[4α(E),5α]-4-(2-butenyl)cholestan-3α-ol represented by the formula:

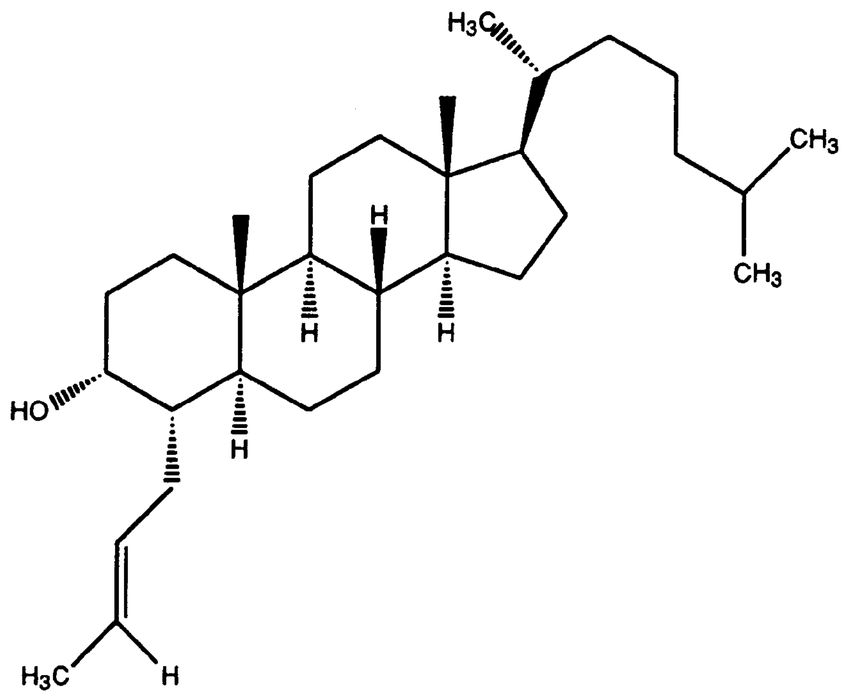

and

[4α,5α]-4-(2-propenyl)cholestan-3α-ol represented by the formula:

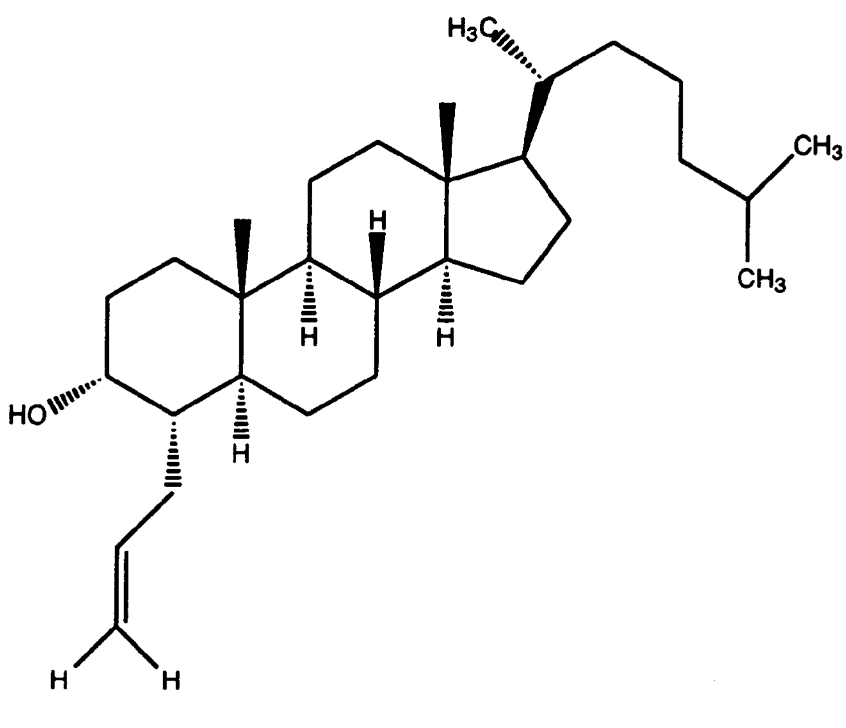

Other species of the compounds of the invention which have cholesterol and/or lipid lowering effects are represented by formulae IV and V as defined below:

For formula IV:

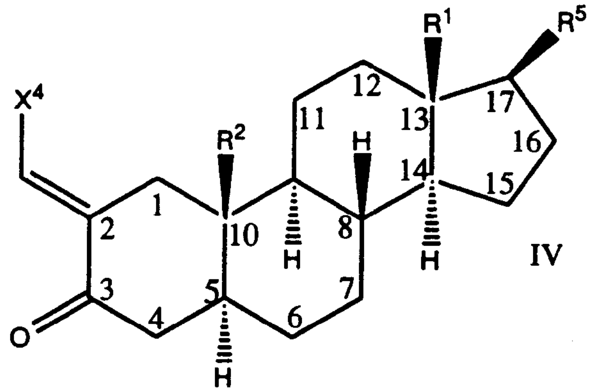

IV

wherein:

$R^1$ is a straight chain $C_1$-$C_4$ alkyl or $C_1$-$C_4$ halo alkyl;

$R^2$ is hydrogen, methyl, or halomethyl;

$R^5$ is the group

-A-Z-$R^{10}$-$X^3$

where

A is a bond, -O-, -$CH_2$-, -$CH(CH_3)$-, -$CH(CH_2CH_3)$-, -CH(halo)-, -$C(halo)_2$-, or

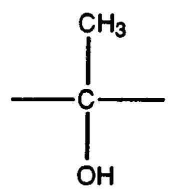

;

and

Z is a bond, -O-, -$CH_2$-, -$CH(CH_3)$-, -$CH(CH_2CH_3)$-, -CH(halo)-, -$C(halo)_2$-, or

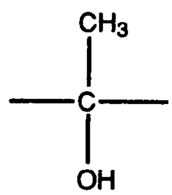

;

provided that only one of A and Z are -O-, -$CH(CH_3)$-, -$CH(CH_2CH_3)$-,-CH(halo)-, -$C(halo)_2$-, or

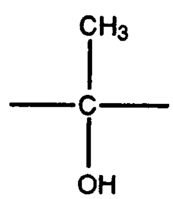

;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$-$C_{12}$ alkene,

where the substituents of (i) and (ii) are one or more of the same or different hydroxy, -$CH_2N(alkyl)_2$,

substituted amino, amino, mercapto, $-S(C_1-C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, $-S(C_1-C_6$ alkyl), $-CF_3$, -CN, $C_2-C_6$ alkenyl, $-OC(F)_3$, $C_1-C_4$ alkoxy, $-C(O)C_1-C_4$ alkyl, $-C(O)(C_2-C_6$ alkenyl) -CHO, -COOH, $-COO(C_1-C_4$ alkyl), $-NR^{11}R^{12}$, $-C(O)NR^{11}R^{12}$ where $R^{11}$ and $R^{12}$ are independently hydrogen or $C_1-C_4$ alkyl;

$X^4$ is hydrogen, -OH, $C_1-C_6$ alkoxy, substituted or unsubstituted phenoxy, substituted or unsubstituted benzyloxy or $-NR^8R^9$ where $R^8$ and $R^9$ are independently hydrogen or $C_1-C_4$ alkyl or combine with the nitrogen atom to which they are attached to form a substituted or unsubstituted nitrogen containing heterocyclic ring.

For formula V:

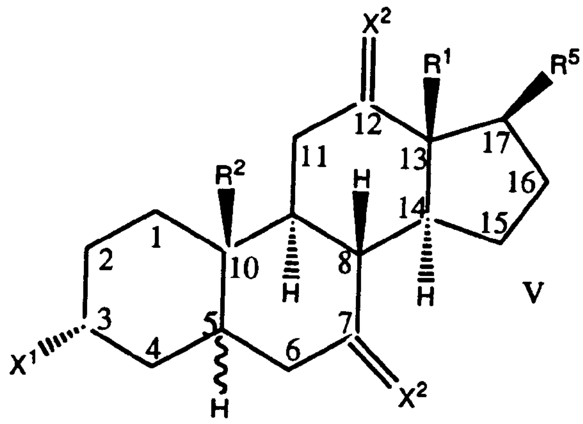

V

wherein:

$R^1$ is a straight chain $C_1-C_4$ alkyl or $C_1-C_4$ halo alkyl;

$R^2$ is hydrogen, methyl, or halomethyl;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

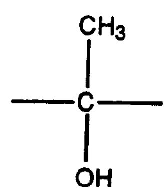

;

and

Z is a bond, -O-, $-CH_2-$, $-CH(CH_3)-$, $-CH(CH_2CH_3)-$, -CH(halo)-, $-C(halo)_2-$, or

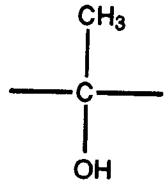

;

provided that only one of A and Z are -O-, $-CH(CH_3)-$, $-CH(CH_2CH_3)-$,-CH(halo)-, $-C(halo)_2-$, or

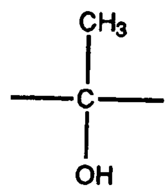 ;

$R^{10}$ is

(i) a divalent unsubstituted or substituted, branched or unbranched radical derived from a $C_1$-$C_{12}$ alkene, or

(ii) a divalent unsubstituted or substituted, branched or unbranched, unsaturated radical derived from a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxyhalo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), or monocyclic heterocyclic ring;

$X^1$ is hydroxy, acyloxy, amino, acetamido, substituted amino, mercapto, =O, or ($C_1$-$C_4$ alkoxy)carbonyloxy; and

each $X^2$ is independently oxygen; hydrogen, hydrogen; hydrogen, hydroxy; hydrogen, mercapto; halo, hydrogen; or halo, halo.

As mentioned above, the invention includes pharmaceutically acceptable salts of the compounds defined by the above formula. A particular compound of this invention can react with any of a number of nontoxic inorganic bases, and nontoxic inorganic and organic acids, to form a pharmaceutically acceptable salt. Acids commonly employed to form acid addition salts are inorganic acids such as hydrochloric acid, hydrobromic acid, hydroiodic acid, sulfuric acid, phosphoric acid, and the like, and organic acids such as p-toluene-sulfonic, methanesulfonic acid, oxalic acid, p-bromo-phenylsulfonic acid, carbonic acid, succinic acid, citric acid, benzoic acid, acetic acid, and the like. Examples of such pharmaceutically acceptable salts thus are the sulfate pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate,benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycollate, tartrate, methanesulfonate, propanesulfonate, naphthalene-1-sulfonate, naphthalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

The compounds of the present invention, or their precursors, are prepared using procedures known to those of ordinary skill in the art.

The compounds of the present invention, which may themselves be used as intermediates for preparing further compounds of the present invention, are prepared generally in accordance with the reactions shown in Schemes 1-8.

SCHEME 1:
A.

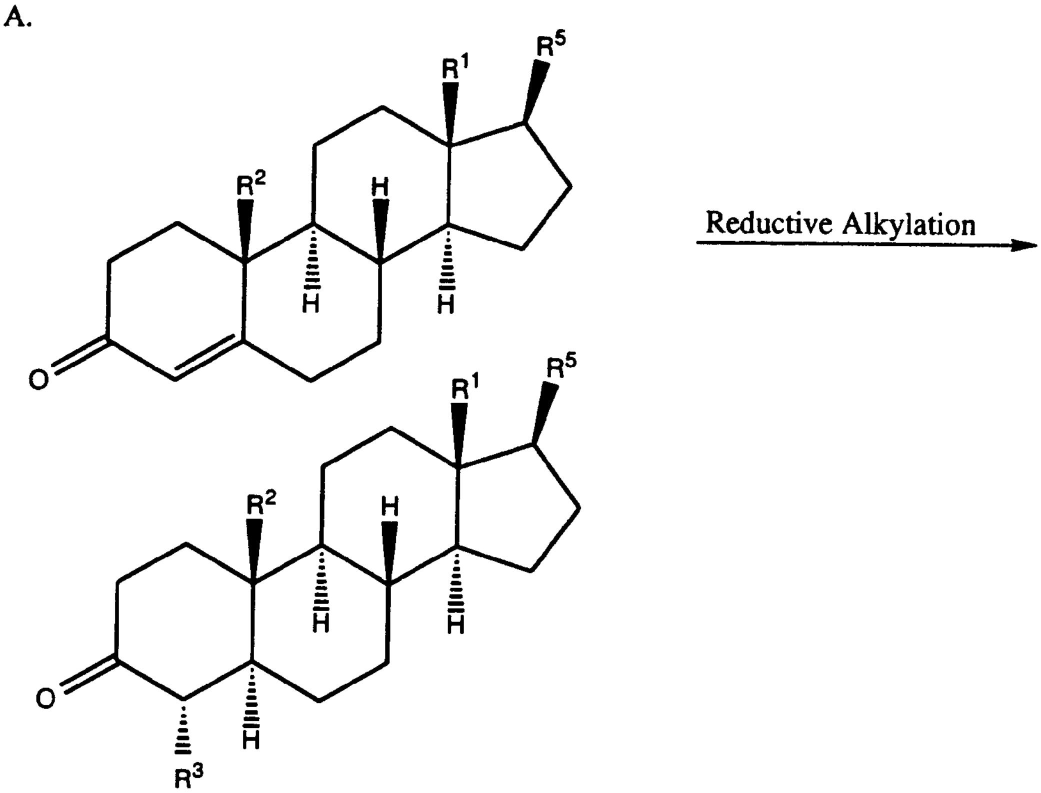


SCHEME 1:
B.

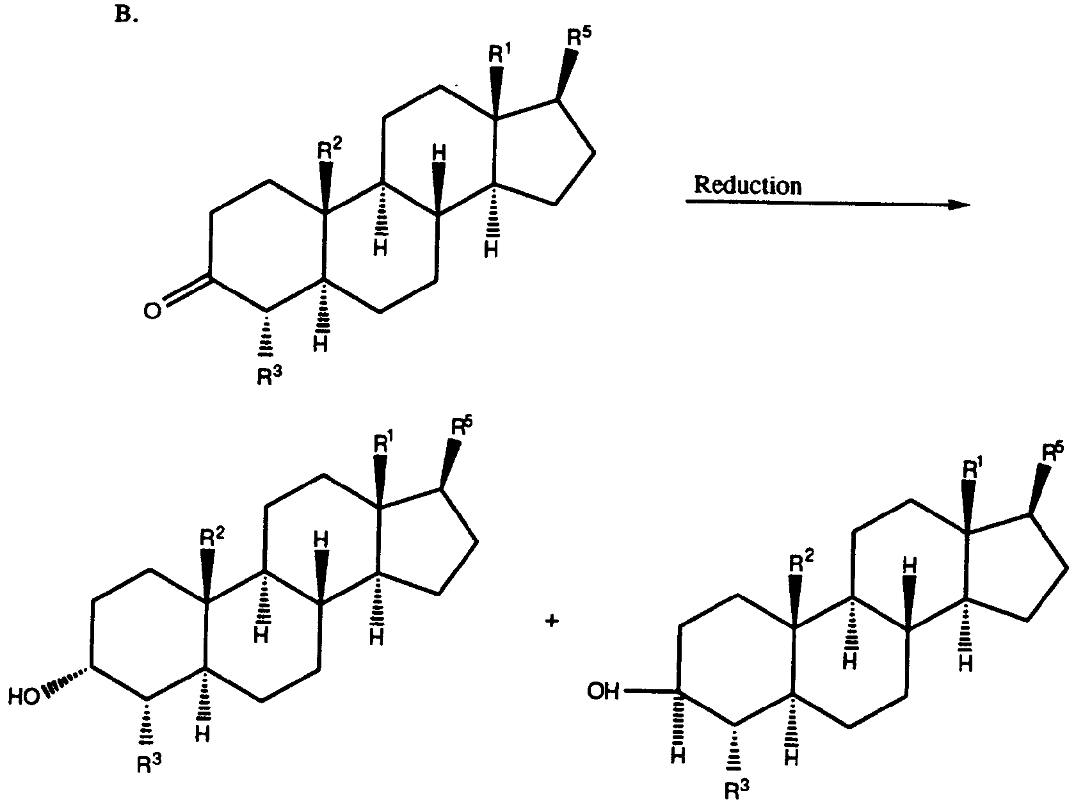

SCHEME 1:
C.

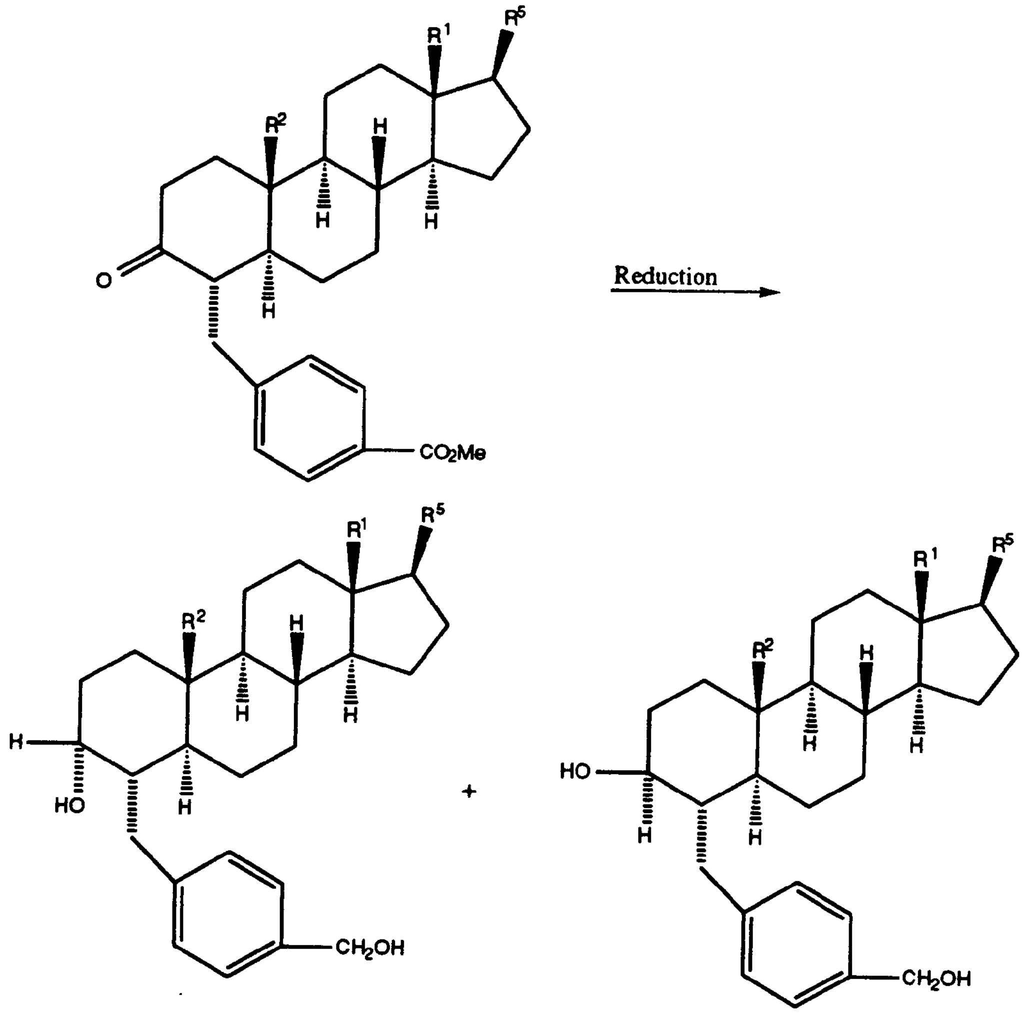

SCHEME 1
D.

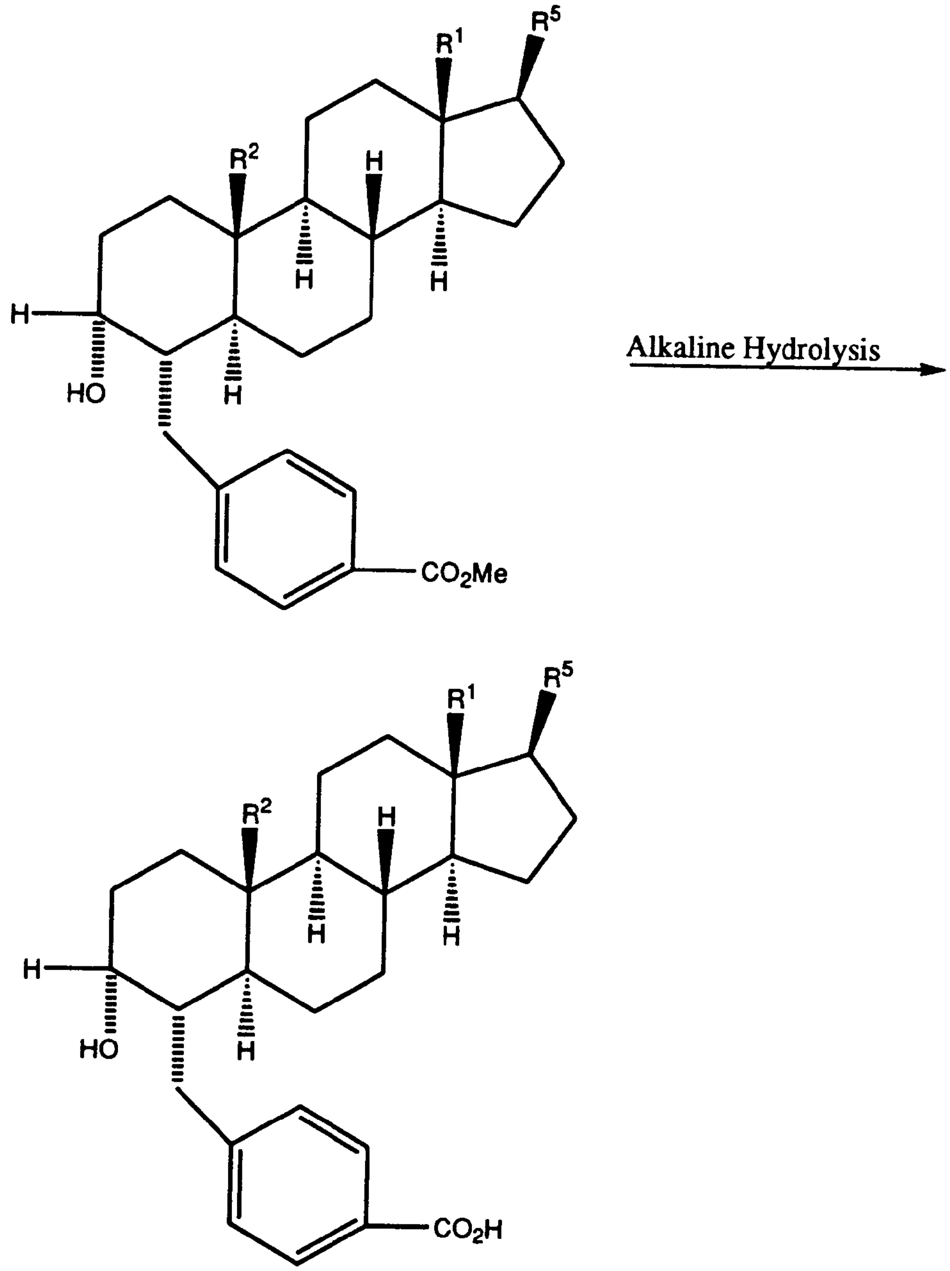

SCHEME 1:
E.

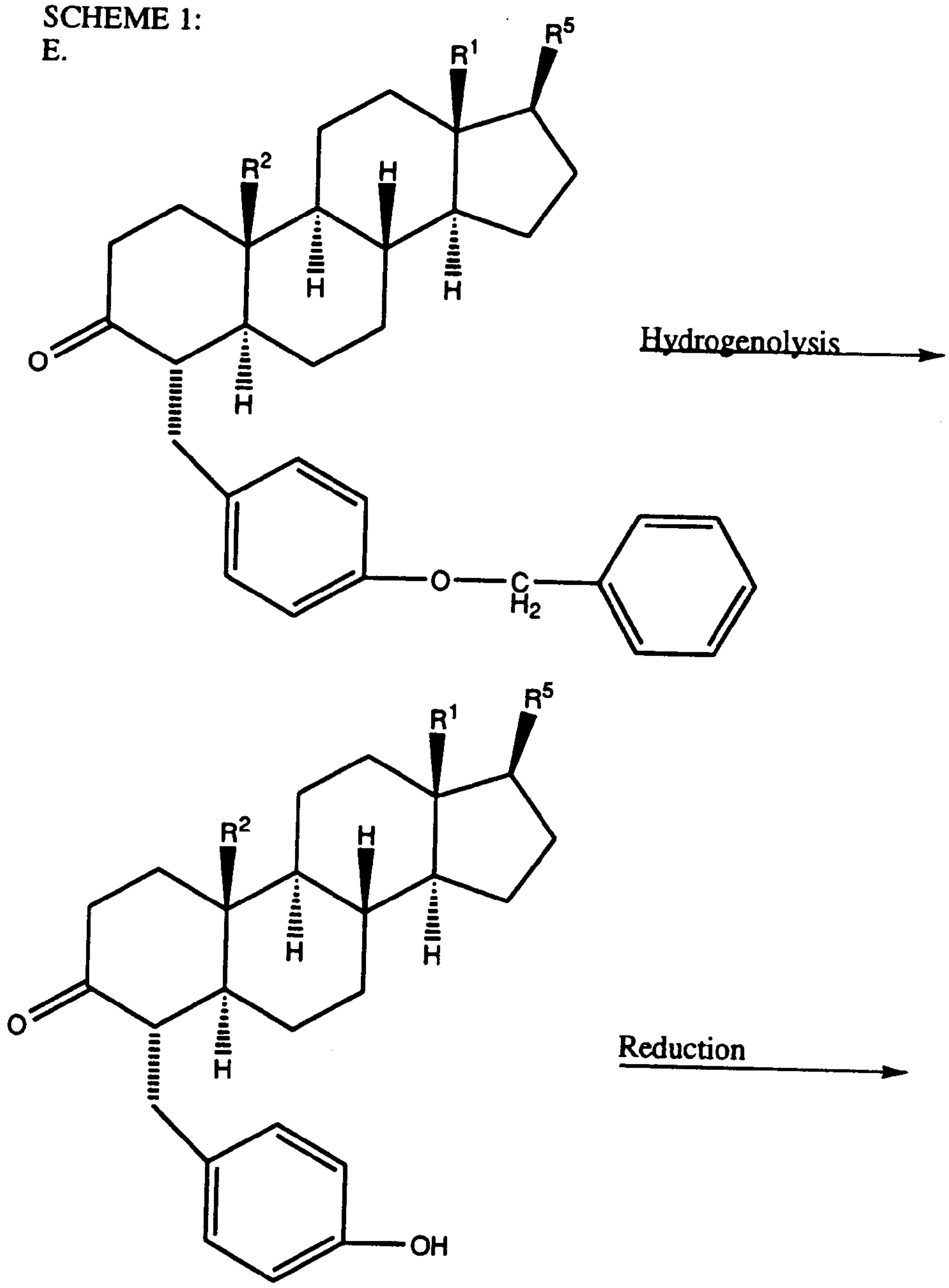

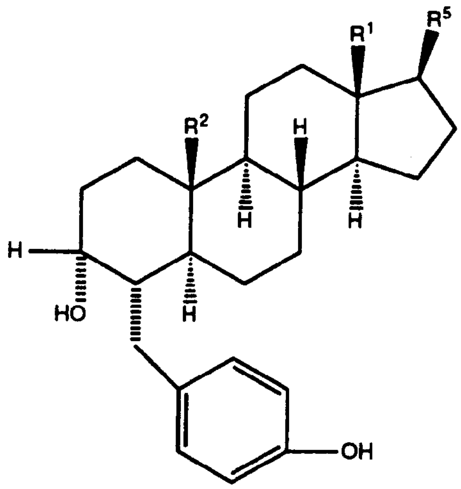

+

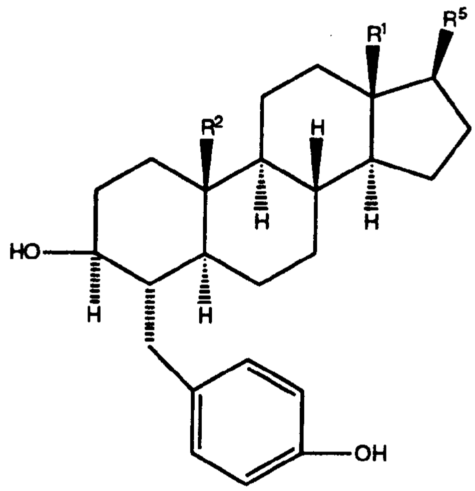

SCHEME 2:

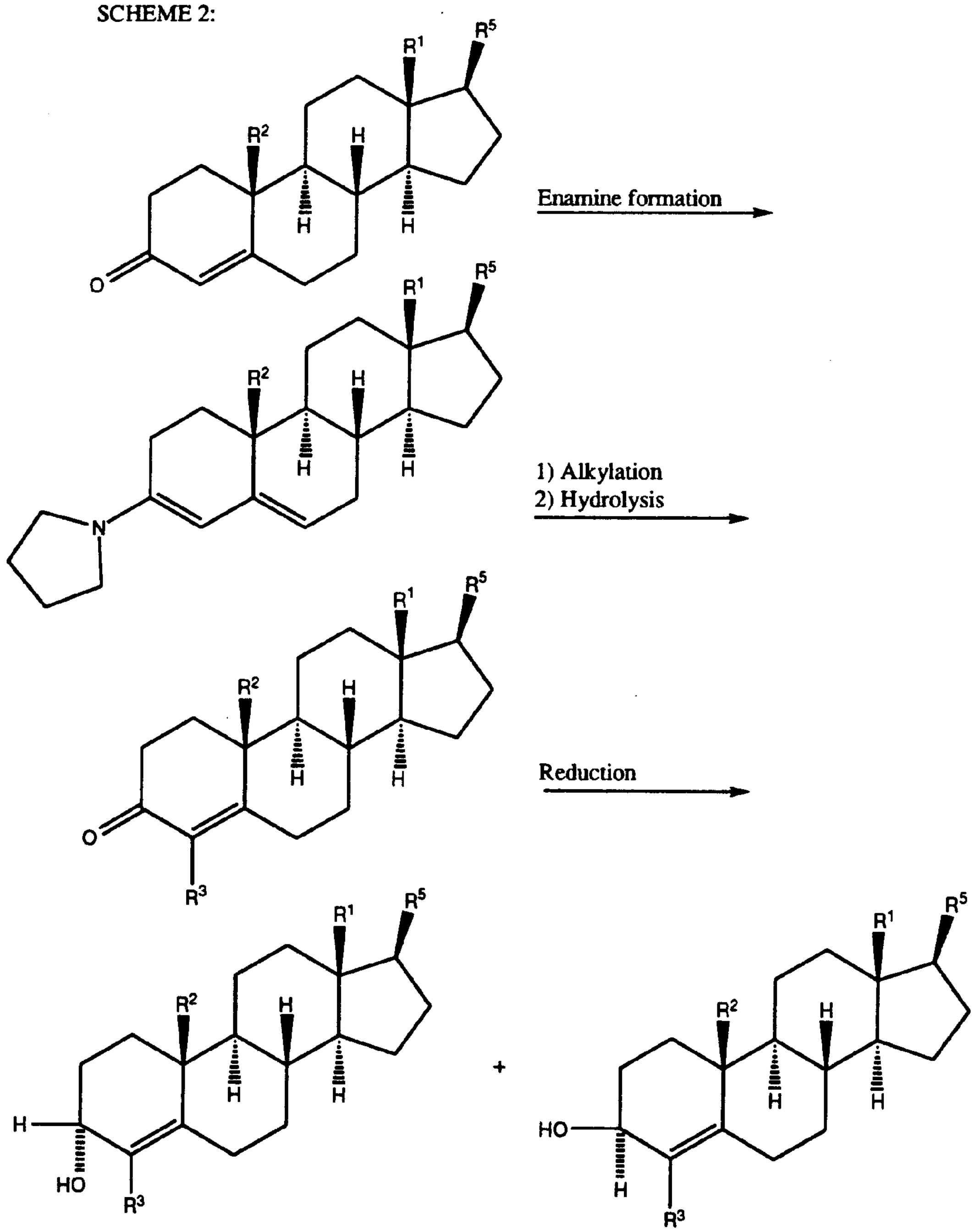

SCHEME 3:
A.

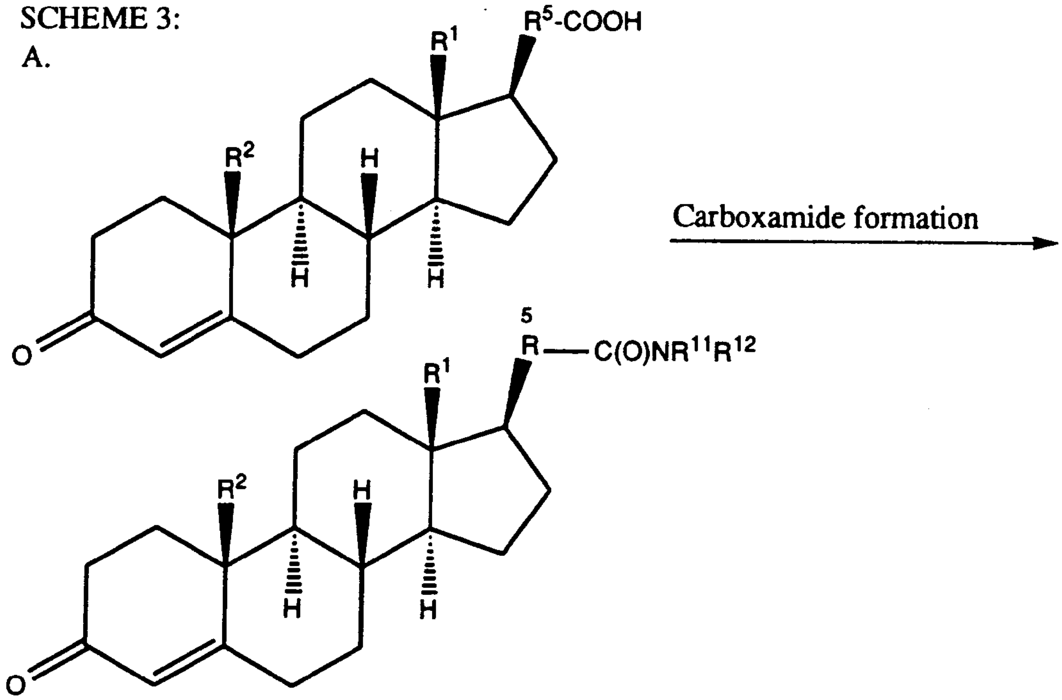


SCHEME 3:
B.

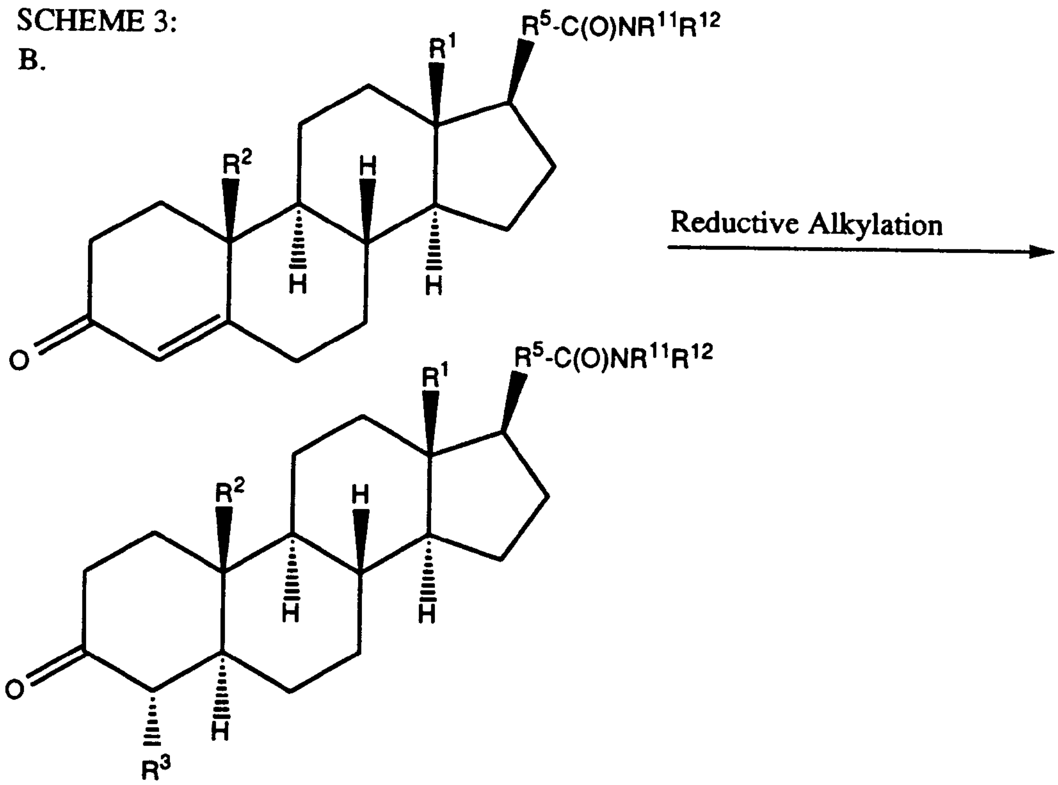

SCHEME 3:
C.

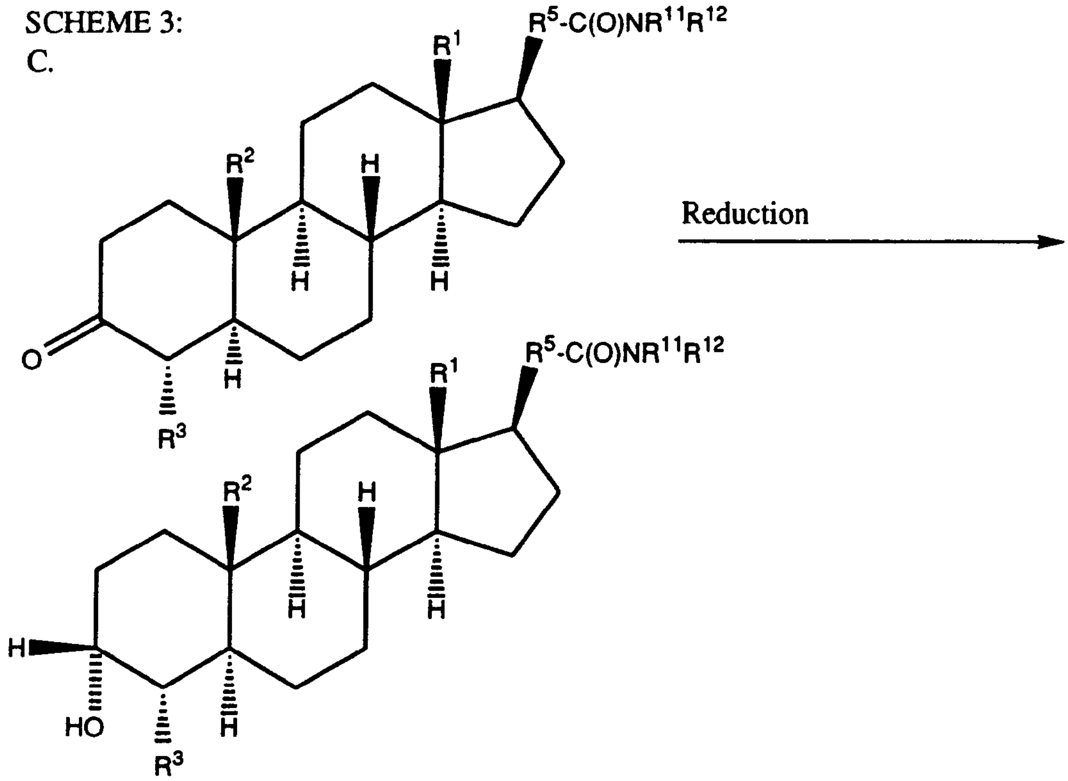


SCHEME 3:
D.

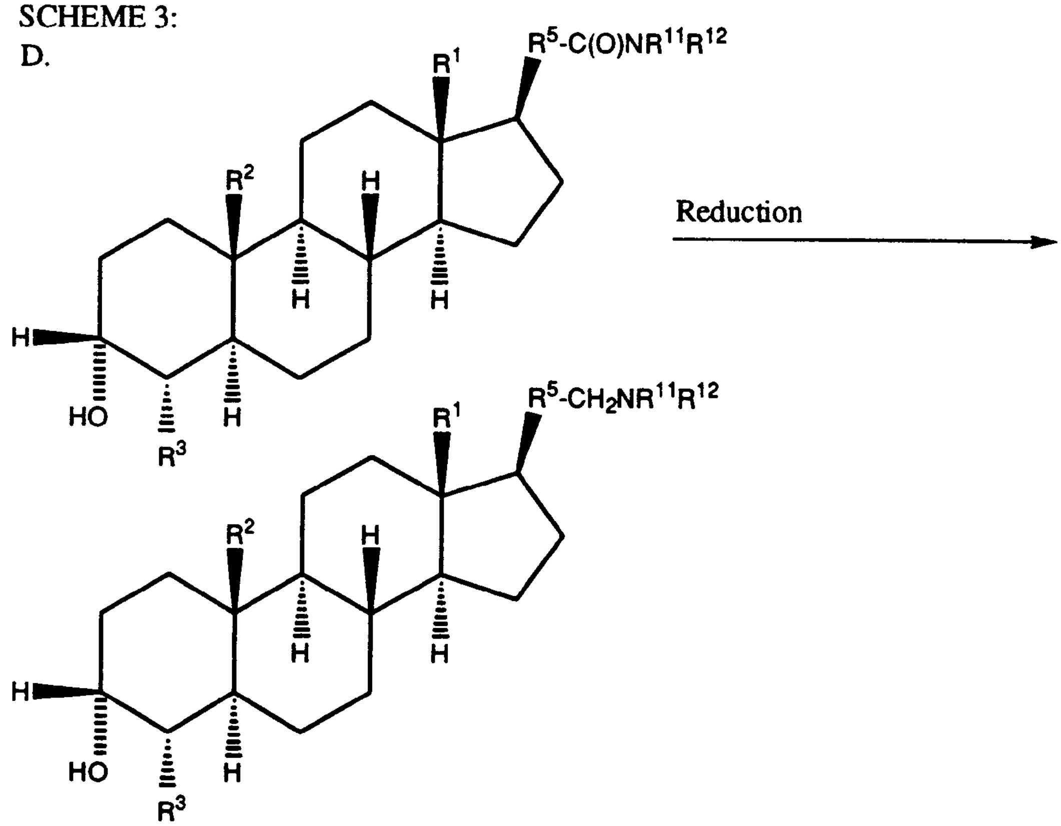

SCHEME 4:
A.

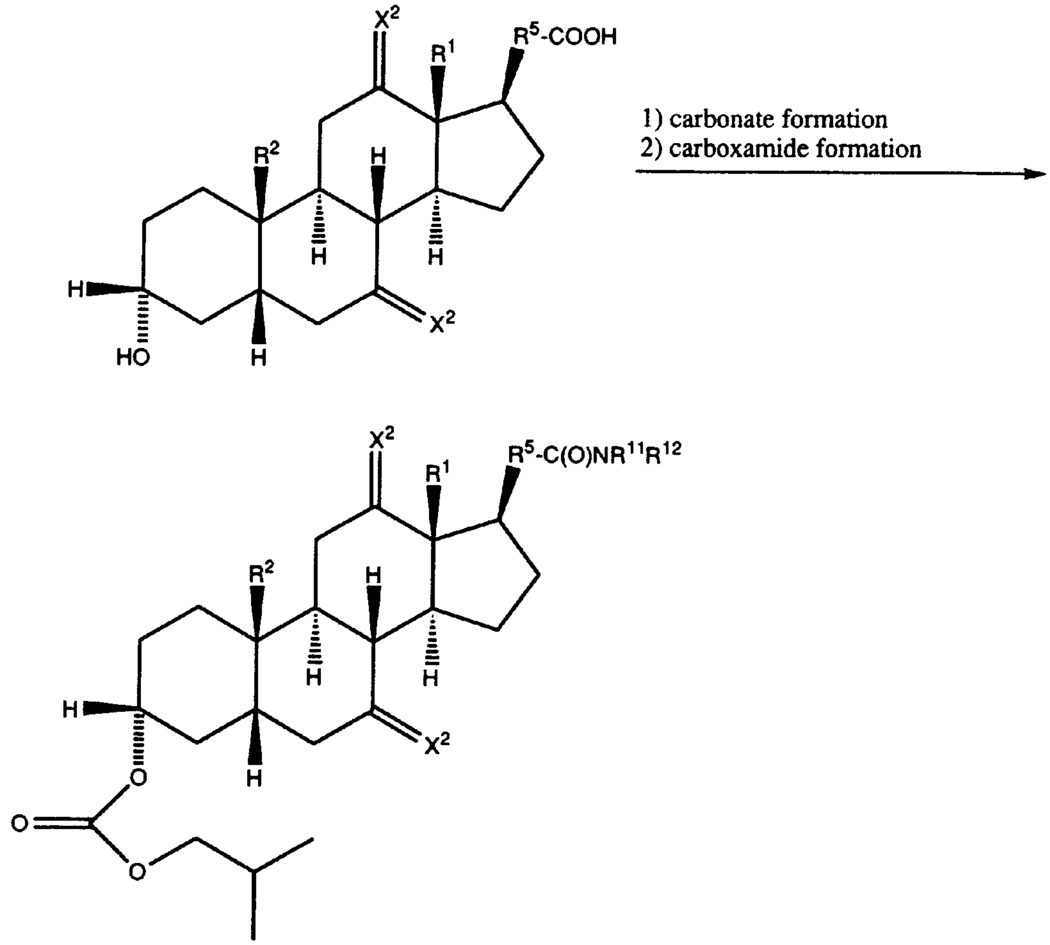

SCHEME 4:
B.

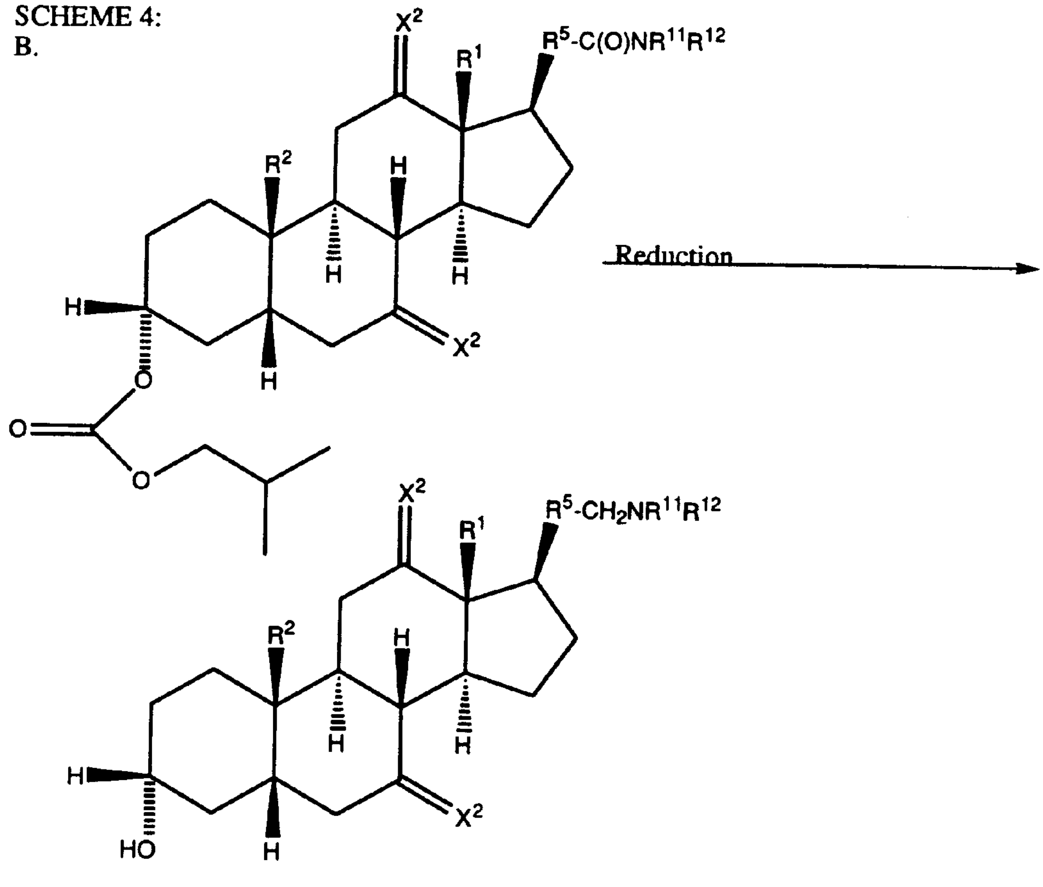

SCHEME 5:
Part 1.

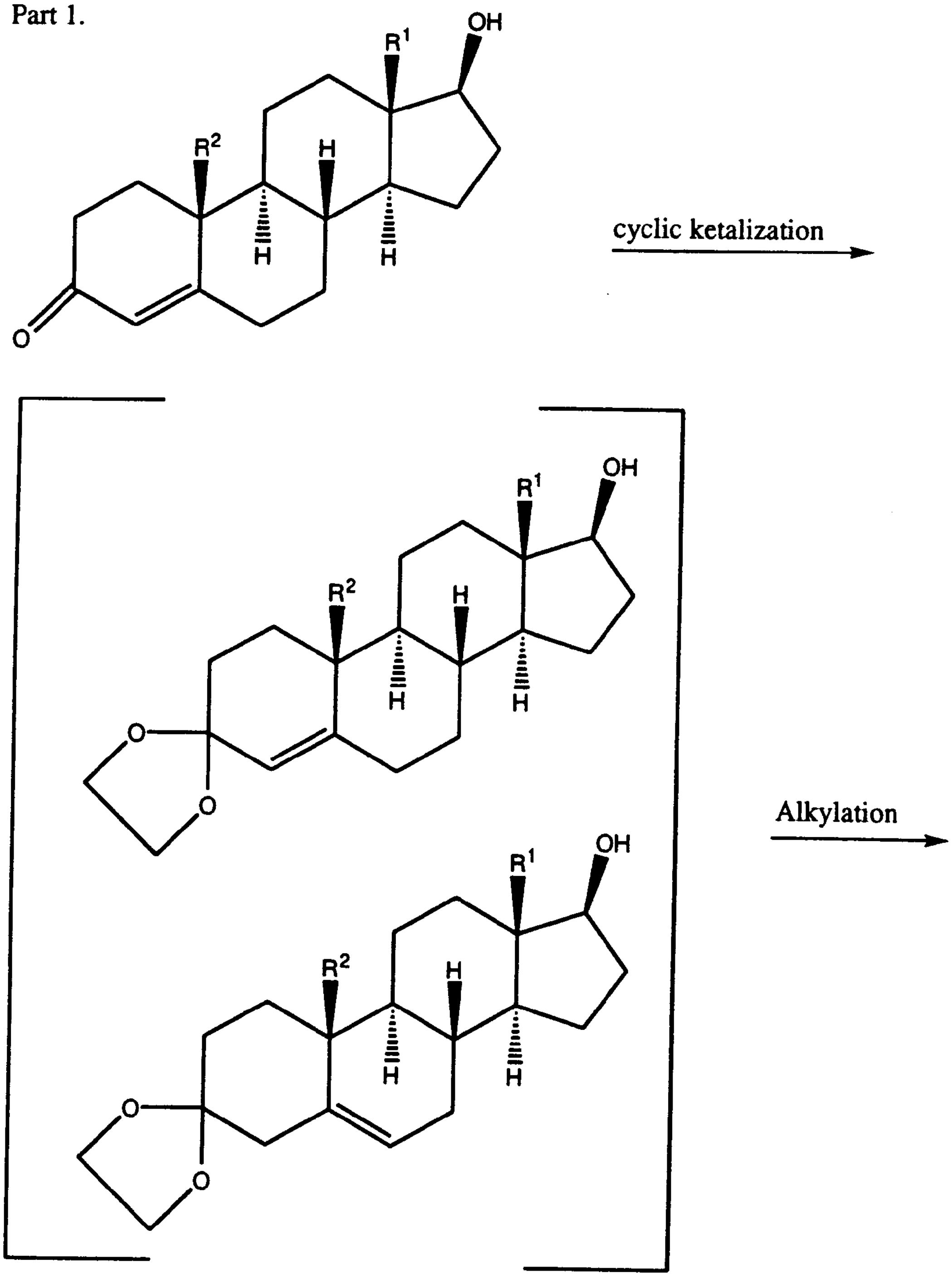

SCHEME 5:
Part 2.

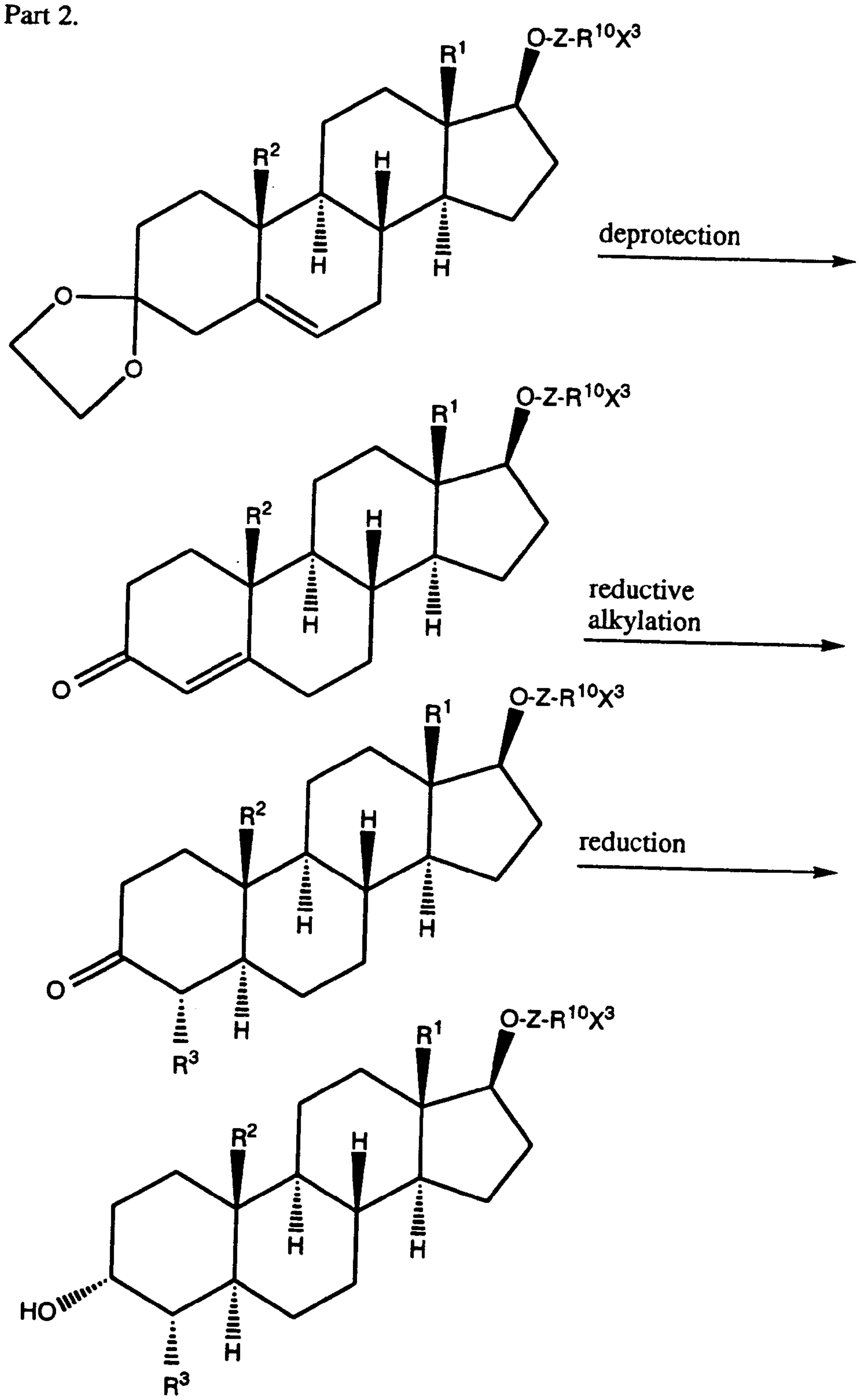

SCHEME6:
Part 1.

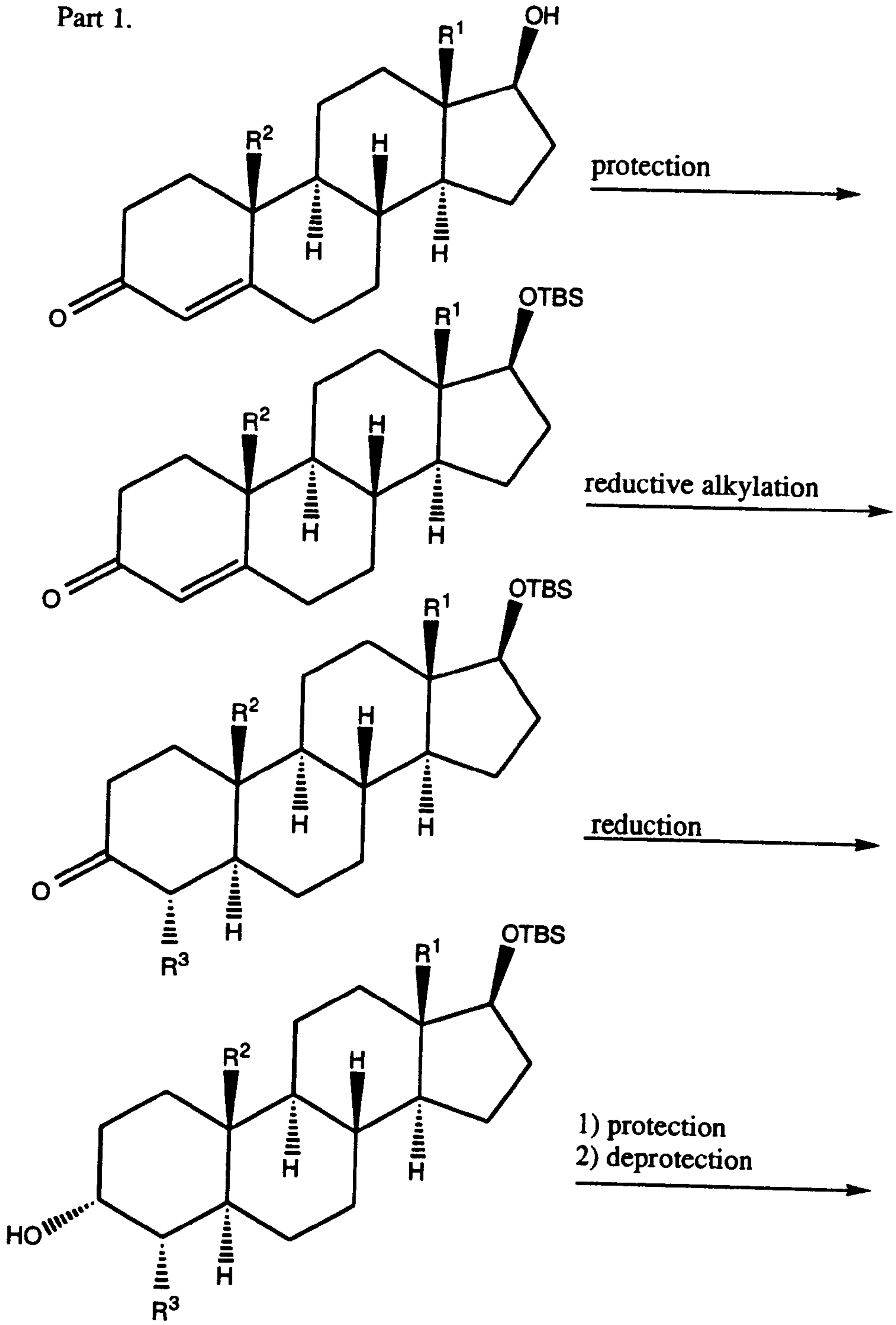

SCHEME 6:
Part 2.

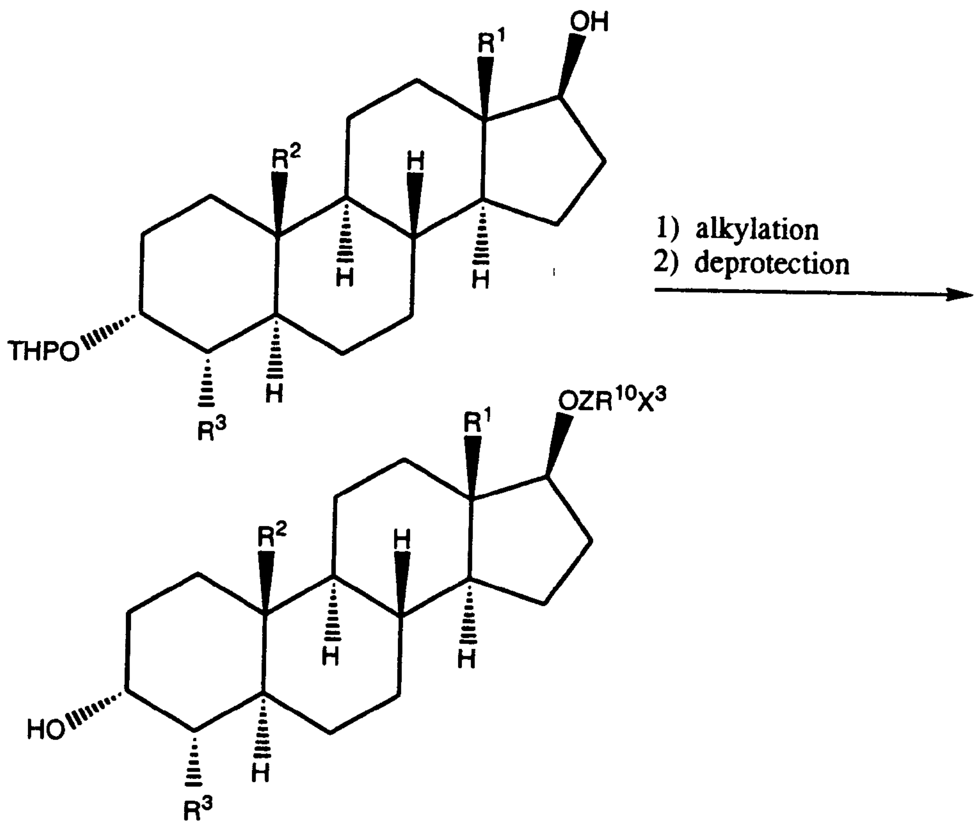

SCHEME 7:
Part 1.

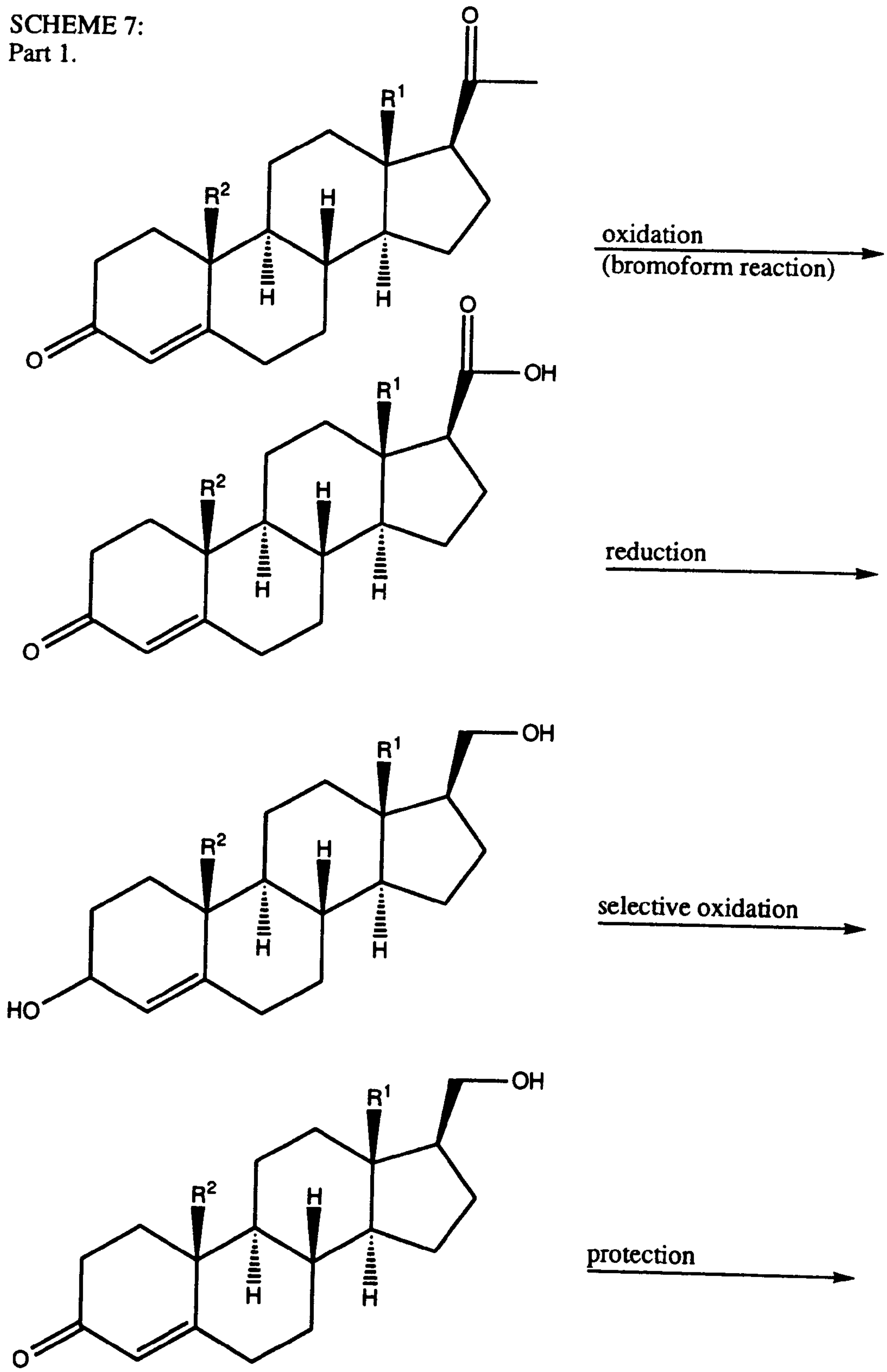

SCHEME 7:
Part 2.

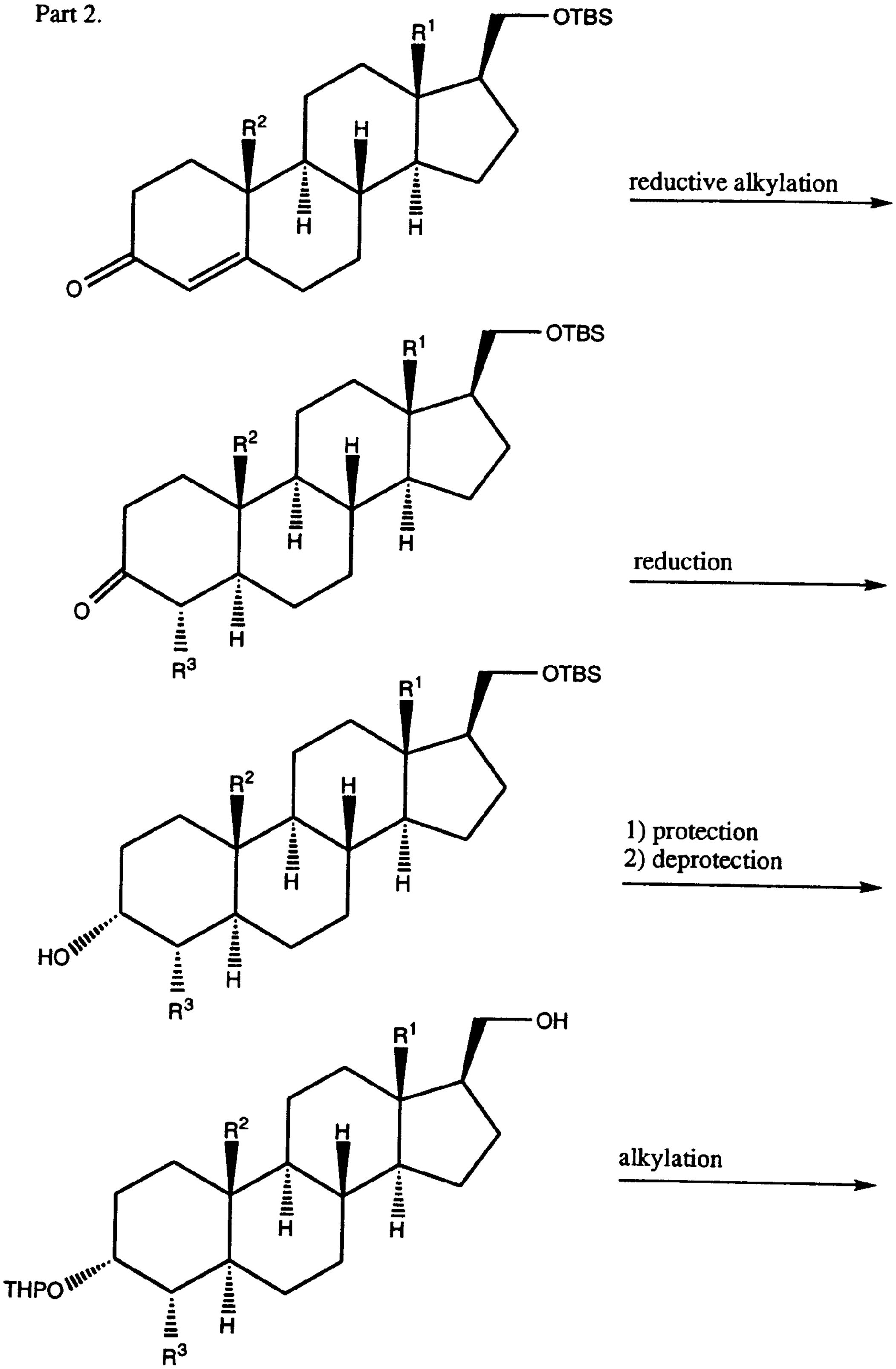

SCHEME 7:
Part 3.

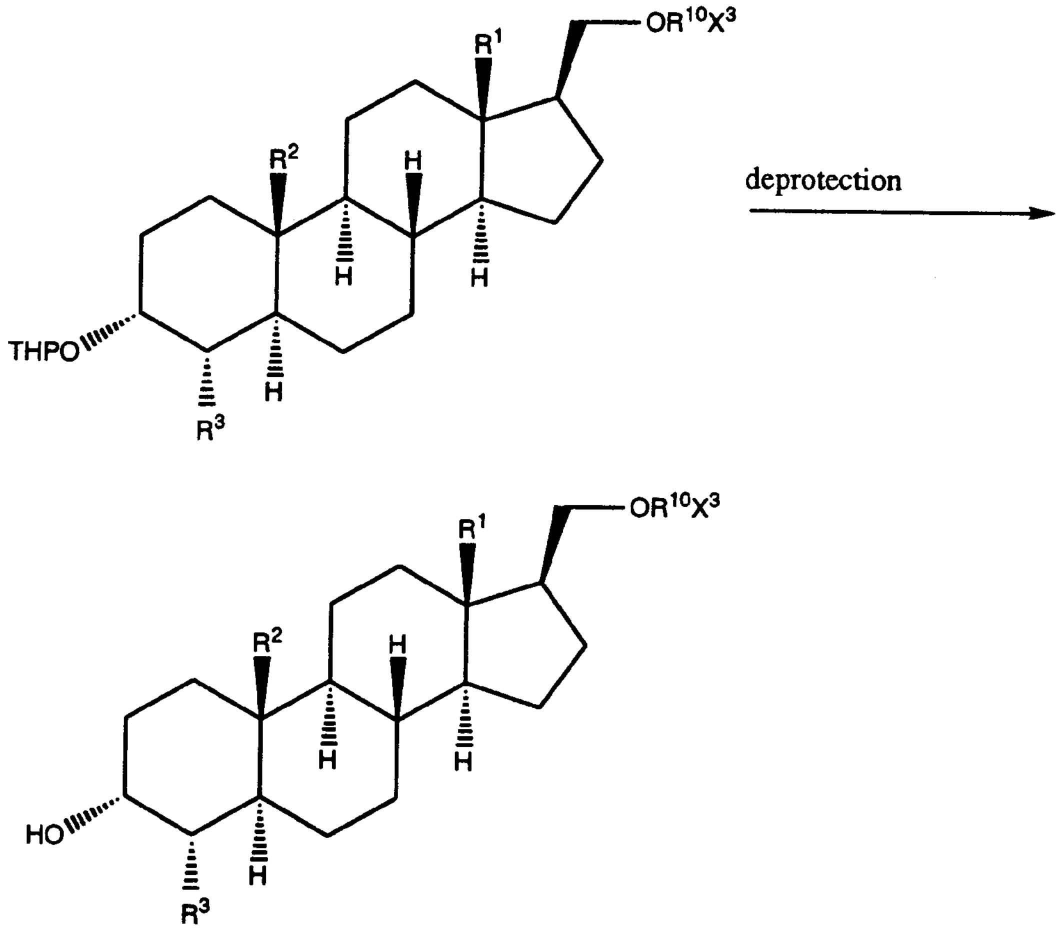

SCHEME 8:

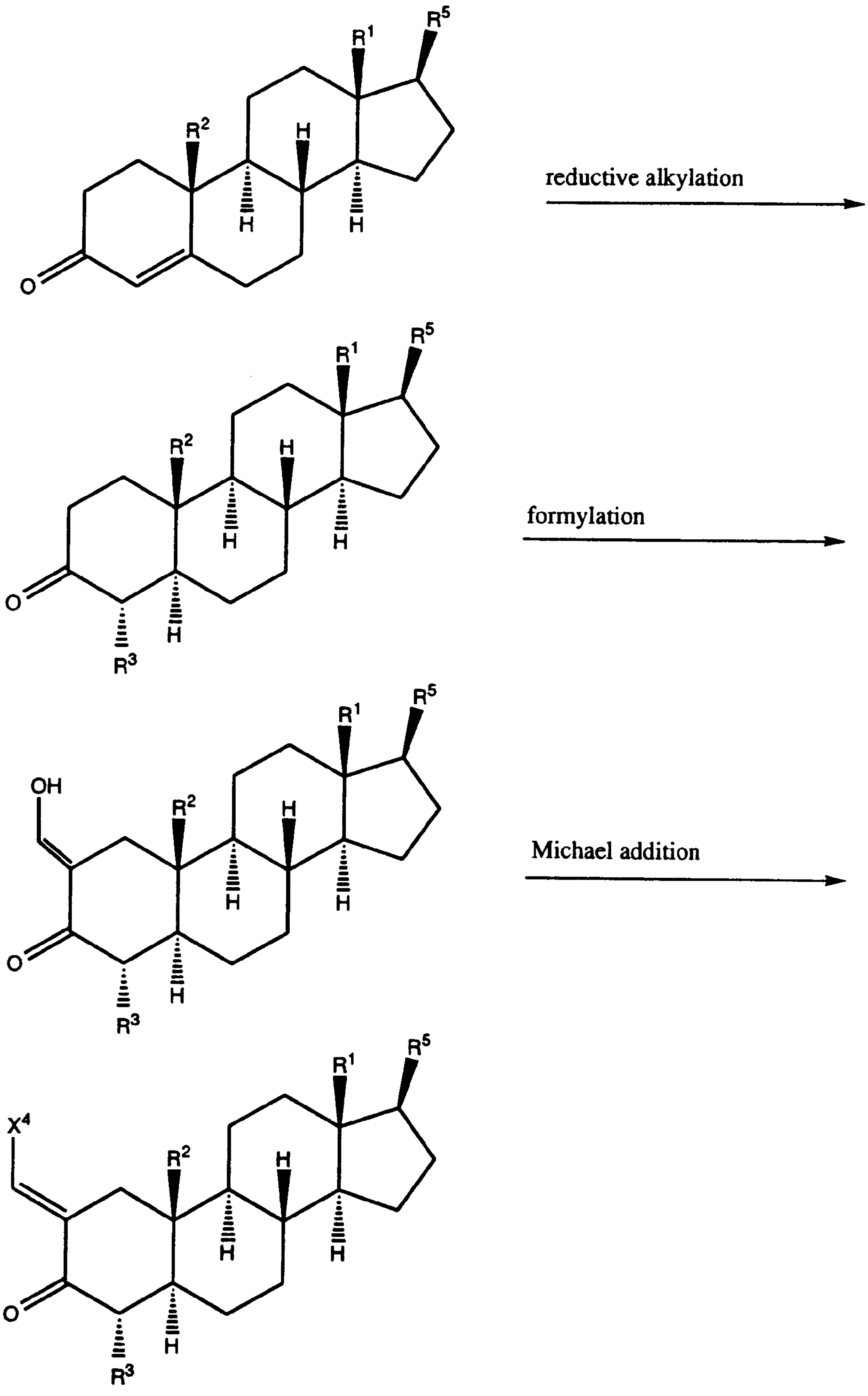

In schemes 1-8, $R^1$, $R^2$, $R^3$, $R^5$, $R^{11}$, $R^{12}$, Z, $R^{10}$, $X^2$, $X^3$ and $X^4$ as defined above for Formula I, unless otherwise noted.

As shown in Scheme 1, step A, a suitable 4-cholesten-3-one is reductively alkylated with a suitable alkyl halide, benzyl halide, or 2-propenyl halide in the presence of a strong base and a proton source in an inert or substantially inert solvent or mixture of solvents to afford the corresponding 4-substituted-cholestan-3-one.

The preferred strong base is an alkali metal, preferably lithium or sodium, in liquid ammonia. Preferably a proton donor is also present and preferably is ethanol, t-butanol and preferably t-butanol. Generally, temperatures of from about -85°C to about -50°C are employed in carrying out this reaction. In step B of Scheme 1, the 3-one compound is reduced to the corresponding α and β isomers through a hydride reduction carried out in an inert or substantially inert solvent or mixture of solvents. Suitable hydride reducing agents include diisobutyl aluminum hydride, potassium tri-sec-butyl- borohydride (K-selectride®) and lithium aluminum hydride. Suitable solvents for this reaction include chlorinated hydrocarbons or etheral solvents. Preferred solvents are etheral and most preferred is tetrahydrofuran (THF). Generally temperatures ranging from about -85°C to about 0°C are employed. The α-hydroxy and β-hydroxy isomers afforded by the step B reaction are generally separated by chromotography although other separation techniques known to those skilled in the art could be employed.

Step C of Scheme 1 illustrates a hydride reduction of the type discussed above for step B and demonstrates such reductions can effect more than one carbonyl group on the molecule, if desired.

Step D of Scheme 1 illustrates further modifications to compounds within the scope of formula I to afford further desired compounds within the scope of formula I. In particular, Scheme D discloses an alkaline hydrolysis employing a suitable base, such as sodium hydroxide, potassium hydroxide, or lithium hydroxide in a mixture of an etheral solvent and an alcohol solvent (generally 4:1(v:v) at about the reflux temperature of the solvent or mixture of solvents.

Step E of Scheme 1 illustrates still another set of reactions which can be used starting with compounds within the scope of formula I to arrive at further compounds of the present invention also within the scope of formula I. In step E the benzyl moiety is removed by hydrogenolysis. Preferably this reaction is carried out in the presence of palladium on carbon in an inert or substantially inert solvent or mixture of solvents, preferably etheral and most preferably THF to afford the corresponding 4α-(4-hydroxybenzyl)cholestan-3-one which is then reduced by a hydride reduction as described above to afford the 3α and 3β isomers which are separated by chromotography.

Scheme 2 illustrates reactions which can be employed to obtain at least those compounds of the present invention containing a 4-substituent and a 4-ene unsaturation. In the first reaction, a suitable 4-cholesten-3-one is reacted with a secondary amine, preferably pyrrolidine (which is illustrated) in an inert or substantially inert solvent or mixture of solvents at about the reflux temperature of said solvent to afford the corresponding 3-pyrrolidino-3,5-cholestadiene. The suitable solvents for this reaction are generally aprotic and benzene is preferred. The 3-pyrrolodino intermediate is then alkylated using a suitable alkyl halide or 2-propenyl halide, preferably bromide or iodide, in an inert or substantially inert solvent or mixture of solvents and then hydrolyzed to afford the corresponding 4-substituted-cholest-4-en-3-one. The preferred solvent for the alkylation reaction is dimethylformamide or dioxane. Preferably a co-solvent is added during the hydrolysis reaction, such as dioxane. The alkylation reaction is carried out at elevated temperatures of from about 150-200°C and the hydrolysis is carried out at temperatures of from about 80°C to about 130°C. The 3-one-4-ene compound may then be reduced through a hydride reduction to the corresponding α-hydroxy and β-hydroxy isomers which are isolated by preferably chromotography.

Although the 17 position of the steroid ring shown as a reactant in step A of Scheme 3 depicts a $R^5$-COOH group, it should be appreciated that the primary focus of this scheme is to illustrate preparation of various compounds within the scope of the present invention having carboxcylic acid, amine, carboxamide and similar type groups within the definition of $X^3$. In step A a suitable compound having a terminal carboxcylic acid group is used as the starting reactant. Said compounds are prepared substantially in accordance with procedures described in the literature such as Miyamoto, et al., Synthetic Communications, 16 No. 5,513-521 (1986) and Demir, et al., Organic Prop. and Proc. International Organic Prep. and Proc., 19 (2-3), 197-208 (1987) which are incorporated herein by reference. The carboxcyclic acid is reacted with a suitable primary or secondary amine salt in the presence of an acid scavenger and a halide source in an inert or substantially inert solvent or mixture of solvents to afford the correponding carboxamide. Suitable acid scavengers for use in this reaction are generally tertiary amines and N-methylmorpholine is preferred. The preferred halide is chloro and butyl chloroformate is preferred. Suitable solvents for this reaction are generally aprotic in nature and chlorinated hydrocarbons are preferred and most particularly methylene chloride. Suitable temperatures for this reaction are from about -25°C to about 25°C. Step B of Scheme 3 shows a reductive alkylation carried out under substantially the same conditions as described above for this reaction.

Similarly, the reduction of the 3-one group to the corresponding 3α-hydroxy compound is carried out with a hydride reducing agent using substantially the same procedures and conditions described above for this reaction.

The carboxamide can be reduced as shown in step D of Scheme 3 to afford the corresponding amino compound by way of a hydride reduction using substantially the same procedures and conditions described above for similar reactions. The preferred reducing agent for this reaction is lithium aluminum hydride in an inert or substantially inert solvent or mixture of solvents at from about -25°C to about 25°C to afford the corresponding amino compound.

Scheme 4 discloses an alternative way of arriving at compounds of the present invention that include a 7-position and/or a 12-position substituent, although it will be appreciated this scheme is not limited to those compounds. In step A of Scheme 4 a 3α-hydroxy compound having a terminal carboxcylic acid group is reacted with a haloformic ester in the presence of an acid scavenger in an inert or substantially inert solvent or mixture of solvents to afford the corresponding dicarbonate which is further reacted with a primary or secondary amine salt, without isolation, to afford the corresponding 3-position carbonate having a terminal carboxamide group as the $X^3$ group at the $R^5$ position. The preferred haloformic ester is isobutylchloroformate. Generally, tertiary amines are used as acid scavengers and the most preferred acid scavenger is N-methylmorpholine. Suitable solvents for this reaction are chloronated hydrocarbons and methylene chloride is most preferred. Temperatures employed in the dicarbonate formation reaction are generally from about -20°C to about 30°C.

Step B of Scheme 4 shows the 3-position carbonate group being reduced by a hydride reduction using the conditions and procedures previously described for this reaction. It should be appreciated that, if desired, the carbonate group could also be hydrolyzed. The preferred reducing agent for this step B reaction is lithium aluminum hydride.

Scheme 5 is primarily directed toward those compounds of the present invention where A is an oxygen atom, although it will be appreciated many other compounds within the scope of the present invention can be afforded by this scheme. The first reaction comprises a cyclic ketalization exemplified is the reaction of testosterone with ethylene glycol in the presence of an acid catalyst, preferably an organic acid, in an inert or substantially inert solvent or mixture of solvents to afford the corresponding 3,3-ethylenedioxy compound along with the corresponding 3,3-ethylenedioxy-5-ene isomer. The preferred organic acid catalyst is p-toluenesulfonic acid monohydrate. The reaction is generally carried out at about the reflux temperature of the solvent. Suitable solvents are those that allow azetropic removal of water such as inert aromatic solvents and preferred is toluene.

The next step is to alkylate the 3,3-ethylenedioxy isomers with the desired alkyl halide or alkenyl (e.g., 2-propenyl) halide in the presence of a strong base in an inert or substantially inert solvent or mixture of solvents to afford the corresponding 17-alkoxy or alkenyloxy compound. Suitable strong basis for this reaction are hydrides and preferred is potassium hydride. Preferred solvents for this reaction are a mixture of THF and dimethylsulfoxide (DMSO) or dimethylformamide (DMF). Suitable temperatures for this reaction are generally from about -20°C to about 50°C.

The next step illustrated is to regenerate the 4-en-3-one group. The 4-en-3-one group is formed by acidifying the 3,3-ethylenedioxy compound in an inert or substantially inert solvent or mixture of solvents. The useful acids are preferably organic acids with acetic acid being most preferred. Generally water is also included along with the acid. Suitable solvents for this reaction are etheral with THF being preferred. Suitable temperatures for this reaction are generally from about 50°C to about 100°C.

Further compounds of the present invention can be afforded by reductively alkylating the 4-en-3-one compound using the procedures described above for this reaction. Still further compounds of the present invention can be afforded by reducing the alkylated intermediate by way of a hydride reduction, also in accordance with the procedures previously described. Preferably, this hydride reduction is accomplished using K-selectride although sodium borohydride is also a useful reducing agent.

Scheme 6 illustrates an alternative synthesis primarily for those compounds of the present invention where A is an oxygen atom. The first step of this scheme shows reacting testosterone, or other suitable starting material with t-butyldimethylsilyl chloride using standard oxygen silylation conditions known to those skilled in the art. The next step in this scheme shows a reductive alkylation in accordance with the procedures previously described for this reaction followed by a hydride reduction, again, using procedures previously described for this reaction. The next step is protecting the 3-hydroxy group with a tetrahydropyran-4-yl (THP) group using standard THP forming conditions known to those skilled in the art. This is followed by deprotection of the t-butyldimethylsilyl (TBS) group using standard desilylation conditions again known to those skilled in the art.

The next step in this scheme is alkylation using an appropriate alkyl halide or 2-propenyl halide in the presence of a strong base in an inert or substantially inert solvent or mixture of solvents. These alkylation conditions are described above. The final synthetic step shown in this scheme is deprotection of the 3-position using standard THP cleaving conditions well known to those skilled in the art.

The reactions shown in Scheme 7 are primarily directed toward those compounds of the present invention where Z is an oxygen atom. The first step in this reaction shown oxidation of an methyl ketone to its corresponding carboxcylic acid using the bromoform reaction. Generally this reaction comprises reacting the methyl ketone compound in mixture of bromine, water and a suitable base, preferably sodium hydride, at a temperature of from about -20°C to about 20°C. It is preferred that a co-solvent such as dioxane be added to improve solubility. The next step in this scheme is a hydride reduction using the procedures previously described for this reaction. Preferably a strong hydride reducing agent is employed for this reduction such as Red-Al®. This reaction affords the corresponding 3-hydroxy 17-hydroxymethyl intermediate. This intermediate is then selectively oxidized using manganese dioxide in an inert or substantially inert solvent or mixture of solvents to afford the corresponding 3-one intermediate. Preferred solvents for this reaction are chlorinated hydrocarbons and chloroform is most preferred. The 20-ol intermediate is then silylated, preferably with TBS, using standard conditions known to those skilled in the art for this reaction. The next step is a reductive alkylation again using the procedures and conditions previously described for this reaction followed by a hydride reduction also using the procedures previously described for this reaction. The next step is then a protection of the 3-hydroxy group using standard THP ether formation conditions known to those skilled in the art. This is followed by disilylation, that is deprotection, using standard conditions known to those skilled in the art for this reaction. The hydroxy group is then alkylated using the alkylation conditions previously described for this reaction with a suitable alkyl or alkenyl (e.g., 2-propenyl) halide alkylating reagent in the presence of a strong base in an inert or substantially inert solvent or mixture of solvents. The THP group is then cleaved using standard conditions for this reaction known to those skilled in the art.

Scheme 8 is primarily directed toward those compounds of the present invention having a 2-position substituent. Generally, a suitable compound is reductively alkylated to afford a substituent in the 4-position. this reductive alkylation is carried out using the conditions and procedures previously described for this reaction. The next step shown in Scheme 8 is formylation. Generally the compound is reacted with a hydride and ethylformate in an inert or substantially inert solvent or mixture of solvents, preferably toluene, to afford the 2-position hydroxy methylene group. Suitable solvents for this reaction are generally aromatic hydrocarbons and preferred is toluene. The 2-hydroxy methylene compound may then be further reacted such as by the illustrated Michael addition reaction to afford still further compounds within the scope of the present invention. The Michael addition is carried out using standard conditions for this reaction known to those skilled in the art.

It will be appreciated that by the reactions illustrated in Schemes 1-8, and combinations of those reactions, one skilled in the art can prepare the compounds of Formula I.

An additional synthesis scheme 9 is shown below for the preparation of the compound of Example 85 (3α,4α,5α,20β)-4-(3,3-difluoro-2-propenyl)cholestan-3-ol using the compounds prepared in Example 5, (3α,4α,5α)-4-(2-propenyl)cholestan-3-one Example 83 (3α,4α,5α)-3-[[(1,1-dimethylethyl)dimethylsilyl]oxy]cholestane-4-acetaldehyde, Example 82 (3α,4α,5α)-3-[[(1,1-dimethylethyl)dimethylsilyl]oxy]cholestane-4-acetaldehyde, and Example 84 (3α,4α,5α,20β)-4-(3,3-difluoro-2-propenyl)cholestan-3-ol.

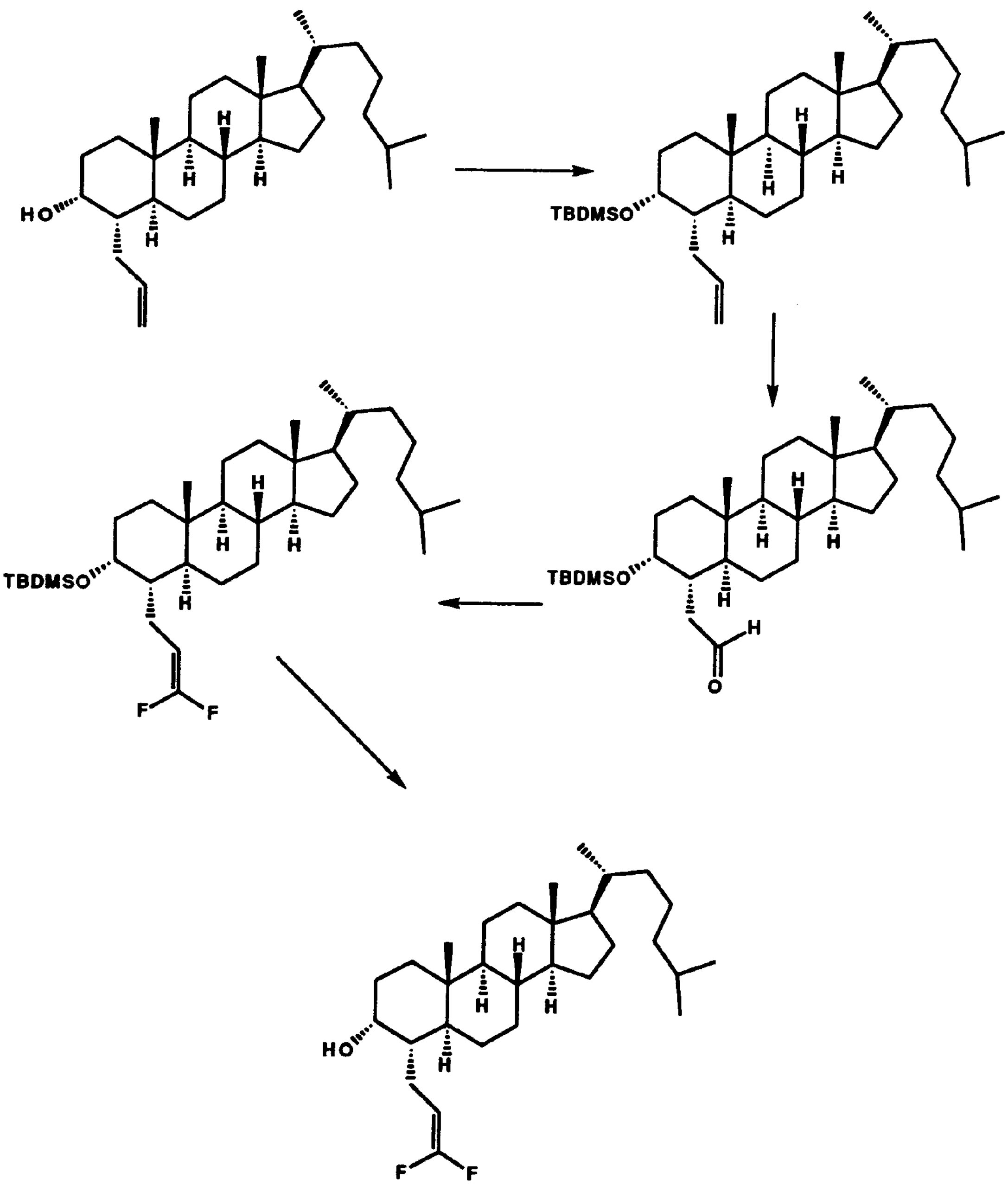

As noted above, the optically active diastereomers of the compounds of Formula (I) are considered part of this invention. Such optically active isomers may be prepared from their respective optically active precursors by the procedures described above, or by resolving the racemic mixtures. The resolution can be carried in the presence of a resolving agent, by chromatography or by repeated crystallization or by some combination of these techniques which are known to those skilled in the art. Further details regarding resolutions can be found in Jacques, et al., Enantiomers, Racemates, and Resolutions, John Wiley & Sons 1981.

The compounds employed as initial starting material in the synthesis of the compounds of this invention are well known and, to the extent not commercially available, are readily synthesized by standard procedures commonly employed by those of ordinary skill in the art.

The pharmaceutically acceptable salts of the invention are typically formed by reacting a compound of Formula I with an equimolar and excess amount of acid or base. The reactants are generally combined in a mutual solvent, such as diethylether or benzene, for acid addition salts, or water or alcohols for base addition salts, and the salts normally precipitate out of solution within about 1 hour to about 10 days and can be isolated by filtration or other conventional methods.

In addition, some of the compounds of Formula I may form solvates with water or with common organic solvents. Such solvates are included within the scope of the compounds of the present invention.

Pharmaceutical Compositions Using a Combination of Hypocholesteremic and/or Hypolipemic Agents

The compounds of this invention, as represented by Formula I, may advantageously be used in combination with other hypocholesteremic and/or hypolipemic agents. Typical of agents useful in pharmaceutical compositions in combination with those of Formula (I) are the following:

(a) Bile Acid Sequestrants - these agents bind bile acids in the intestinal tract and enhance their excretion. Typical bile sequestrants are quaternary amines such as cholestyramine and colestipol.

(b) Nicotinic Acid and Its Derivatives - these B-vitamin inhibit the production of lipoproteins by the liver.

(c) HMG-CoA Reductase Inhibitors - these serum cholesterol lowering drugs inhibit the rate-limiting enzyme in cholesterol synthesis. Typical of drugs in this group are mevastatin, pravastatin, and simvastatin.

(d) Gemfibrozil and Other Fibric Acids - these agents are lipid-lowering drugs. Typical of drugs in this group are gemfibrozil, clofibrate, fenofibrate, benzafibrate and ciprofibrate.

(e) Probucol - this agent is used for cholesterol lowering and appears to prevent the oxidation of LDL. The mechanism of action is uncertain.

(f) Raloxifene and its Derivatives - raloxifene (CAS Registry No. 84449-90-1, 6-hydroxy-2-(4-hydroxyphenyl)-3-[4-(2-piperidinoethoxy)benzoyl]benzothiophene), and its esters and ethers are described in U.S. Patent No. 4,418,068, the disclosure of which is incorporated herein by reference.

(g) mixtures of (a), (b), (c), (d), (e), and (f). These agents have the ability to lower serum cholesterol levels in combination with attendant beneficial effects such as reduced bone loss in post-menopausal women.

Without being bound by any theory of operation, it is believed that the lipid and cholesterol lowering effects of the compounds of Formula (I) of this invention are achieved by interruption of the interaction of oxysterol with regulatory protein, which, in turn, suppresses the activity of the gene encoding the LDL receptor by affecting the promotor region of the gene. The prior art cholesterol and lipid control agents are believed to promote their beneficial effects by other mechanisms.

For example, a pharmaceutical composition may be formed from the compound of Example 5 in combination with mevastatin and/or raloxifene together with suitable excipients and carriers.

The multi-mode pharmaceutical compositions containing a compound of Formulae I, II, III, IV or V with cholesterol control agents (a), (b), (c), (d), (e) or (f) are formulated in a manner to avoid harmful or antagonistic combinations of ingredients known in the art.

Alternatively, a combination of ingredients may be used in a method of preventing cholesterol induced atherosclerosis by administering to a mammal simultaneously, or in any order (1) the compound of the invention, and (2) one or more agents (a), (b), (c), (d), (e), (f), or any combination thereof as previously described. The practive of this therapeutic method only requires that ingredients (1) and (2) be administered to a mammal in a time period where they jointly produce a therapeutic effect.

The following examples further illustrate the compounds of the present invention and methods for their synthesis. The Examples are not intended to be limiting to the scope of the invention in any respect and should not be so construed.

Unless otherwise noted, NMR data appearing in the examples refers to the free base of the subject compound.

Example 1

Preparation of [4α(E),5α]-4-(2-butenyl)cholestan-3-one

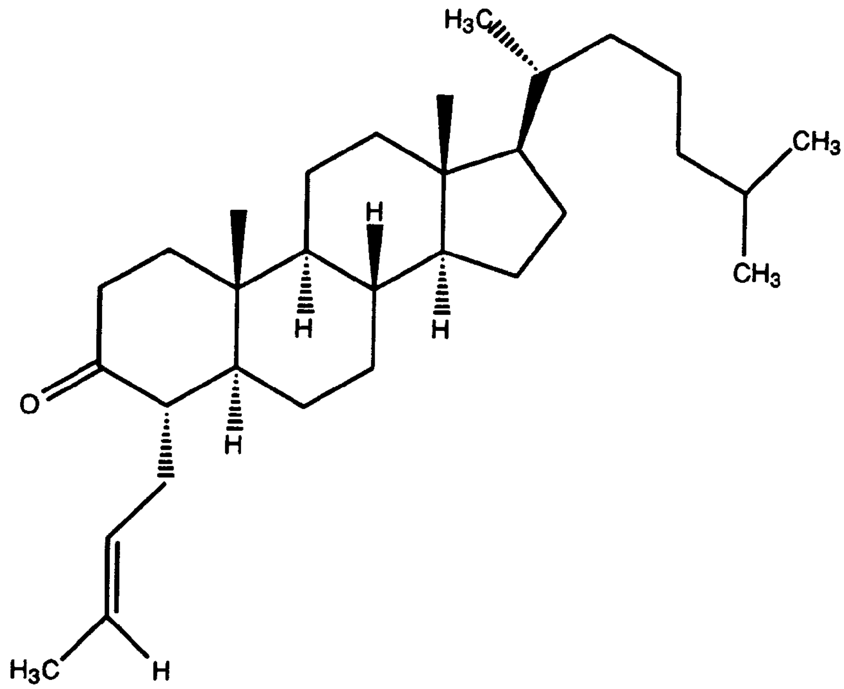

Lithium wire (1.2 g, 0.171 mol) was placed in a flame dried flask fitted with a mechanical stirrer and dry ice condenser under nitrogen. A dry ice/isopropanol bath was used to cool the flask while 60 - 70 mL of liquid ammonia (distilled from Li) was added. The mixture was slowly stirred to provide a uniform slurry to which was added with rapid stirring 50 mL of dry THF to form a bronze precipitate. To the lithium bronze in THF was added dropwise at a rapid rate a solution of (+)-4-cholestene-3-one (30 g, 0.078 mol, Aldrich 18,817-4) in 200 mL dry THF and t-butanol (7.2 mL, 0.078 mol) resulting in formation of a yellow precipitate. Upon completion of the addition, the cooling bath was removed for 5 minutes. Isoprene (5.3 g, 0.078 mol) was added, and the resulting mixture stirred for an additional 5 minutes. Crotyl bromide (52.6 g, 0.390 mole, Aldrich C8,640-5) was rapidly added. The reaction mixture was stirred an additional 10 minutes under the dry ice/isopropanol reflux condenser. The mixture was cooled before cautiously adding solid ammonium chloride at a rate that did not cause the exothermic decomposition to foam out the dry ice condenser. The cooling bath was removed, the reaction mixture stirred for approximately 30 minutes and water (250 mL ) was added. Separation of the organic layer was followed by back extraction of the water layer with ethyl acetate. The combined organic extracts were dried ($MgSO_4$) and concentrated to afford an oil which was further purified by prep HPLC using a gradient of 0-8% ethylacetate in hexane over 30 minutes and collecting 375 mL fractions every 1.5 minutes. Fractions containing the product were combined and evaporated to give a white solid which was recrystallized from ethanol/water to give 18.75 g (55%) of the title compound: 1H NMR ($CDCl_3$, 300 MHz) d 5.55 - 5.30 (m, 2H), 2.50 - 1.95 (m, 5H), 1.64 (m, 3H), 1.05 (s, 3H), 0.87 (m, 9H), 0.68 (s, 3H).

By following the procedures described above in Example 1, the compounds of Examples 2 and 3 were prepared.

Example 2

Preparation of [4α,5α]-4-(1-propenyl)cholestan-3-one

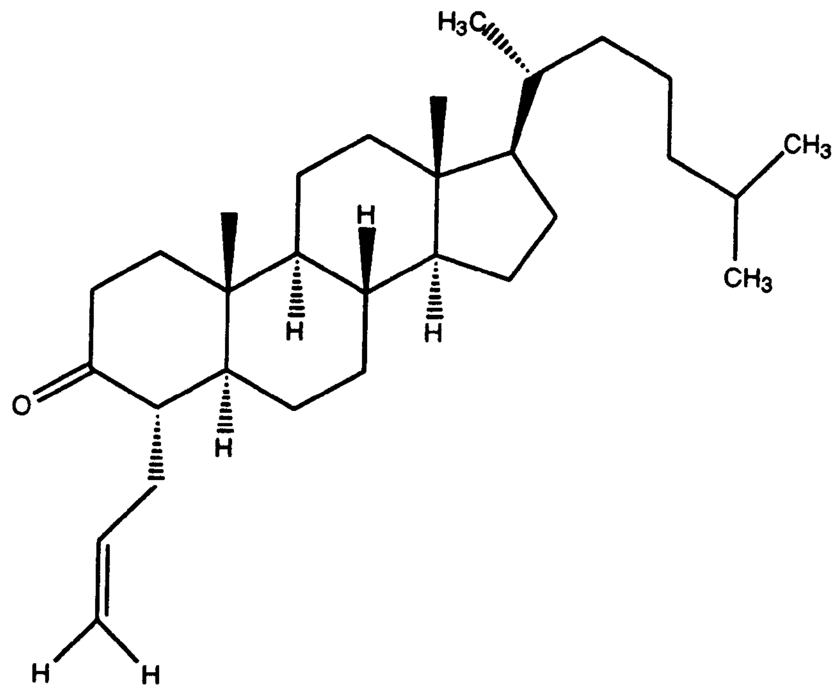

Lithium (2.6 g, 0.378 mol), (+)-4-cholestene-3-one (66.12 g, 0.172 mol) and allyl bromide (62.4 g, 0.516 mol, Aldrich A2,958-5) provided 27.33 g (37%) of the title compound as a white crystalline solid: MS/FD m/e 426; 1H NMR ($CDCl_3$, 300 MHz)

Example 3

Preparation of [4α(E),5α]-4-(2-butenyl)-25-hydroxycholestan-3-one

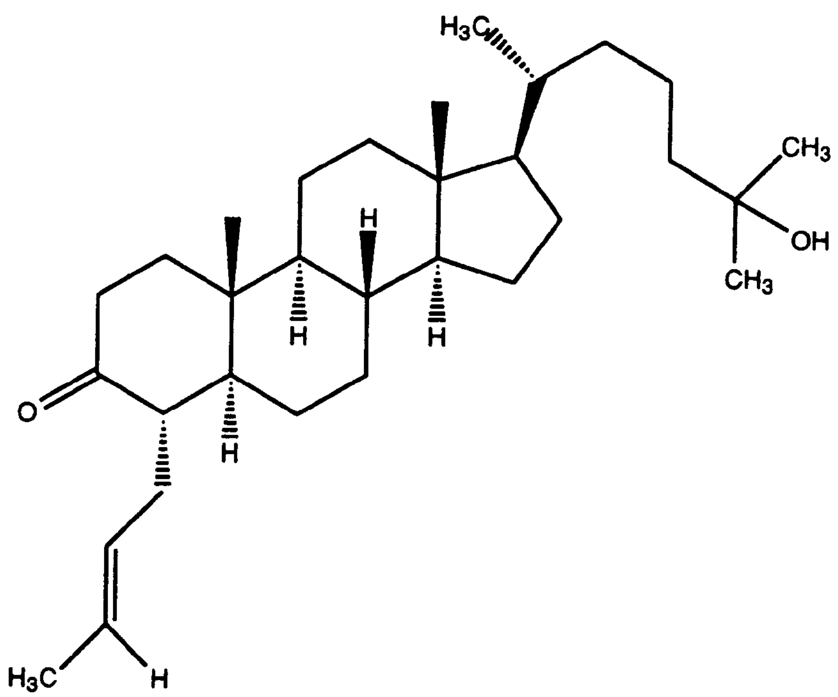

Lithium (20.5 mg, 2.98 mmol), 25-hydroxy-(+)-4-cholestene-3-one [500 mg, 1.35 mmol, obtained from Oppenauer Oxidation of 25-hydroxycholesterol (Steraloids Inc. C 6510)] and crotyl bromide (1.1 g, 8.1 mmol, Aldrich C8,640-5) provided 167.2 mg (27%) of the title compound as a white solid: MS/FD m/e 457; 1H NMR ($CDCl_3$, 300 MHz)

Example 4

Preparation of [4α(E),5α]-4-(2-butenyl)cholestan-3α-ol:

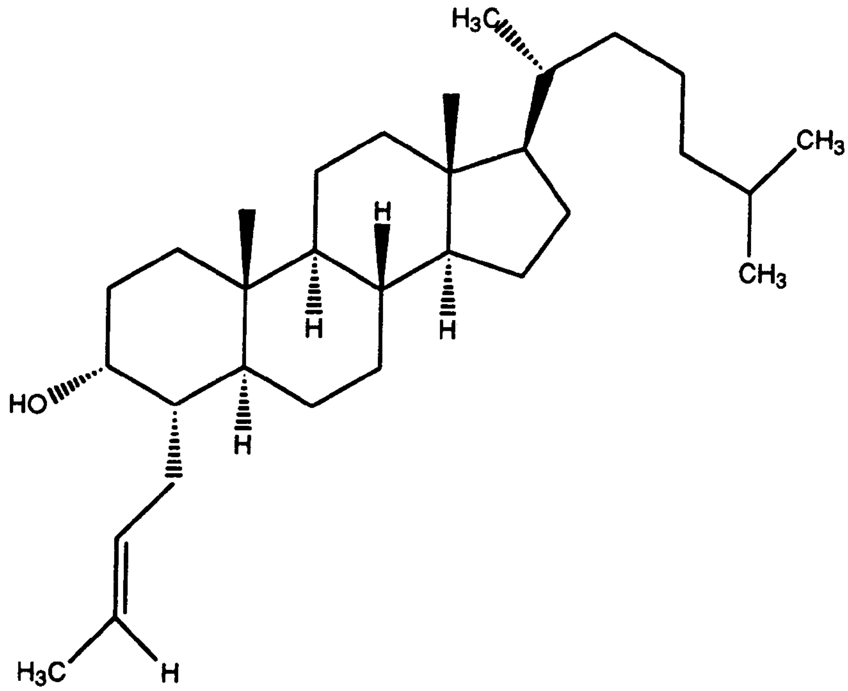

A solution of 7.6 g (17 mmol) of the compound of Example 1 in 75 mL of dry THF was added dropwise to 34 mL (2 equivalents) of K-Selectride (1M) in THF at -78°. The reaction was strirred for 2.5 hr then warmed to 0°, followed by addition of 3.11 mL of water, 11.75 mL 2B ethanol and 9.31 mL of 5N sodium hydroxide. Then 11.75 mL of 30% hydrogen peroxide was added dropwise keeping the temperature below 20°. After coming to room temperature, the solution was evaporated under reduced pressure to remove the THF. The aqueous layer was extracted with ether, and the organic extracts were dried ($MgSO_4$) and concentrated to afford 7.43 g (97%) of a yellow foam which was further purified by prep HPLC using a gradient of 0-8% ethylacetate in hexane over 30 minutes and collecting 375 mL fractions every 1.5 minutes. Fractions containing the product were combined and evaporated to give a white solid which was recrystallized from ethanol/water to give 6.77 g (90%) of the title compound: mp 108-109°; MS/FD m/e 442; 1H NMR ($CDCl_3$, 300 MHz)

By substantially following the procedures of Example 4, the compounds of Examples 5, 6, and 7 were prepared.

Example 5

Preparation of [4α,5α]-4-(2-propenyl)cholestan-3α-ol:

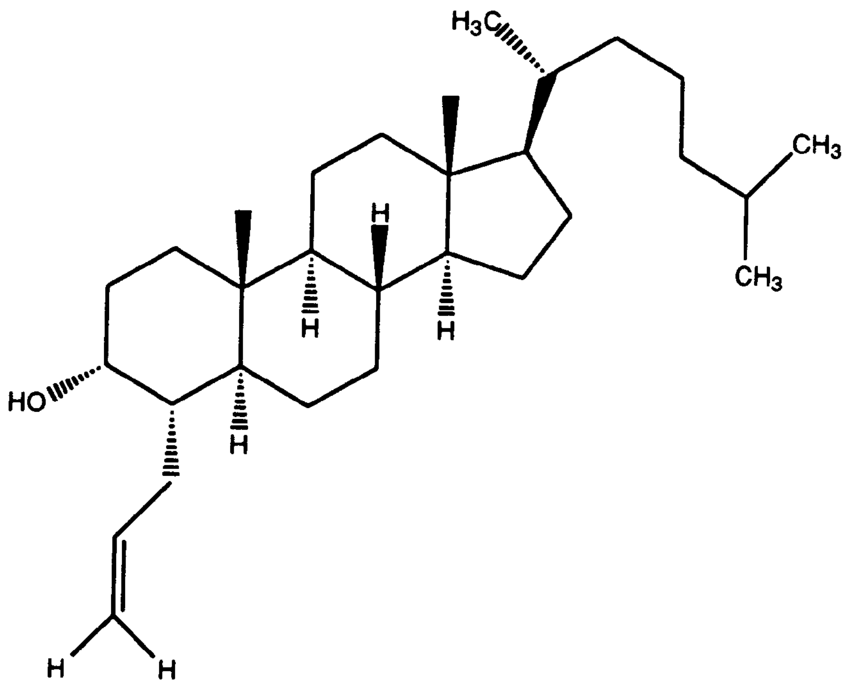

The compound of Example 2 [4α,5α]-4-(1-propenyl)cholestan-3-one [27.33 g, .064 mol] provided 34.5 g (90%) of the title compound as a white crystalline solid: MS/FD m/e 428; 1H NMR ($CDCl_3$, 300 MHz)
1H NMR ($CDCl_3$, 300 MHz)

Example 6

Preparation of [4α(E),5α]-4-(2-butenyl)-25-hydroxycholestan-3α-ol

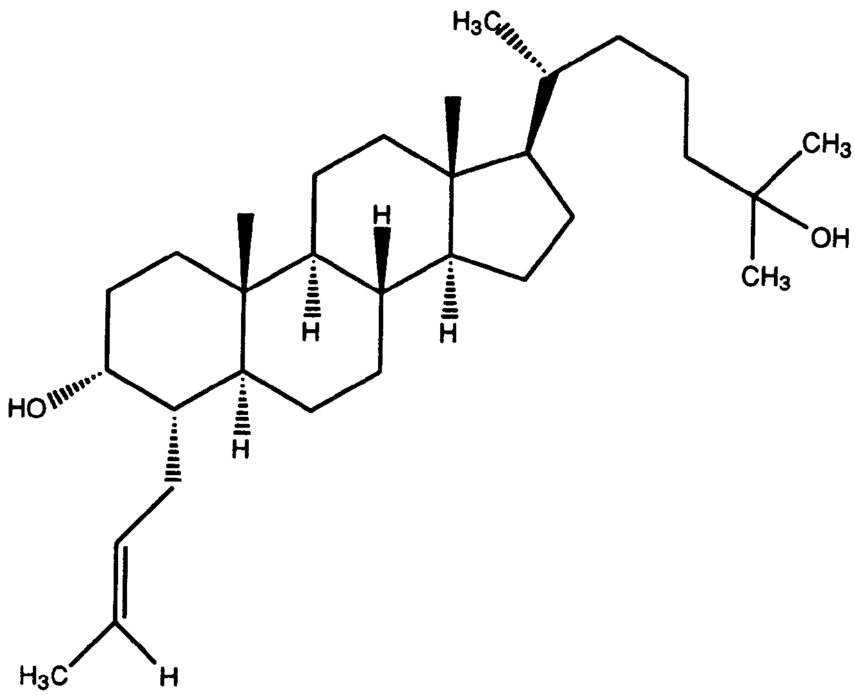

The compound of Example 3 [4α(E),5α]-4-(2-butenyl)-25-hydroxycholestan-3-one [140 mg, .307 mmol] provided, after flash chromatography (EtOAc), 28.1 mg (21%) of the title compound as a white solid: MS/FD+ m/e 428; 1H NMR ($CDCl_3$, 300 MHz)

Example 7

Preparation of [4α,5α]-4-butylcholestan-3α-ol

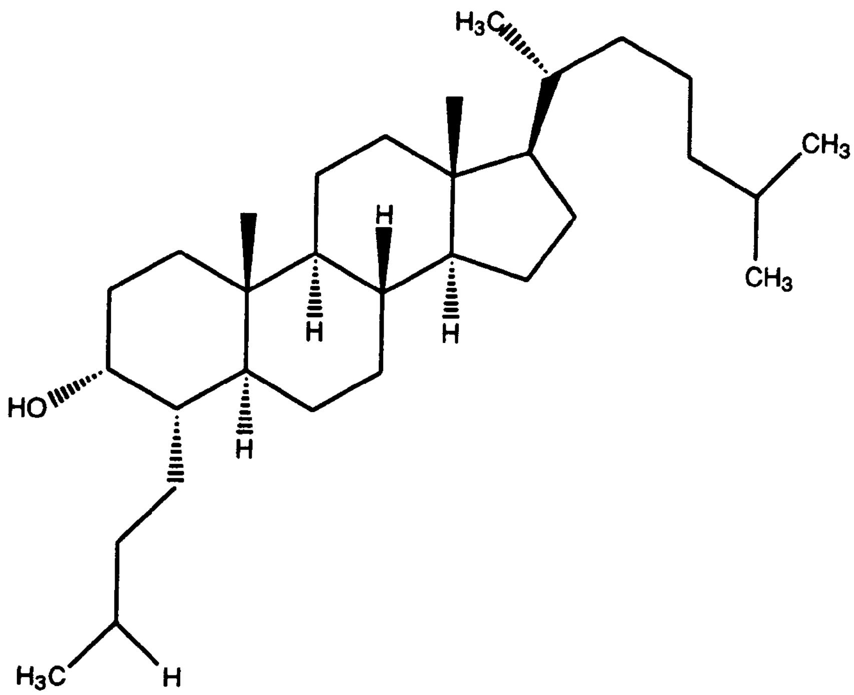

A mixture of 533 mg (1.2 mmol) of the compound of Example 4 and 53 mg of 5% Pd/C in 50 mL of ethyl acetate was subjected to 60 psi of hydrogen at room temperature for 8 hours. Filtration of the reaction mixture over Fuller's earth followed by evaporation gave 436 mg (82%) of the title compound as a white solid whose purity was indicated by one spot on TLC (10% EtOAc:hexane): MS/FD m/e 444; 1H NMR ($CDCl_3$, 300 MHz)

Example 8

Preparation of [4α(E), 5α]-4-(2-butenyl)-3α-aminocholestone

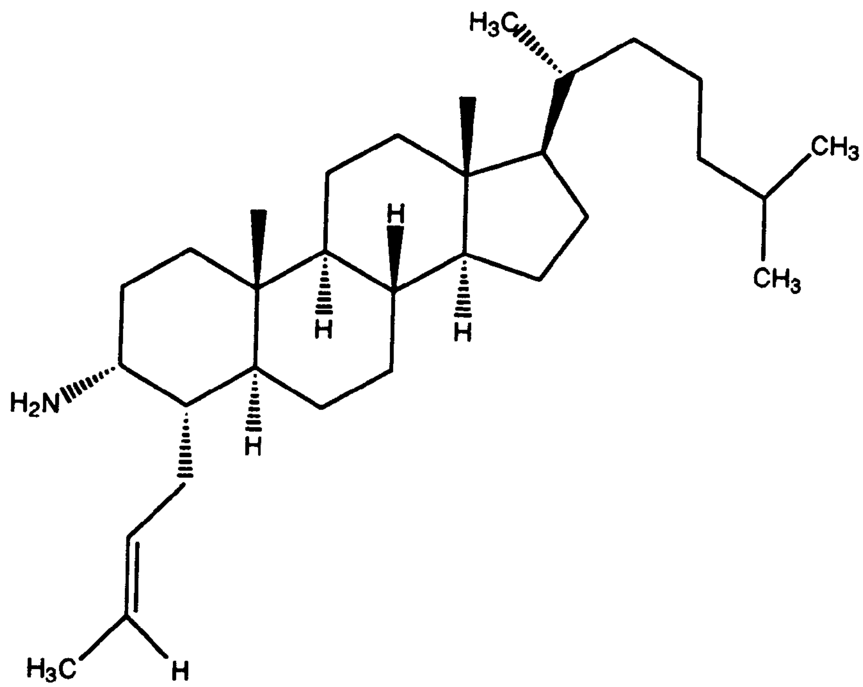

To a mixture of 5.0 g (11.3 mmol) the compound of Example 1, 8.7 g (113 mmol, 10 equiv) ammonium acetate, 4.0 g (63.7 mmol, 5.6 equiv) sodium cyanoborohydride and 70 mL of dry THF in a flame dried flask under nitrogen was added 70 mL of methanol. The solution was stirred at room temperature overnight, followed by addition of 5% sodium hydroxide. The aqueous solution was extracted with ether, the organic extracts combined, dried (MgSO4) and concentrated to afford 4.95 g (99%) of the title compound as a glassy semi solid.

<u>Examples 9 and 10</u>

Preparation of [4α(E)5α]-4-(2-butenyl)-3α and 3β-acetamidocholestones

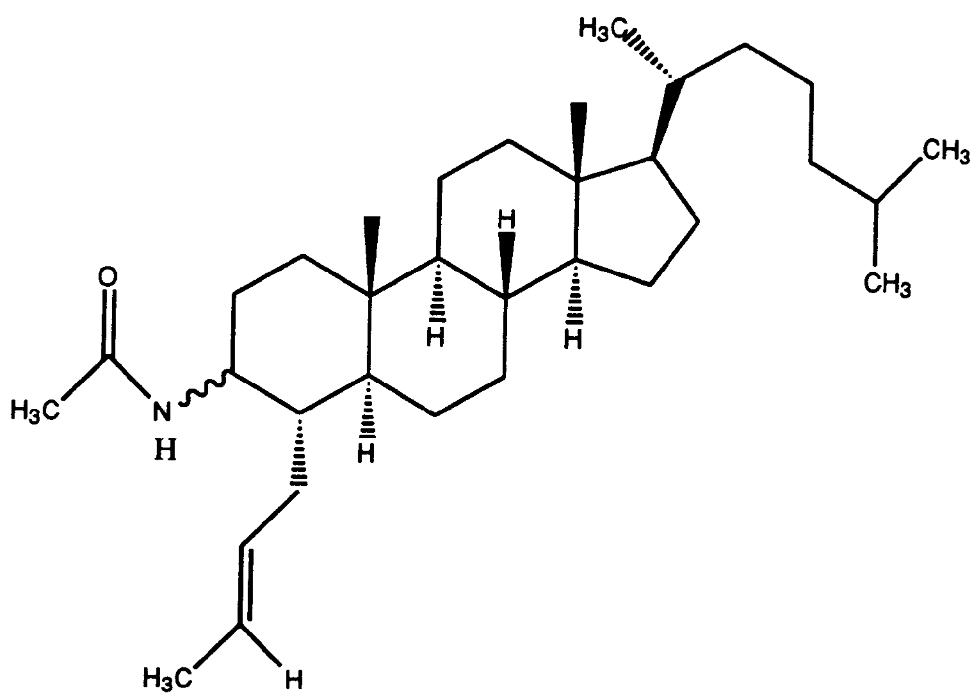

A mixture of 4.0 g (9.05 mmol) of the compound of Example 8, 2.3 g (2.5 equiv) of acetic anhydride and 3.6 mL (5.0 equiv) of pyridine in 50 mL of toluene was heated at reflux for 1 hour. After cooling to rt, the mixture was concentrated to afford a white solid which was further purified by HPLC using a gradient of 15-50% EtOAc:hexane over 20 minutes. Fractions containing products were combined and evaporated to give the individual 3-acetamidocholestane isomers as white solids. Example 9: 1.08 g (24.6% of the 3α isomer MS/FD m/e 483; 1H NMR ($CDCl_3$, 300 MHz) Example 10: 1.31 g (30%) of the 3β isomer: MS/FD m/e 483; 1H NMR ($CDCl_3$, 300 MHz)

Example 11

Preparation of 4α-(4-cyanobenzyl)cholestan-3-one.

A flame-dried, three necked flask equipped with an ammonia inlet, a dry ice condenser and a septum was charged with a glass stir bar and lithium chips (79.4 mg, 11.4 mmol) under an argon atmosphere. 30 ml dry ammonia was collected in the flask which was in a dry ice/acetone bath. The resulting deep blue solution was stirred for 10 min. before it was diluted with 10 ml dry tetrahydrofuran.

A solution of 4-cholesten-3-one (2.00g, 5.20 mmol) and tert-butyl alcohol (340 μl, 3.64 mmol) in 15 ml dry tetrahydrofuran was added dropwise to the deep blue solution with vigorous stirring over a 3 min period at -78°C. The light blue solution was stirred at -78°C for 45 min. A solution of α-bromo-p-tolunitrile (3.05g, 15.6 mmol). in 20 ml dry tetrahydrofuran was added to the blue solution in a fast fashion via a cannula. The dry ice/ acetone bath was removed and the resulting greenish suspension was allowed to warm slowly with evaporation of ammonia for 3 hr. A 10 ml aqueous ammonium chloride (588 mg, 11.0 mmol) solution was added to the reaction mixture, followed by 30 ml diethyl ether/methylene chloride (2:1); and the organic layer was separated. The aqeous layer was extracted with diethyl ether/methylene chloride (2:1) (50 ml x 2). The combined organic layers were washed with 20 ml brine, dried over anhydrous magnesium sulfate, filter and concentrated to obtain a white solid residue, which was subject to flash column chromatography on silica gel (ethyl acetate/ toluene 0 → 4% as eluent) to obtain 1.28g (2.55 mmol 49%) 4α-(4-cyanobenzyl)cholestan-3-one. Recrystallization in toluene gave white needle crystal.

IR($CHCl_3$, $cm^{-1}$): 2230, 1706

300MHz $^1H$ NMR($CDCl_3$, ppm) δ 0.68(s,3H, $CH_3$), 0.87 (d,J=6.6Hz,6H, $-CH(CH_3)_2$), 0.91(d,J=6.5Hz,3H, $-CH(CH_3)-$), 1.09(s,3H, $-CH_3$), 0.70-1.65(m,22H), 1.70-1.95(m,3H), 1.95-2.10(m, 2H), 2.26-2.35(m,1H), 2.44(td, J=14.0 Hz, 6.2Hz, 1H), 2.54-2.64 (m, 1H), 2.87 (dd, J=14.2Hz; 3.1Hz, 1H), 3.02(dd,J=14.2 Hz; 7.8Hz, 1H), 7.31(d,J=8.2Hz, 2H)

Mass(FD):501($M^+$);

| Elementary Anal.: Calcd for $C_{35}H_{51}ON$: | | | |
|---|---|---|---|
| | C, 83.78; | H, 10.24, | N, 2.79; |
| Found: | C, 84.07; | H, 10.42, | N 2.86 |

By substantially following the procedures described above for Example 11, the compounds of Example 12 through 21 were prepared.

Example 12

Preparation of 4α-benzylcholestan-3-one

16.9% yield; mp 163.0-168°C ($CH_2Cl_2/CH_3CN$);

IR($CHCl_3$) 1704 $cm^{-1}$;

NMR 300MHz ($CDCl_3$) δ 0.65(s,3H, methyl), 0.85(d,J=6.6Hz,6H), 0.88(d,J=6.6Hz,3H.), 0.70-1.60(m,22H), 1.04(s,3H,methyl), 1.65-1.90(m,3H), 1.90-2.06(m,2H), 2.25-2.36(m,1H), 2.42(td,J=1.39, 6.3Hz, 1H), 2.50-2.59(m,1H), 2.80(dd,J=14.3,3.2Hz, 1H), 3.01(dd,J=14.2, 7.2Hz,1H), 7.10-7.28(m,5H, aromatic);

MS-FD M/e 476($M^+$);

Anal. Calcd for $C_{34}H_{52}O$:

C, 85.65; H, 10.99

Found: C, 85.94; H, 11.37.

Example 13

Preparation of 4α-(4-fluorobenzyl)cholestan-3-one
19.7% yield; mp 175.0-177.0°C ($CH_2Cl_2/CH_3CN$);
IR($CHCl_3$) 1704 $cm^{-1}$;
NMR 300MHz ($CDCl_3$) δ 0.65(s,3H, methyl), 0.85(d,J=6.5Hz,6H), 0.88(d,J=6.5Hz,3H.), 0.65-1.60(m,22H), 1.04(s,3H,methyl), 1.70-1.84(m,3H), 1.94-2.02(m,2H), 2.26 (brd. J=11.8 Hz, 1H), 2.38 (dd, J=13.8, 6.4 Hz, 1H), 2.44-2.55 (m,1H), 2.8 (br dd, J-14.3, 3.0 Hz, 1H), 2.92(dd, J=1.42, 7.4 Hz, 1H), 6.89 (t,J=8.7 Hz, 2H aromatic), 7.10-7.14 (m,2H aromatic);
MS-FD M/e 494($M^+$);

| Anal. Calcd for $C_{34}H_{51}FO$: | | |
|---|---|---|
| | C, 82.54; | H, 10.39 |
| Found: | C, 82.63; | H, 10.46. |

Example 14

Preparation of 4α-(4-bromobenzyl)cholestan-3-one
24% yield; mp 163.0-165.8°C ($CH_2Cl_2/CH_3CN$);
IR($CHCl_3$) br 1705 $cm^{-1}$;
NMR 300MHz ($CDCl_3$) δ 0.68(s,3H, methyl), 0.87(d,J=6.6Hz,6H), 0.91(d,J=6.5Hz,3H.), 0.70-1.60(m,22H), 1.07(s,3H,methyl), 1.72-1.92(m,3H), 1.97-2.08(m,2H), 2.29 (br d, J=9.1 Hz, 1H), 2.41 (dd, J=15.2, 7.1 Hz, 1H), 2.52-2.56 (m,1H), 2.79 (dd, J-14.2, 3.1 Hz, 1H), 2.93(dd, J=1.42, 7.4 Hz, 1H), 7.07 (d,J=8.2 Hz, 2H aromatic), 7.35(d,J=8.3Hz,2H aromatic);
MS-FD M/e 556($M^+$);

| Anal. Calcd for $C_{34}H_{51}BrO$: | | |
|---|---|---|
| | C, 73.49; | H, 9.25 |
| Found: | C, 73.72; | H, 9.28 |

Example 15

Preparation of 4α-(4-iodobenzyl)cholestan-3-one
17.3% yield; mp 161.5-163.0°C ($CH_2Cl_2/CH_3CN$);
IR($CHCl_3$) 1705 $cm^{-1}$;
NMR 300MHz ($CDCl_3$) δ 0.68(s,3H, methyl), 0.87(d,J=6.6Hz,6H), 0.90(d,J=6.8Hz,3H.), 1.07(s,3H,methyl), 0.70-1.60(m,22H), 1.71-1.86(m,3H), 1.97-2.05 (m,2H), 2.29 (br d, J=11.8 Hz, 1H), 2.43 (td, J=13.9, 6.4 Hz, 1H), 2.49-2.58 (m,1H), 2.78 (dd, J=14.2, 2.8 Hz, 1H), 2.92(dd, J=14.2, 7.3 Hz, 1H), 6.95(d, J=8.2 Hz, 2H aromatic), 7.55(d,J=8.2Hz,2H aromatic);
MS-FD M/e 602($M^+$);

| Anal. Calcd for $C_{34}H_{51}IO$: | | |
|---|---|---|
| | C, 67.76; | H, 8.53 |
| Found: | C, 67.98; | H, 8.59 |

Example 16

Preparation of 4α-(4-trifluoromethylbenzyl)cholestan-3-one

22% yield; mp 165.0-166.0°C ($CH_2Cl_2/CH_3CN$);
IR($CHCl_3$) 1705 $cm^{-1}$;
NMR ($CDCl_3$) δ 0.68(s,3H, methyl), 0.87(d,J=6.6Hz,6H), 0.91(d,J=6.5Hz,3H.), 1.09 (s,3H, methyl) 0.70-1.60(m,22H), 1.73(m,22H), 1.73-1.87(m,3H), 1.96-2.07(m,2H), 2.31 (br d, J=12.0 Hz, 1H), 2.44 (td, J=14.0, 6.4 Hz, 1H), 2.56-2.63 (m,1H), 2.86 (br dd, J-14.2, 3.0 Hz, 1H), 3.05(dd, J=14.2, 7.6 Hz, 1H), 7.31 (d,J=8.1 Hz, 2H aromatic), 7.49(d,J=8.1Hz,2H aromatic);
MS-FD M/e 544($M^+$);

| Anal. Calcd for $C_{35}H_{51}F_3O$: | | |
|---|---|---|
| | C,77.17; | H, 9.44 |
| Found: | C,77.46; | H, 9.60 |

Example 17

Preparation of 4α-(4-methoxycarbonylbenzyl)cholestan-3-one
IR($CHCl_3$ $cm^{-1}$): 1711
300MHz $^1H$ NMR($CDCl_3$, ppm) δ 0.67(s,3H, $CH_3$), 0.87 (d,J=6.6Hz,6H, $-CH(CH_3)_2$), 0.90(d,J=6.8Hz,3H, $-CH(CH_3)-$), 1.08(s,3H, $-CH_3$), 0.70-1.66(m,22H), 1.66-1.92(m,3H), 1.92-2.10(m, 2H), 2.26-2.35(m,1H), 2.44(td, J=13.9 Hz, 6.2Hz, 1H), 2.55-2.65 (m, 1H), 2.87 (dd, J=14.2Hz; 3.2Hz, 1H), 3.06(dd,J=14.2 Hz; 7.4Hz, 1H), 3.90(s, 3H, $-CO_2CH_3$), 7.27 (d,J=8.11Hz, 2H), 7.91(d,J=8.1Hz, 2H).
Mass(M/z, $FD^+$):534($M^+$);

| Elemental Anal.: Calcd for $C_{36}H_{54}O_3$: | | |
|---|---|---|
| | C, 80.85; | H, 10.18 |
| Found: | C, 81.01; | H 10.05 |

Example 18

Preparation of 4α-(4-benzyloxybenzyl)cholestan-3-one
12.5% yield; mp 175.0-178.0°C ($CH_2Cl_2/CH_3CN$);
IR($CHCl_3$) 1703 $cm^{-1}$;
NMR 300MHz ($CDCl_3$) δ 0.68(s,3H, methyl), 0.87(d,J=6.6Hz,6H), 0.91(d,J=6.5Hz,3H.), 0.70-1.62(m,22H), 1.70-1.90(m,3H), 1.95-2.08(m,2H), 2.28(br d, J=14.6 Hz, 1H), 2.35-2.58(m,2H), 2.80 (br dd, J-14.4, 3.2 Hz, 1H), 2.93(dd, J=14.0, 7.0 Hz, 1H), 5.03 (s, 2H, benzylic), 6.86(d,J=8.5Hz,2H), 7.10(d,J=8.5 Hz, 2H), 7.36-7.50(m,5H, aromatic);
MS-FD M/e 582($M^+$);

| Anal. Calcd for $C_{41}H_{58}O_2$: | | |
|---|---|---|
| | C, 84.48; | H, 10.03 |
| Found: | C, 84.58; | H, 10.07 |

Example 19

Preparation of 4α-(4-trifluoromethoxybenzyl)cholestan-3-one
14% yield; mp 128-130°C ($CH_2Cl_2/CH_3CN$);
IR ($CHCl_3$) 1705 $cm^{-1}$;
NMR ($CDCl_3$) δ 0.68(s, 3H, methyl), 0.87 (d, J=6.6 Hz, 6H), 0.91 (d, J=6.5 Hz, 3H), 1.08 (s, 3H, methyl), 0.70-1.66 (m, 22H), 1.73-1.87 (m, 3H), 1.96=2.07 (m, 2H), 2.26-2.36 (m, 1H), 2.39-2.61 (m, 2H), 2.81 (dd, J=14.2, 2.7 Hz, 1H), 2.98 (dd, J=14.3, 7.5 Hz, 1H), 7.07 (d, J= 8.4 Hz, 2H, aromatic), 7.21 (d, J=8.6 Hz, 2H, aromatic);
MS-FD m/e 560 ($M^+$);

| Anal. Calcd. for $C_{35}H_{51}F_3O_2$: | | |
|---|---|---|
| | C, 74.96; | H, 9.17 |
| Found: | C, 75.00; | H, 9.09 |

Example 20

Preparation of 4α-(4-chlorobenzyl)cholestan-3-one
37% yield; mp 157-159°C ($CH_2Cl_2/CH_3CN$);
IR ($CHCl_3$) 1704 $cm^{-1}$;
NMR ($CDCl_3$) δ 0.68(s, 3H, methyl), 0.87 (d, J=6.6 Hz, 6H), 0.91 (d, J=6.5 Hz, 3H), 1.07 (s, 3H, methyl), 0.72-1.68 (m, 22H), 1.73-1.87 (m, 3H), 1.96-2.07 (m, 2H), 2.26-2.36 (m, 1H), 2.39-2.61 (m, 2H), 2.81 (dd, J=14.2, 2.7 Hz, 1H), 2.98 (dd, J=14.3, 7.5 Hz, 1H), 7.12 (d, J= 8.4 Hz, 2H, aromatic), 7.20 (d, J=8.3 Hz, 2H, aromatic);
MS-FD m/e 511 ($M^+$), 513 ($^{37}Cl$ $M^+$);

| Anal. Calcd. for $C_{35}H_{51}ClO$: | | |
|---|---|---|
| | C, 79.88; | H, 10.06 |
| Found: | C, 79.78; | H, 10.15 |

Example 21

Preparation of 4α-(3,4-dichlorobenzyl)cholestan-3-one
9% yield; mp 156-158°C ($CH_2Cl_2/CH_3CN$);
IR ($CHCl_3$) 1706 $cm^{-1}$;
NMR ($CDCl_3$) δ 0.68 (s, 3H, methyl), 0.87 (d, J=6.6 Hz, 6H), 0.91 (d, J=6.5 Hz, 3H), 1.07 (s, 3H, methyl), 0.72-1.68 (m, 22H), 1.73-1.87 (m, 3H), 1.96-2.07 (m, 2H), 2.26-2.36 (m, 1H), 2.39-2.61 (m, 2H), 2.81 (dd, J=14.2, 2.7 Hz, 1H), 2.98 (dd, J=14.3, 7.5 Hz, 1H), 7.01-7.04 (m, 2H, aromatic),7.25-7.36 (m, 2H, aromatic);
MS-FD m/e 545 ($M^+$), 547 ($^{37}Cl$ $M^+4$);

| Anal. Calcd. for $C_{34}H_{50}Cl_2O$: | | |
|---|---|---|
| | C, 74.84; | H, 9.24 |
| Found: | C, 75.07; | H, 9.31 |

Example 22

Preparation of 4α-benzylcholestan-3α-ol.

Reduction with diisobutylaluminum hydride.

A solution of diisobutyl aluminum hydride (1.0M in toluene, 0. 31 ml, 0.31 mmol) was added dropwise to a stirred solution of 4α-benzylcholestan-3-one (159mg, 0.33 mmol) in 6 ml dry methylenechloride at -78°C under an argon atmosphere. The resulting mixture was stirred at -78°C for 30 min before it was allowed to warm up to 0°C, where it was quenched with 5 ml of 1N HCl(aq.) and 10 ml diethyl ether. The biphasic mixture was stirred vigorously for 30 min. The organic layer was separated and washed sequentially with 5 ml saturated aqueous sodium bicarbonate and 5 ml brine, dried over anhydrous MgS04 filtered and concentrated. The residue was subjected to MPLC separation on silica gel (n-hexane → 15% ethyl acetate/n-hexane as eluent) to give 45.0 mg (28% yield) of the desired 4α-benzylcholestan-3α-ol and 85.0 mg (53% yield) 4α-benzylcholestan-3β-ol.

28% yield; mp 145.0-146.0°C ($CH_2Cl_2/CH_3CN$);
IR(KBr) br 3485 $cm^{-1}$;
NMR 300 MHz($CDCl_3$) δ 0.64(s,3H, methyl), 0.82(s,3H, methyl) 0.85(d,J=6.5Hz, 6H), 0.89(d,J=6.5 Hz,3H), 0.70-1.90(m,30H), 1.90-2.00(M,1H), 2.37 (br t, J=12.0 Hz, 1H), 2.85(dd,J=13.1; 4.5 Hz, 1H), 3.46 (br d, J=1.9Hz 1H), 7.10-7.32(m,5H);

MS-FD m/e 478($M^+$);

| Anal. Calcd for $C_{34}H_{54}O$: | | |
|---|---|---|
| | C, 85.29; | H, 11.36 |
| Found: | C, 85.59; | H, 11.72 |

By substantially following the procedures described in Example 22, the compounds of Examples 23 through 27 were prepared.

Example 23

Preparation of 4α-(4-fluorobenzyl)cholestan-3α-ol
27% yield; mp 174.0-175.5°C ($CH_2Cl_2$/$CH_3CN$);
IR($CHCl_3$) br 3618 $cm^{-1}$;
NMR 300 MHz ($CDCl_3$) δ 0.64(s,3H, methyl), 0.81(s, 3H, methyl), 0.85(d,J=6.5 Hz, 6H), 0.89(d, J=6.6Hz, 3H), 0.65-1.90(m,30H), 1.95(br d, J=11.7 Hz, 1H), 2.37(br t,J=12.3Hz, 1H), 2.80 (dd, J=13.2, 4.4 Hz, 1H), 3.44(br d, J=2.0 Hz, 1H), 6.94(t,J=8.5 Hz, 2H aromatic), 7.15 (dd, J=8.1, 6.7 Hz, 2H aromatic);
MS-FD M/e 497(1+$M^+$);

| Anal. Calcd for $C_{34}H_{53}FO$: | | |
|---|---|---|
| | C, 82.20; | H, 10.75 |
| Found: | C, 82.23; | H, 10.83 |

Example 24

Preparation of 4α-(4-bromobenzyl)cholestan-3α-ol
41.5% yield; mp 177.0-178.0°C ($CH_2Cl_2$/$CH_3CN$);
IR($CHCl_3$) br 3625 $cm^{-1}$;
NMR 300 MHz ($CDCl_3$) δ 0.65(s,3H, methyl), 0.81(s,3H, methyl) 0.85(d, J=6.5 Hz, 6H), 0.89(d, J=6.4 Hz, 3H), 0.70-1.88 (m, 30H), 1.96(br d, J=12.0Hz, 1H), 2.32-2.40(m,1H), 2.78(dd,J=13.2, 4.7Hz, 1H), 3.43(d,J=2.5 Hz, 1H), 7.08(d, J=8.2Hz, 2H aromatic), 7.37(d,J=8.2Hz, 2H);
MS-FD M/e 558($M^+$);

| Anal. Calcd for $C_{34}H_{53}BrO$: | | |
|---|---|---|
| | C, 73.22; | H, 9.58 |
| Found | C, 73.34; | H, 9.49 |

Example 25

Preparation of 4α-(4-iodobenzyl)cholestan-3α-ol
37.3% yield; mp 187.0-188.5°C ($CH_2Cl_2$/$CH_3CN$);
IR($CHCl_3$) br 3625 $cm^{-1}$;
NMR 300 MHz ($CDCl_3$) δ 0.67(s,3H, methyl), 0.83(s,3H, methyl) 0.88 (d,J=6.6 Hz, 6H), 0.91(d, J=6.3Hz, 3H), 0.70-1.90 (m,30H), 1.98 (br dd, J=11.9Hz, 1H), 2.37(br t, J=12.2Hz, 1H), 2.80 (dd,J=13.2, 4.4Hz, 1H), 3.46(br d., J=2.4Hz, 1H), 7.0(d,J=8.1 Hz, 2H aromatic), 7.59(d,J=8.1Hz,2H aromatic);
MS-FD M/e 604($M^+$);

| Anal. Calcd for $C_{34}H_{53}IO$: | | |
|---|---|---|
| | C, 67.53; | H, 8.84 |
| Found: | C, 67.58; | H, 8.96 |

Example 26

Preparation of 4α-(4-trifluoromethylbenzyl)cholestan-3α-ol
33% yield; mp 174.5-175.5°C ($CH_2Cl_2/CH_3CN$);
IR($CHCl_3$) br 3610 $cm^{-1}$;
NMR 300 MHZ ($CDCl_3$) δ 0.65(S,3H, methyl), 0.82(s,3H, methyl) 0.85(d, J=6.6Hz, 6H), 0.89(d,J=6.5 Hz, 3H), 0.70-1.90(M,30H), 1.94-1.99(m,1H), 2.48 (br d, J=12.1 Hz, 1H), 2.88 (dd, J=13.2, 4.3 Hz, 1H), 3.40 (br d, J=2.4 Hz, 1H), 7.31(d, J=7.9 Hz 2H aromatic), 7.51 (d,J=8.0 Hz, 2H aromatic);
MS-FD M/e 546($M^+$);

| Anal. Calcd for $C_{35}H_{53}F_3O$: | | |
|---|---|---|
| | C, 76.88; | H, 9.77 |
| Found: | C, 76.96; | H, 9.83 |

Example 27

Preparation of 4α-(3,4-dichlorobenzyl)cholestan-3α-ol
12.5% yield; mp 168-170°C ($CH_2Cl_2/CH_3CN$);
IR 3617 (br) $cm^{-1}$;
NMR ($CDCl_3$) δ 0.67(s, 3H, methyl), 0.84 (s, 3H, methyl), 0.88 (d, J=6.5 Hz, 6H), 0.91 (d, J=6.3 Hz, 3H), 0.70-1.90 (m, 30H), 1.95-2.03 (m, 1H), 2.41 (br. t, J=11.7 Hz, 1H), 2.79 (dd, J=13.0, 4.3 Hz, 1H), 3.46 (br s, 1H), 7.05-7.15 (m, 1H, aromatic), 7.33-7.40 (m, 2H, aromatic)
MS-FD m/e 547 ($^{35}Cl,M^+$), 549 ($^{37}Cl, M^+$);

| Anal. Calcd. for $C_{34}H_{52}Cl_2O$: | | |
|---|---|---|
| | C, 74.56; | H, 9.57 |
| Found: | C, 74.80; | H, 9.42 |

Example 28

Preparation of 4α-(4-cyanobenzyl)cholestan-3α-ol
Reduction with K-selectride (potassium tri-sec-butyl-borohydride

K-selectride (1.0M in THF, 0.553 ml, 0.553 mmol) was added dropwise to a stirred solution of 4α-(4-cyanobenzyl)cholestan-3-one (154 mg, 0.307 mmol) in 4 ml dry THF at -20°C under an argon atmosphere. After 1 hour at -20°C, the reaction mixture was quenched with 500 μl acetic acid and allowed to warm up to ambient temperature where it was stirred for an additional 15 min. The suspension was filtered through a short pad of silica gel (ethyl acetate as eluent), the fractions containing product were collected and concentrated to give a white solid residue which was recrystallized in acetonitrile/methylene chloride to give 135 mg (0.268 mmol, 87% yield) 4α-(4-cyanobenzyl)cholestan-3α-ol.
IR($CHCl_3$, $cm^{-1}$): 3616, 2230
300MHZ $^1H$ NMR($CDCl_3$, ppm) δ 0.67(S,3H, $C\underline{H}_3$), 0.84 (s, 3H, $-C\underline{H}_3$), 0.88(d, J=6.6Hz, 6H, $-CH(C\underline{H}_3)_2$), 0.91(d,J=6.7Hz, 3H, $-CH(C\underline{H}_3)-$), 0.70-1.92 (m, 30H), 1.99 (broad d, J=12.0 Hz, 1H), 2.52 (broad t, J=12.0Hz, 1H), 2.88(dd, J=13.1Hz, 4.4Hz, 1H), 3.39 (broad s, 1H), 7.34 (d, J=8.0Hz, 2H), 7.57(d, J=8.0Hz, 2H)
Mass($FD^+$):503($M^+$);

| Elemental Anal.: Calcd for $C_{35}H_{53}NO$: | | | |
|---|---|---|---|
| | C, 83.44; | H, 10.60, | N, 2.78; |
| Found: | C, 83.14; | H, 10.67, | N 2.85 |

Example 29

Preparation of 4α-(4-methoxycarbonylbenzyl)cholestan-3α-ol
IR($CHCl_3$, $cm^{-1}$): 3625, 1717
300MHz $^1H$ NMR($CDCl_3$, ppm) δ 0.67(s,3H, $CH_3$), 0.84 (d,J=6.6Hz,3H, -CH($CH_3$)-), 0.88(d,J=6.6Hz,3H, -CH($CH_3$)-), 0.91(d, J=6.5Hz, 3H, -CH($CH_3$)-), 0.70-1.92(m, 30H), 1.98 (broad d, J=12.9Hz, 1H), 2.50(broad t, J=12.0Hz, 1H), 2.90(dd, J=13.1 Hz, 4.6Hz, 1H), 3.43(d, J=2.5Hz, 1H), 3.91(S, 3H, -$CH_3$), 7.30(d, J=8.1Hz, 2H), 7.95 (d, J=8.1Hz, 2H).
Mass(FD):535($M^+$-1);

| Elementary Anal.: Calcd for $C_{36}H_{56}O_3$: | | |
|---|---|---|
| | C, 80.54; | H, 10.51; |
| Found: | C, 80.43; | H, 10.49 |

Example 30

Preparation of 4α-(4-trifluoromethoxybenzyl)cholestan-3α-ol
48% yield;
IR ($CHCl_3$) 3600 (br), $cm^{-1}$;
NMR ($CDCl_3$) δ 0.64(s, 3H, methyl), 0.82 (s, 3H, methyl), 0.85 (d, J=6.6 Hz, 6H), 0.89 (d, J=6.4 Hz, 3H), 0.70-1.90 (m, 30H), 1.94-1.99 (m, 1H), 2.48 (br t, J=12.1 Hz, 1H), 2.88 (dd, J= 13.2 H, 3 Hz, 1H), 3.40 (br d. J=2.4 Hz, 1H), 7.08-7.15 (m, 1H, aromatic), 7.20-7.23 (m, 1H, aromatic);
MS-FD m/e 562 ($M^+$);

Example 31

Preparation of 4α-(4-chlorobenzyl)cholestan-3α-ol
8% yield;
NMR ($CDCl_3$) δ 0.67(s, 3H, methyl), 0.84 (s, 3H, methyl), 0.88 (d, J=6.5 Hz, 6H), 0.91 (d, J=6.4 Hz, 3H), 0.70:1.94 (m, 30H), 1.96-2.03 (m, 1H), 2.40 (dd, J=13.1, 11.3 Hz, 1H), 2.82 (dd, J= 13.2, 4.6 Hz, 1H), 3.46 (br d, J=2.5 Hz, 1H), 7.15 (d, J= 8.3 Hz, aromatic), 7.24 (d, J=8.2 Hz, aromatic)

Example 32

Preparation of 4α-(4-benzyloxybenzyl)cholestan-3α-ol
By substantially following the procedures of Example 28 and then Example 22, and utilizing the compound of Example 18, the title compound was prepared.
44.8% yield; mp 208.0-209.5°C ($CH_2Cl_2$/$CH_3CN$);
IR($CHCl_3$) br 3600 $cm^{-1}$;
NMR 300 MHz ($CDCl_3$) δ 0.67(s,3H, methyl), 0.84(s,3H, methyl) 0.88(d, J=6.6Hz, 6H), 0.92(d,J=6.4 3H), 0.70-1.90(m,30H), 2.0 (br d., J=12.0Hz, 1H), 2.35(br t. J=11.3 Hz, 1H), 2.82 (dd, J=13.4, 4.4 Hz, 1H), 3.51 (br d. J=2.3 Hz, 1H, 5.05 (s, 2H, benzylic), 6.92 (d, J=8.4 Hz, 2H, aromatic ), 7.13 (d, J=8.4 Hz, 2H, aromatic), 7.30-7.50 (m, 5H aromatic);
MS-FD M/e 584($M^+$);

| Anal. Calcd for $C_{41}H_{60}O_2$: | | |
|---|---|---|
| | C, 84.19; | H, 10.34 |
| Found: | C, 84.44; | H, 10.28 |

Example 33

Preparation of 4α-(4-hydroxymethylbenzyl)cholestan-3α-ol
A solution of diisobutylaluminum hydride (1.0M in toluene, 1.69 ml, 1.69 mmol) was added dropwise to a stirred solution of 4α-(4-methoxycarbonylbenzyl)cholestan-3-one(200mg, 0.375 mmol) in 6 ml dry THF at -10°C under an argon atmosphere. The resulting mixture was stirred at -10°C for 1 hr and then 5 ml 1N HCl(aq) was added. The biphasic mixture was stirred vigorously at ambient temperature for 30 min. The organic layer was separated and the aqueous layer was extracted with methylene chloride (30 ml x 2). The combined organic layers were washed sequentially with 3 ml saturated $NaHCO_3$(aq) ,3 ml $H_2O$ and 3 ml brine, dried over anhydrous magnesium sulfate, filtered and concentrated. The residual white solid was dissolved in a small amount of THF/$CH_2Cl_2$ (1 ml), then subject to MPLC separation on silica gel (ethylacetate/toluene : 10% → 35% as eluent) to give 59.0 mg (0.11 mmol, 31% yield) 4α-(4-hydroxymethylbenzyl)-cholestan-3α-ol and 131 mg (0.258 mmol, 69% yield) 4α-(4-hydroxymethylbenzyl)cholestan-3β-ol. Both were recrystallized in acetonitrile to give white needles.
IR($CHCl_3$, $cm^{-1}$): 3615
300MHz $^1H$ NMR($CDCl_3$, ppm) δ 0.67(s,3H, $CH_3$), 0.84 (s, 3H, -$CH_3$), 0.88(d, J=6.6Hz, 6H, -$CH(CH_3)_2$), 0.91(d,J=6.5Hz, 3H, -$CH(CH_3)$), 0.70-1.90 (m, 31H), 1.98 (broad d, J=12.0 Hz, 1H), 2.41 (dd, J=13.0Hz, 1H), 2.87(dd, J=13.3Hz, 4.6Hz, 1H), 3.48 (d, J=2.0Hz, 1H), 4.67(s, 2H), 7.22(d, J=8.0Hz, 2H), 7.29(d, J=8.0 Hz, 2H).
Mass ($FD^+$):508($M^+$);

| Elementary Anal.: Calcd for $C_{35}H_{56}O_2$: | | |
|---|---|---|
| | C, 82.62; | H, 11.09; |
| Found: | C, 82.87; | H, 11.27 |

Example 34

Preparation of 4α-(4-carboxybenzyl)cholestan-3α-ol

An amount 0.784 ml of 2N LiOH(aq) was added to a stirred clear solution of 4α-(4-methoxycarbonylbenzyl)cholestan-3α-ol (84.0g, 0.157 mmol) in a solution of THF (4ml)/$CH_3OH$(1 ml) at ambient temperature. The resulting suspension was heated to reflux for 1 hour under nitrogen atmosphere. At ambient temperature the reaction mixture was treated with a 1.8 ml of 1N HCl(aq), followed by 30 ml $H_2O$. The resulting white precipitate was filtered off and washed with $H_2O$. After drying in a vacuum oven at 60°C for 1 hour, 84.1 mg (0.142 mmol, yield 90%) of the desired product as a white solid was obtained.
IR($CHCl_3$, $cm^{-1}$): 3620, 1690
300MHz $^1H$ NMR($CDCl_3$, ppm) δ 0.67(s,3H, $CH_3$), 0.85 (s, 3H, -$CH_3$), 0.88(d, J=6.6Hz, 6H, -$CH(CH_3)_2$), 0.91(d,J=6.5Hz, 3H, -$CH(CH_3)$-), 0.70-1.90 (m, 31H), 1.99 (broad d, J=12.0, 1H), 2.53 (broad t, J=12.1 1H), 2.92(dd, J=13.3Hz, 4.4Hz, 1H), 3.44 (broad s, 1H), 7.34 (d, J=8.1 Hz, 2H), 8.02(d, J=8.1 Hz, 2H)
Mass(FD):522($M^+$)· 505 ($M^+$-OH);

| Elementary Anal.: Calcd for $C_{35}H_{54}O_3 \cdot C_4H_80$(THF): | | |
|---|---|---|
| | C, 78.74; | H, 10.50; |
| Found: | C, 78.43; | H, 10.34 |

Example 35

Preparation of 4α-(4-hydroxybenzyl)cholestan-3-one
[NAME CORRECTED]
10% Pd/C (154 mg) palladium on carbon was added to a stirred solution of 4α-(4-benzyloxybenzyl)cholestan-3-one(770 mg, 1.32 mmol) in 8 ml THF at ambient temperature under an argon atmosphere. Argon was removed and hydrogen was introduced from a balloon filled with hydrogen. The reaction mixture was stirred under hydrogen atmosphere overnight. After filtration through a short pad of Celite®, the filtrate was concentrated and the residual solid was recrystallized from toluene to yield 400 mg (62% yield) 4α-(4-hydroxybenzyl)cholestan-3-one.
53% yield; mp 234.0-236.0°C ($CH_2Cl/CH_3CN$);
IR ($CHCl_3$): br 3604, 1702 $cm^{-1}$;
NMR 300 MHz ($CDCl_3$) δ 0.68(s,3H, methyl), 0.87(J=6.6Hz, 6H), 0.90(d,J=6 Hz 3H), 1.05(s, 3H, methyl), 0.70-1.65(m,22H), 1.72-1.90 (m, 3H), 1.96-2.08 (m, 2H), 2.28 (br d., J=6.9, 1H), 2.35-2.55(m, 2H), 2.80(dd, J=14.3, 3.3 Hz, 1H), 2.91 (dd, J=14.3, 7.2 Hz, 1H) 4.70 (br. s, 1H, -OH), 6.70 (d, J=8.3 Hz, 2H aromatic), 7.06 (d, J=8.3 Hz, 2H, aromatic);
MS-FD M/e 492($M^+$);

| Anal. Calcd for $C_{34}H_{52}O_2$: | | |
|---|---|---|
| | C, 82.87; | H, 10.64 |
| Found: | C, 83.11; | H, 10.41 |

Example 36

Preparation of 4α-(4-hydroxybenzyl)cholestan-3α-ol
The above 4α-(4-hydroxybenzyl)cholestan-3-one from Example 35 was reduced, as described previously in Example 22, using diisobutylaluminum hydride to yield 42% yield of 4α-(4-hydroxybenzyl)cholestan-3α-ol and 44% yield of 4α-(4-hdyroxybenzyl)cholestan-3β-ol.
42% yield; mp decomposes at 215.0°C ($EA/CH_3CN$);
IR ($CHCl_3$) br 3607 $cm^{-1}$;
NMR 300 MHz ($CDCl_3$) δ 0.67(s,3H), 0.84(2, 3H, methyl), 0.88(d,J=6.6Hz, 6H), 0.91(d,J=6.4 Hz 3H), 0.70-1.90(m, 31H), 2.0 (br d, J=11.9 Hz, 1H), 2.33(br t, J=11.3 Hz, 1H), 2.81 (dd, J=13.5, 4.4Hz, 1H), 3.51(br d, J=2.5Hz, 1H), 6.76(d, J=8.2Hz, 2H, aromatic), 7.08(d, J=8.2Hz, 2H, aromatic);
MS-FD M/e 494($M^+$);

| Anal. Calcd for $C_{34}H_{54}O_2$: | | |
|---|---|---|
| | C, 82.53; | H, 11.00 |
| Found: | C, 82.73; | H, 10.72 |

Example 37

Preparation of 4-benzyl-4-cholesten-3-one

A solution of 4-cholesten-3-one (30.4 g, 79.0 mmol) and pyrrolidine (33.0 ml, 395 mmol) in 120 ml benzene was heated to reflux under nitrogen with continuous removal of water for 24 hours. The reaction mixture was concentrated in vacuo to dryness to give 34.9 g (79.0 mmol, 100% yield) of 3-pyrrolidino-3,5-cholestadiene, as a yellowish solid.

A stirred suspension of 3-pyrrolidino-3,5-cholestadiene (438 mg, 1.00 mmol) and benzyl bromide (178 µl, 1.50 mmol) in 6 ml dry DMF was heated in an oil bath at 180°C under Argon for 2 hours, then the oil bath temperature was cooled down to ~100°C. To the reaction mixture was added 4 ml water and 3 ml dioxane. The resulting mixture was stirred at 100°C for 1.5 hour before it was allowed to cool down to ambient temperature. Ether, 20 ml, and 20 ml water were added to the mixture. The organic layer was separate and the aqueous layer was extracted with ethyl ether (20 ml x 2). The combined organic layers were washed with 10 ml brine, dried over anhydrous $MgS0_4$, filtered and concentrated. The oily residue was subject to MPLC separation

on silica gel (ethyl acetate/n-hexane: 10 → 20% as eluent) to give 252 mg (0.532 mmol, 53% yield) 4-benzyl-4-cholesten-3-one as a colorless viscous oil.
mp: oily product
IR(film, $cm^{-1}$): 1667
300MHz $^1H$ NMR($CDCl_3$, ppm) δ 0.71(s,3H, $CH_3$), 0.87 (d, J=6.4Hz, 6H, -CH($CH_3$), 0.92(d, J=6.4Hz, 3H, -$CHCH_3$-), 1.23 (s, 3H, -$CH_3$), 0.77-1.65(m, 19H), 1.65-1.95 (m, 3H), 1.95-2.19(m,3H), 2.40-2.59(m, 2H), 2.76(broad d, J=14.7 Hz, 1H), 3.70(d, J=15.4 Hz, 1H), 3.76 (d, J=15.4 Hz, 1H), 7.09-7.30(m, 5H).c
Mass(M/Z, FAB): Calcd for $C_{34}H_{51}O$ ($M^+$+1)475.3940;:
Elementary Anal.:
Found: C, 475.3935

## Example 38

Preparation of 4-benzyl-4-cholesten-3α-ol
4α-benzyl-4-cholesten-3-one(213 mg. 0.449 mmol) from Example 37 was reduced as described previously, using diisobutylaluminum hydride to give 21.5 mg (0.0452 mmol, 10% yield) 4α-benzyl-4-cholesten-3α-ol and 189 mg (0.397 mmol, 88% yield) 4α-benzyl-4-cholesten-3β-ol, both as colorless viscous oils.
IR ($CHCl_3$, $cm^{-1}$): 3608
300MHz $^1H$ NMR($CDCl_3$, ppm) δ 0.65(s,3H, $CH_3$), 0.89 (d, J=6.6Hz, 6H, -CH($CH_3$)$_2$), 0.92(s, 3H, -$CH_3$, 0.97 (d, J=6.5 Hz, 3H, -CH($CH_3$)-), 0.68-1.65 (m, 25H), 1.71-1.86(m,2H), 1.95 (broad d, J=12.4 1H), 2.50(broad d, J=14.2Hz, 1H), 3.50 (d, J=15.6 Hz, 1H), 3.56 (d, J=15.6 Hz, 1H), 3.79 (s, 1H), 6.98-7.16 (m, 5H)
Mass 475 ($M^+$-1);

## Example 39

Preparation of 4α-(2-propenyl)-5-cholesten-3-one
By substantially following the procedures described above in Example 37 and using allyl bromide as a reactant, the title compound was prepared.
10% yield; mp 77-78.5°C ($CH_2Cl_2$/$CH_3CN$);
NMR ($d_6$-benzene) δ 0.58(s, 3H, methyl), 0.85 (s, 3H, methyl), 0.86 (d, J=6.8 Hz, 6H), 0.94(d, J=.4 Hz, 3H), 0.70-1.59 (m, 21H), 1.70-2.10 (m,5H), 2.20-2.33 (m, 1H), 2.78-2.91 (m, 1H), 2.93-3.0 (m, 1H), 4.96-5.09 (m, 2H); 5.23-5.29 (m, 1H); 5.87-6.02(m, 1H);
MS-FD m/e 424 ($M^+$);

| Anal. Calcd. for $C_{30}H_{48}O$: | | |
|---|---|---|
| | C, 84.84; | H, 11.39 |
| Found: | C, 85.09; | H, 11.71 |

## Example 40

Preparation of 4α-(2-propenyl)-5-cholesten-3α-ol
By substantially following the procedures described in Example 28, the title compound was prepared.
30% yield;
NMR ($CDCl_3$) δ 0.66(s, 3H, methyl), 0.85 (d, J=6.6 Hz, 6H), 0.90 (d, J=6.6 Hz, 3H), 1.02 (s, 3H, methyl), 0.70-2.43 (m, 30H), 3.89 (br s, 1H), 4.96-5.13 (m, 2H), 5.35-5.46 (m, 1H), 5.76-5.93 (m, 1H).

## Example 41

Preparation of 4-cholesten-24-N,N-dimethylamide-3-one:
The compound 4α-cholest-4-en-24-oic acid -3-one was prepared substantially according to the procedures described in Miyamoto, et al., Synthetic Communications, 16, No. 5, 513-521 (1986) and Demir, et al., Organic Prep. and Proc. International, 19 (2-3), 197-208 (1987) which are incorporated herein by reference.

A -5°C solution of 4α-cholest-4-en-24-oic acid -3-one (2.5g, 6.71 mmol) in dry methylene chloride (20.0 ml) was treated with N-methyl-morpholine (2.58 ml, 23.49 mmol) followed by isobutylchloroformate (1.05 ml, 8.1 mmol). The suspension was stirred for 45 minutes and then N,N-dimethylamine hydrochloride salt (1.1g,

13.42 mmol) was added. The reaction was stirred for 45 minutes then diluted with 40 ml $CH_2Cl_2$. The reaction mixture was filtered over a pad of Celite® and then the organic material was washed with 30 ml distilled water, then brine (30 ml). The dried organic was then filtered, and concentration of the filtrate yielded a yellow solid which was subjected to flash-chromatography (60% EtOAc/tol to 80% EtOAc/tol) to afford the desired compound (2.51g, 6.73 mmol, 94%) as a solid. Crystallization from $CH_2Cl_2$/EtOAc and hexane gave an off-white solid. mp 173-174.5°C.
IR ($CHCl_3$), 1632, 1047 $cm^{-1}$;
$^1$HNMR ($CDCl_3$) δ 0.72(s, 3H, methyl), 0.95 (d, J=6.4 Hz, 3H, methyl), 1.19 (s, 3H, methyl), 0.74-2.5 (m, 25H), 2.9 (br s, 6H), 5.73 (s, 1H, vinyl);
MS-FD m/e 399 ($M^+$);

| Anal. Calcd. for $C_{26}H_{41}NO_2$: | | | |
|---|---|---|---|
| | C, 78.15; | H, 10.34, | N, 3.51 |
| Found: | C, 78.39; | H, 10.31, | N, 3.71 |

Example 42

Preparation of 4α-(2-propenyl)-cholan-24-N,N-dimethyl amide-3-one.

4α-(2-propenyl)-cholan-24-N,N-dimethylamide-3-one (420 mg, 0.95 mmol., 38% was prepared according to the procedures described in Example 22 from 4-cholesten-24-N,N-dimethyl amide-3-one (1.0 g, 2.5 mmol), lithium wire (38 mg, 5.5 mmol) distilled liquid ammonia (15 ml), tertiary butyl alcohol (165 μl, 1.75 mmol) and allyl bromide (650 μl, 7.5 mmol) dissolved in dry toluene (16 ml) and dry THF (25 ml.) at -78°C.
38% yield; mp 83-85°C
IR ($CHCl_3$) 1704, 1631, 1049 $cm^{-1}$;
$^1$HNMR ($CDCl_3$) δ 0.69(s, 3H, methyl), 0.94 (d, J=6.4 Hz, 3H, methyl), 1.06 (s, 3H, methyl), 0.71-2.56 (m, 29H), 2.98 (br s 6H), 4.95-5.06 (m, 2H vinyl), 5.70-5.86 (m, 1H vinyl),
MS-FD m/e 441 ($M^+$);

| Anal. Calcd. for $C_{29}H_{47}NO_2$: | | | |
|---|---|---|---|
| | C, 78.86; | H, 10.73, | N, 3.17 |
| Found: | C, 79.06; | H, 10.85, | N, 3.10 |

Example 43

Preparation of 4α-(2-propenyl)-cholan-24-N,N-dimethylamide-3α-ol.

4α-(2-propenyl)-cholan-24-N,N-dimethyl amide-3α-ol (92 mg, 0.20 mmol, 46%) was prepared according to the procedures described in Example 28 using 4α-(2-propenyl)-cholan-24-N,N-dimethylamide-3-one (200 mg, 0.45 mmol), and 1.0 M K-selectride (1.58 ml, 1.58 mmol) in dry THF (2.0 ml) at -78°C.
53% yield; mp 218-220°C (toluene);
IR ($CHCl_3$) 1631, 1052 $cm^{-1}$;
$^1$HNMR ($CDCl_3$) δ 0.66(s, 3H, methyl), 0.82 (s, 3H, methyl), 0.95 (d, J=6.4 Hz, 3H, methyl), 0.71-2.04 (m, 27H), 2.16-2.46 (m, 3H), 2.99 (br s, 6H), 3.91 (br s, 1H), 5.00-5.16 (m, 2H, vinyl), 5.80-5.99 (m, 1H, vinyl)
MS-FD m/e 443 ($M^+$+1)

Example 44

Preparation of 4α-(2-propenyl)-cholan-24-N,N-dimethylamino-3α-ol.
An ice-cold suspension of lithium aluminum hydride (26 mg, 0.69 mmol) in dry THF (2.0 ml) was treated with 4α-(2-propenyl)-cholan-24-N,N-dimethylamide-3α-ol (100 mg, 0.23 mmol) dissolved in dry THF (5.0 ml), and added to the reaction via cannula. The reaction was stirred 1 hour at 0°C, quenched with 5.0 ml EtOAc followed by 2N NaOH (5.0 ml). The reaction mixture was stirred vigorously for 45 minutes, the phases separated and the organics washed with brine (4.0 ml), and concentrated in vacuo to solid. The solids were purified further with flash chromatography ($CHCl_3$ to 5% MeOH/$CHCl_3$ with 0.45% $NEt_3$) to afford the desired compound

(88mg, 0.198 mmol; 96%). Crystallization from $CH_3CN/CH_2Cl_2$ gave a white solid.(mp 146-148°C).
IR ($CH_3Cl$) 3618 (br), 1049 $cm^{-1}$;
$^1$HNMR ($CDCl_3$) δ0.66 (s, 3H, methyl), 0.82 (s, 3H, methyl),) 0.92 (d, J=6.4 Hz, 3H, methyl), 0.70-2.04 (m, 27H), 2.23-2.53 (m, 3H), 3.91 (br s, 1H), 5.00-5.16 (m, 2H vinyl), 5.80-5.98 (m, 1H, vinyl),
MS-FD m/e 429 ($M^+$+1)

Example 45

Preparation of 3α-(isobutyloxycarbonyloxy)-12α-hydroxycholan-24-oxo-N,N-dimethylamide

N-methylmorpholine (2.82 ml, 25.6 mmol) was added to a stirred suspension of deoxycholic acid (1.00 g, 2.55 mmol) in 6 ml of dry $CH_2Cl_2$ at ambient temperature under an argon atmosphere. The resulting clear solution was cooled to 0°C in an ice bath and then treated dropwise with isobutylchloroformate (1.02 ml, 7.90 mmol) to form a white suspension. After 45 minutes at 0°C, solid dimethylamine hydrochloride (624 mg, 7.65 mmol) was added to the reaction mixture and the reaction mixture was stirred at 0°C for an additional 2 hours. The reaction mixture was diluted with 40 ml of EtOAc before it was filtered through a short pad of silica gel (EtOAc as eluent). The filtrate containing the desired amide was concentrated in vacuo to give a white solid, which was purified by flash column chromatography on silica gel (gradient EtOAc/n-hexane: 50% → 80%) to give 989 mg (1.91 mmol, 75%) of white solid. Recrystallization from $CH_2Cl_2$/n-hexane gave white crystals, mp 156.0-157.0°C.
IR (KBr): 3418 (br), 2941, 2871, 1747, 1632 and 1256 $cm^{-1}$
NMR (300 MHz, $CDCl_3$): 0.70 (s, 3H), 0.94 (s, 3H), 0.96 (d, J=6.8 Hz, 6H), 1.00 (d, J=6.3 Hz, 3H), 2.10-0.85 (m, 26H), 2.30-2.16 (m, 1H), 2.47-2.33 (m, 1H), 2.96 (s, 3H), 3.03 (s, 3H), 3.91 (d, J=6.7 Hz, 2H), 3.99 (br s. 1H), 4.66-4.52 (m, 1H)
Mass (FAB): 520 ($M^+$+1), 519 ($M^+$)

By substantially following the procedures described in Example 45, the compounds of Examples 46, 47, 48 and 49 were prepared.

Example 46

Preparation of 3α-(isobutyloxycarbonyloxy)-cholan-24-oxo-N,N-dimethylamide
Formula weight 503.65
$C_{31}H_{55}NO_4$
$^1$HNMR ($CDCl_3$)

Example 47

Preparation of 3α-(isobutyloxycarbonyloxy)-7α-hydroxy-cholan-24-oxo-N,N-dimethylamide
Yield 76% (foam)
IR (Film): 33420 (br), 2940, 2870, 1730, 1630 and 1255 $cm^{-1}$
$^1$HNMR (300 MHz, $CDCl_3$): 0.65 (s, 3H), 0.91 (s, 3H), 0.93 (d, J=6.8 Hz, 6H), 0.94 (d, J=6.3 Hz, 3H), 2.03-1.00 (m, 25H), 2.26-2.12 (m, 1H), 2.47-2.28 (m, 2H), 2.93 (s, 3H), 3.00 (s, 3H), 3.84 (brs,. 1H), 3.86 (d, J=6.7 Hz, 2H) and 4.50-4.36 (m, 1H)
Mass (FAB): 520 ($M^+$+1) $C_{31}H_{54}NO_5$, 520.4002
Found 520.4001

Example 48

Preparation of 3α-(isobutyloxycarbonyloxy)-7α,12α-dihydroxy-cholan-24-oxo-N,N-dimethylamide
Yield 57% (gum)
IR (Neat) 3420 (br), 1260, 1250, 780, and 760 $cm^{-1}$
$^1$HNMR (300 MHz, $CDCl_3$): 0.71 (s, 3H), 0.92 (s, 3H), 0.95 (d, J=6.7 Hz, 6H), 1.01 (d, J=6.3 Hz, 3H), 2.07-0.90 (m, 23H), 2.32-2.13 (m, 2H), 2.50-2.32 (m, 2H), 2.95 (s, 3H), 3.03 (s, 3H), 3.96-3.76 (m, 1H), 3.89 (d, J=6.7 Hz, 2H), 4.05-4.96 (m, 1H), and 4.55-4.35 (m, 1H)
Mass (FAB): 536 ($M^+$+1) , 535 ($M^+$)

Example 49

Preparation of 3α-(isobutyloxycarbonyloxy)-7β-hydroxy-cholan-24-oxo-N,N-dimethylamide

Yield 85%
$^1$HNMR (300 MHz, $CDCl_3$): 0.64 (s, 3H), 0.91 (d, J=6.8 Hz, 9H), 0.92 (s, 3H), 2.00-0.80 (m, 26H), 2.24-2.10 (m, 1H), 2.39-2.26 (m, 1H), 2.90 (s, 3H), 2.98 (s, 3H), 3.60-3.45 (m, 1H), (d, J=6.6 Hz, 2H), 4.56-4.42 (m, 1H)

Example 50

Preparation of 3α, 12α-dihydroxy-25-azacoprostane

Lithium aluminum hydride (110 mg, 2.89 mmol) was added to a stirred solution of 3α-(isobutyloxycarbonyloxy)-12α-(hydroxy)cholan-24-oxo N,N-dimethylamide (300mg, 0.578 mmol) in 6 ml of dry THF at 0°C under argon. The resulting dark gray suspension was stirred at 0°C for 2 hours. The reaction mixture was treated dropwise with 2 ml of methanol and 12 ml of 2N aq. NaOH sequentially, and then stirred vigorously at ambient temperature for 30 min. The mixture was extracted with $EtOAc/CH_2Cl_2$ (3/1; 30 ml x 3). The combined organic layers were washed with saturated aq NaCl (30 ml x 3), dried over $MgSO_4$, filtered and concentrated in vacuo. The residue was subjected to flash column chromatography on silica gel (gradient $Et_3N/CH_3OH/CH_2Cl_2$: 0/10/90 → 5/10/85 → 10/10/80) to give 215 mg (0.531 mmol), 92% of white solid.
Recrystallization from $CH_2Cl_2$/n-hexane gave white crystals, mp 240°C (dec.).
Yield 92%
IR (KBr) 3418 (br), 2937, 2864 $cm^{-1}$
$^1$HNMR (300 MHz, $CDCl_3$): 0.69 (s, 3H), 0.92 (s, 3H), 1.02 (d, J=6.5 Hz, 3H), 2.00-1.00 (m, 28H), 2.81 (s, 3H), 2.83 (s, 3H), 3.07-2.85 (m, 2H), 3.69-3.58 (m, 1H), and 3.99 (br s, 1H).
Mass (FAB): 406 ($M^+$+1), 405 ($M^+$)

Example 51

Preparation of 3α-hydroxy-25-azacoprostane
IR (KBr): 3379 (br), 1068, 1041 $cm^{-1}$
$^1$HNMR ($CDCl_3$): δ 0.72 (s, 3H, methyl), 0.79-1.00 (m, 6H), 1.01-2.00 (m, 30H), 2.4-2.63 (m, 7H), 3.53-3.70(m, 1H);
MS-FD, m/e: 389 ($M^+$)

Example 52

Preparation of 3α,7α-dihydroxy-25-azacoprostane
Yield 94%; mp ($CH_3OH/Et_2O$): 196.0°C (dec)
IR (KBr) 3355 (br), 2939, 2867 $cm^{-1}$
NMR (300 MHz, $CDCl_3$): 0.68 (s, 3H), 0.92 (s, 3H), 0.96 (d, J=6.5 Hz, 3H), 2.06-0.85 (m, 27H), 2.22 (J=12.4 Hz, 1H), 2.57 (s, 6H), 2.75-2.42 (m, 2H), 3.58-3.40 (m, 1H), 3.87 (br. s, 1H)
Mass (FAB): 406 ($M^+$+1), 405 ($M^+$)

Example 53

Preparation of 3α,7α,12α-trihydroxy-25-azacoprostane
Yield 92% mp (foam)
IR ($CHCl_3$): 3613, 3417 (br), 2945 and 2867 $cm^{-1}$
$^1$HNMR (300 MHz, $CDCl_3$): 0.70 (s, 3H), 0.91 (s, 3H), 1.01 (d, J=6.4 Hz, 3H), 2.40-0.98 (m, 29H), 2.23 (s, 6H), 3.55-3.40 (m, 1H), 3.90-3.82 (m, 1H), and 4.00 (br s, 1H)
Mass (FAB): Calc for $C_{26}H_{48}NO_3$, 422.3634
Found: 422.3635

| Elem. Anal. Calcd for ($C_{26}H_{47}NO_5$ $H_2O$) | | | |
|---|---|---|---|
| | C, 71.03; | H, 11.23; | N, 3.19 |
| Found: | C, 70.94; | H, 10.93; | N, 2.95 |

Example 54

Preparation of 3α,7α-dihydroxy-25-azacoprostane
Yield 83%; mp ($CH_3OH/CH_2Cl_2/Et_2O$): 195°C
IR (KBr): 3413 (br), 2935, 2862 $cm^{-1}$
NMR (300 MHz, $CDCl_3$): 0.69 (s, 3H), 0.95 (d, J=6.5 Hz, 3H), 0.96 (s, 3H), 1.98-0.95 (m, 27H), 2.02 (br d, J=12.1 Hz, 1H), 2.38 (br s. 8H, $CH_2N(CH_3)_2$), and 3.70 - 3.55 (m, 2H)
Mass (FAB): Calc for $C_{26}H_{48}NO_2$ 406.3685
Found: 406. 3697

Example 55

A.

Preparation of 3,3-ethylendioxy-17-β-pentyloxycholest-5-ene

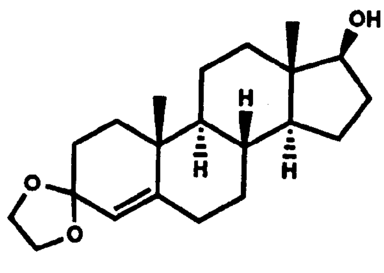
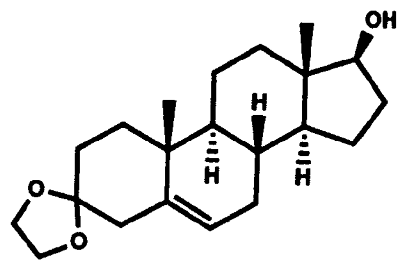

(2,3)

Testosterone, (14 g, 48 mmol), was mixed with toluene (100 mL), ethylene glycol (100 mL) and p-toluenesulfonic acid monohydrate (1.4 g, 7 mmol). The mixture was refluxed with a Dean-Stark trap for 8 h. Concentration in vacuo gave an oil that was mixed with EtOAc, washed with a saturated $NaHCO_3$ solution, dried over $Na_2SO_4$, and concentrated under reduced pressure. The residue was purified by preparative silica gel HPLC (15-30% EtOAc-hexanes) to give 10.1 g (62%) of a colorless oil as a 4:1 mixture of isomers **2** and **3**, respectively. $^1$H-NMR

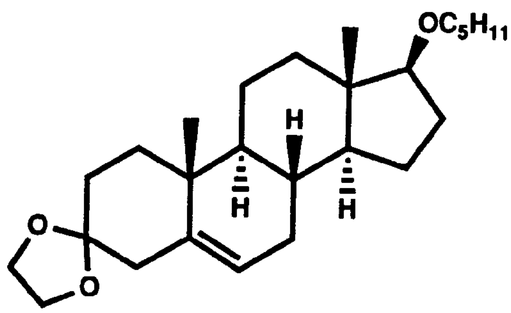

(4)

B.

To a flask containing potassium hydride (0.72 g, 0.18 mmol) under argon was added dry DMSO (20 mL). The mixture was warmed gently until gas evolution stopped. To this was added a solution of isomers 2 and 3 (4 g, 12.1 mmol) in dry THF (25 mL). This was cooled to 0°C before adding pentyl bromide (2.7 g, 18 mmol) as a neat liquid all at once. After stirring overnight at ambient temperature, the reaction was poured into water and extracted with EtOAc. The extracts were washed with brine several times, dried over $MgSO_4$, then concentrated under reduced pressure to a solid which was purified by flash chromatography ($SiO_2$, 10-50% EtOAc-hexanes) to yield 1.89 g starting material and 1.42 g (55%) of 4. $^1$H-NMR

Example 56

Preparation of 3,3-ethylendioxy-17β-octyloxycholest-5-ene

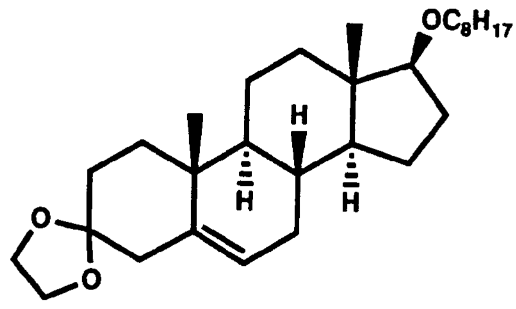

**(5)**

To sodium hydride (3 g, 76 mmol, 60% oil dispersion) which had been washed with hexanes under argon was added dry DMSO (50 mL). The mixture was warmed to 80°C until gas evolution stopped. Cooled to 10°C and added a solution of isomers 2 and 3 prepared according to Example 55, part A, in dry THF (60 mL). Octyl bromide (7.34 g, 38 mmol, passed through neutral alumina) was added. The cooling bath was removed and the reaction was stirred at ambient temperature overnight. The reaction mixture was quenched with $H_2O$, extracted with EtOAc, the organic layer washed with brine several times and dried over $MgSO_4$, then concentrated under reduced pressure to a solid which was chromatographed ($SiO_2$, 10-15% EtOAc-hexanes) to yield 1.89 g of **5** as a white solid. $^1$H-NMR

Example 57

Preparation of 3,3-ethylendioxy-17β-(4-methylpentyloxy)cholest-5-ene

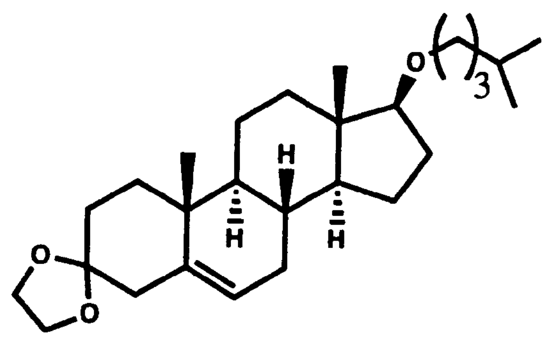

**(6)**

To a flask containing potassium hydride (2 g, 50.6 mmol) and dry THF (20 mL) under argon was added a solution of isomers 2 and 3 prepared according to Example 55, part A, in THF (30 mL). The mixture was diluted with DMF (10 mL) and cooled to 0°C before adding 1-bromo-4-methyl pentane (8.34 g, 50.6 mmol) dropwise and the cooling bath removed. After stirring for 2 h at ambient temperature, the reaction was quenched with $H_2O$ and extracted with EtOAc. The organic extracts were washed with brine then concentrated under reduced pressure. The resulting solid was purified by flash chromatography ($SiO_2$, 2.5-5% EtOAc-hexanes) to yield 3.65 g (87%) of **6** as a solid. $^1$H-NMR, IR, MS. Anal. ($C_{27}H_{44}O_3$) C, H, N

Example 58

Preparation of 17β-pentyloxy-4-cholesten-3-one

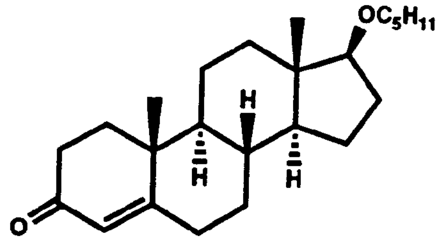

(7)

The compound of Example 55, part B, prepared according to the procedures described in Example 55 (4.22 g, 10.5 mmol) was mixed with acetic acid (66 mL), $H_2O$ (33 mL) and THF (50 mL). The resulting mixture was heated at 80°C for 3 h, concentrated in vacuo, mixed with toluene and reconcentrated under reduced pressure. The residue was purified by flash chromatography ($SiO_2$, 10-15% EtOAc-hexanes) to yield 3.6 g of starting material and 8.1 g (87%) of **7** as a white solid. $^1$H-NMR

Example 59

Preparation of 17β-octyloxy-4-cholesten-3-one

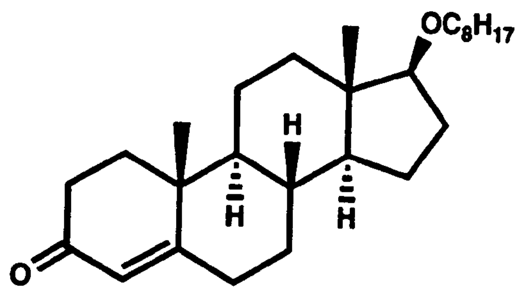

(8)

The compound of Example 56, prepared according to the procedures described there (7.6 g, 17.1 mmol) was mixed with acetic acid (75 mL), $H_2O$ (25 mL) and THF (50 mL). The resulting mixture was heated at 80°C for 5 h, concentrated in vacuo, mixed with toluene and reconcentrated under reduced pressure. The residue was purified by flash chromatography ($SiO_2$, 15% EtOAc-hexanes) to yield 6 g (87%) of **8** as a colorless oil.

Example 60

Preparation of 17β-(4-methylpentyloxy)-4-cholesten-3-one

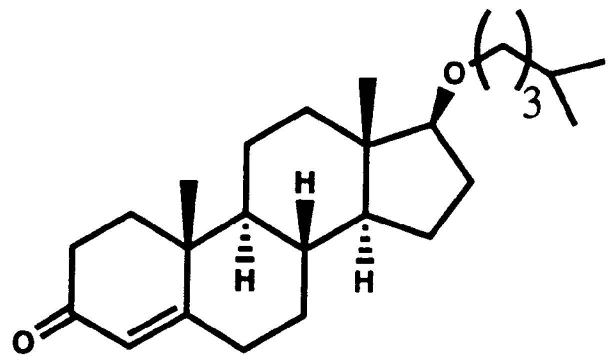

**9**

The title compound was prepared by substantially following the procedures described in Example 59. (87%). $^1$H-NMR, IR, MS. Anal. ($C_{27}H_{44}O_3$) C, H, N

Example 61

Preparation of 4α-(2-propenyl)-17β-pentyloxycholestan-3-one

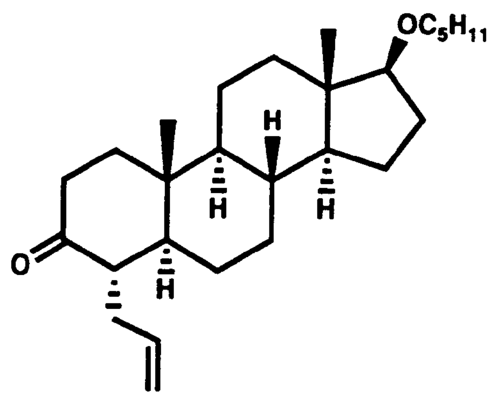

**(10)**

Ammonia (35 mL) was condensed into a flask containing lithium metal (0.2 g, 29 mmol) under argon by immersing it in a dry ice/acetone bath. Cooling was continued with stirring for 10 min before adding dry THF (10 mL) followed by a solution of the compound of Example 58 (3.44 g, 10 mmol) and $H_2O$ (130 µL, 8 mmol) in THF (15 mL). The cooling bath was removed and stirred for 10 min. Isoprene (2 mL), was added and the mixture stirred for 5 min then recooled to -78. Allyl bromide (3.6 g, 30 mmol) was added and stirring continued for 7 min before quenching with $NH_4Cl$ (2 g, 37 mmol) and $H_2O$ (10 mL). The organics were extracted with EtOAc, dried over $MgSO_4$ and concentrated under reduced pressure to an oil which was purified by flash chromatography ($SiO_2$, 10-15% EtOAc-hexanes) to yield 0.94 g (24%) of **10** as an oil. $^1$H-NMR

Example 62

Preparation of 4α-(2-propenyl)-17β-octyloxycholestan-3-one

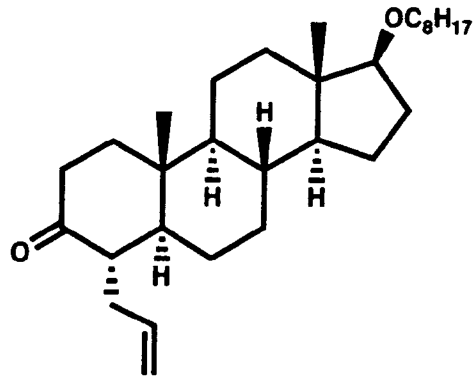

(11)

Ammonia (25 mL) was condensed into a flask containing lithium metal (0.3 g, 44.2 mmol) under argon by immersing it in a dry ice/acetone bath. Cooling was continued with stirring for 10 min before adding dry THF (20 mL) followed by a solution of the compound of Example 59 (5.9 g, 15 mmol) and $H_2O$ (270 μL, 15 mmol) in THF (20 mL). The cooling bath was removed and stirring was continued for 15 min. Isoprene (2 mL), was added and the reaction mixture stirred for 10 min then recooled to -78. Allyl bromide (9.1 g, 75 mmol) was added and stirring continued for 8 min before quenching with $NH_4Cl$ (5 g, 94 mmol) and $H_2O$ (10 mL). Brine was added and the organics extracted with EtOAc, dried over $MgSO_4$ and concentrated under reduced pressure to an oil which was purified by preparative silica gel HPLC (5% EtOAc-hexanes) to yield 1.72 g (26%) of **11** as an oil. $^1$H-NMR

Example 63

Preparation of 4α-(2-propenyl)-17β-(4-methylpentyloxy)cholestan-3-one

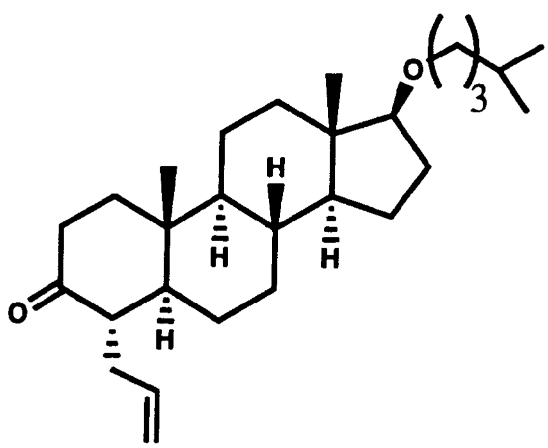

(12)

By substantially following the procedures described in Example 61, the title compound was prepared. (yield: 42%)

Example 64

Prearation of (3α, 4α, 5α)-17-(pentyloxy)-4-(2-propenyl)androstan-3-ol

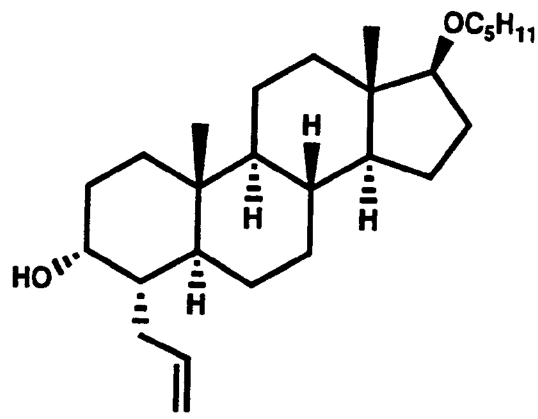

(13)

To a solution of the compound of Example 61 (400 mg, 1 mmol) in dry THF (30 mL) under argon at 78°C was added K-Selectride®, (2 mL, 2 mmol, 1 M in THF) dropwise. The cooling bath was removed and the reaction mixture was stirred at ambient temperature overnight. Cooled in ice bath, then quenched with $H_2O$ (0.18 mL), EtOH (0.68 mL), 30% $H_2O_2$ (0.68 mL) and 5 N NaOH (0.5 mL), concentrated in vacuo, and the residue mixed with EtOAc and brine. The organic layer was dried over $MgSO_4$ and concentrated under reduced pressure to an oil which was purified by flash chromatography ($SiO_2$, 5% EtOAc-hexanes) to yield 196 mg (24%) of **13** as a white solid, mp 74-75°C. $^1$H-NMR, IR, MS. Anal. ($C_{27}H_{46}O_2$) C, H, N.

Example 65

Preparation of (3α,4α,5α)-17-(octyloxy)-4-(2-propenyl)androstan-3-ol

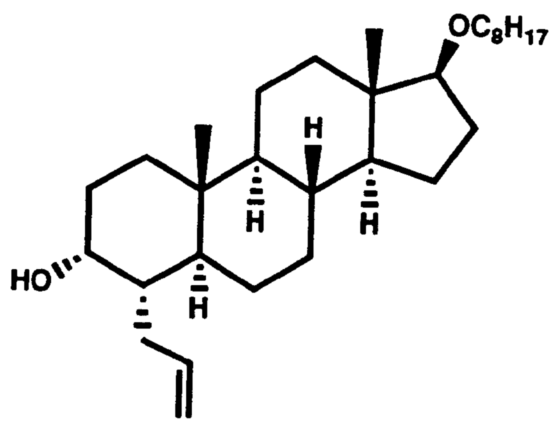

(14)

To a solution of the compound of Example 62 (.6 g, 1.4 mmol) in THF (10 mL) and EtOH (10 mL) at room temperature was added $NaBH_4$ (0.1 g, 2.8 mmol). After stirring for 2 h, the reaction was quenched with 1 N HCl (3 mL) then concentrated in vacuo. The resulting solid was partitioned between 2 N NaOH and EtOAc. The organic layer was dried over MgSO4 and concentrated under reduced pressure to an oil which was purified by flash chromatography ($SiO_2$, 10% EtOAc-hexanes) to yield 118 mg of the desired isomer **14** (20%), mp 57-60°C. Anal. ($C_{30}H_{52}O_2$) C, H, N. Also recovered was 340 mg of the equatorial isomer, 3β-ol, mp 103-105°C. Anal. ($C_{30}H_{52}O_2$) C, H, N.

Example 66

Preparation of (3α,4α)-17-[(4-methylpentyl)oxy]-4-(2-propenyl)androstan-3-ol

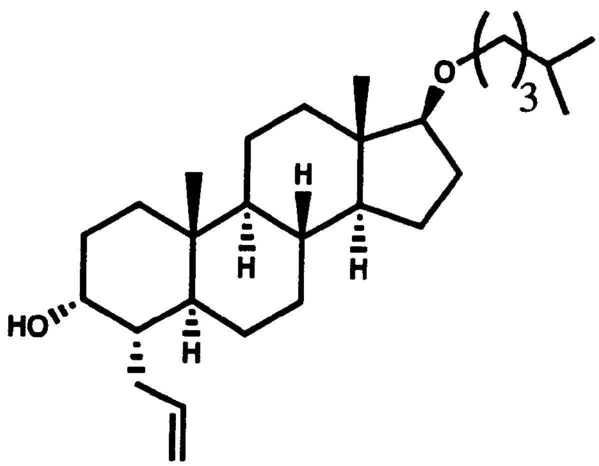

(15)

By substantially following the procedures described in Example 64, the title compound was prepared. (yield: 87%), mp 68-71°C. $^1$H-NMR, IR, MS. ($C_{28}H_{48}O_2$)

Example 67

Preparation of (3α,4α)-17-(3-phenylpropoxy)-4-(2-propenyl)androstan-3-ol

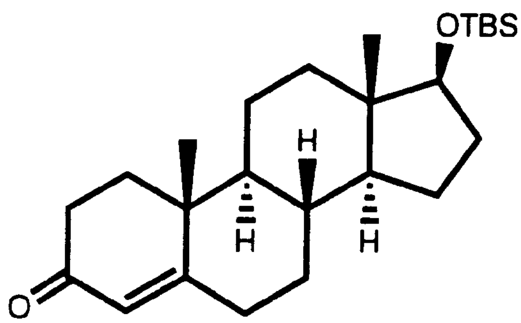

(16)

A.

Testosterone (22.38 g, 77.6 mmol), imidazole (13.2 g, 194 mmol) and t-butyldimethylsilyl (TBS) chloride (17.6 g, 117 mmol) were combined with dry DMF (150 mL), and the mixture stirred at room temperature overnight. The reaction mixture was quenched with $H_2O$ and extracted with EtOAc. The organic layer was washed with $H_2O$ 3 times and brine 3 times, dried over $MgSO_4$ and concentrated under reduced pressure to yield 30.5 g (>100%) of **16** that was used without further purification.

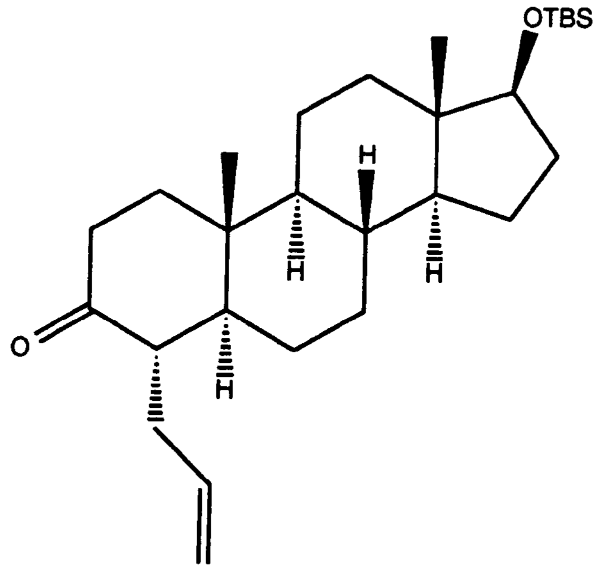

(17)

(17)

B.

Ammonia (25 mL) was condensed into a flask containing lithium metal (1.2 g, 167 mmol) under argon by immersing it in a dry ice/acetone bath. Dry THF (50 mL) was added and cooling continued while stirring for 5 min before adding a solution of **16** (30.5 g, 75.9 mmol) and t-butanol (5.7 mL, 61 mmol) in THF (125 mL). The cooling bath was removed and the reaction mixture stirred for 5 min. Allyl bromide (29 g, 239 mmol) was added followed by isoprene (10 mL) and stirring continued for 30 min. The reaction mixture was quenched with $NH_4Cl$ (10 g, 187 mmol) and $H_2O$, and extracted with EtOAc. The organic layer was dried over $MgSO_4$ and concentrated under reduced pressure to an oil that was purified by preparative silica gel HPLC (2% EtOAc-hexanes) to yield 15.5 g (35%) of the desired compound (17) as an oil. $^1$H-NMR

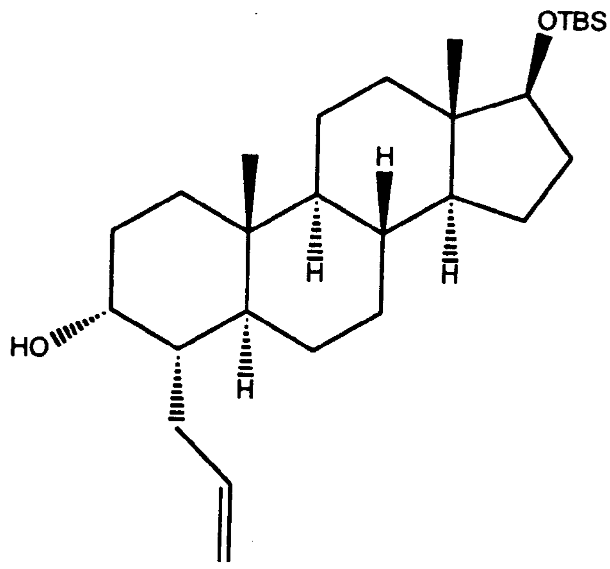

(18)

C.

To a solution of **17** (15 g, 34 mmol) in dry THF (60 mL) under argon at -78°C was added K-Selectride®, (68 mL, 68 mmol, 1 M in THF) dropwise. The cooling bath was removed and the reaction mixture was stirred at ambient temperature for 48 h. The reaction mixture was cooled in ice bath, then quenched with 5 N NaOH (15 mL) and 30% $H_2O_2$ (15 mL). The cooling bath was removed and the reaction mixture stirred at ambient temperature for 1 h. The reaction mixture was concentrated under reduced pressure, mixed with brine and extracted with EtOAc. The organic layer was dried over $MgSO_4$ and concentrated under reduced pressure to an oil which was purified by preparative silica gel HPLC (5% EtOAc-hexanes) to yield 10.57 g (70%) of **18** as a white solid.

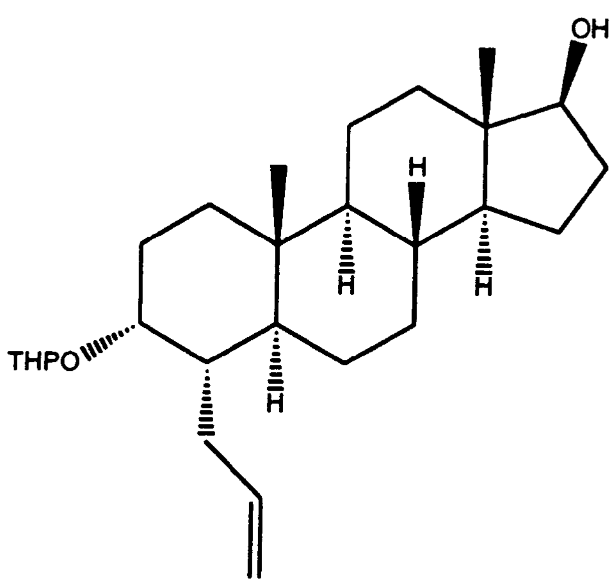

(19)

D.

To a solution of **18** (10 g, 22.4 mmol) in $CH_2Cl_2$ (50 mL) was added 3,4-dihydro-2H-pyran (5.7 g, 67.3 mmol) and pyridinium p-toluenesulfonate (0.6 g, 2.4 mmol) and the reaction mixture stirred at room temperature overnight. The mixture was concentrated under reduced pressure and partitioned between EtOAc and $H_2O$. The organic layer was washed with $H_2O$ then brine, dried over $MgSO_4$ and concentrated under reduced pressure. The residue was mixed with THF (50 mL) and treated with tetra-n-butyl ammonium fluoride (112 mL, 112 mmol, 1 M in THF) at 60°C for 16 h. The mixture was concentrated under reduced pressure, mixed with hexanes/EtOAc (1/1) and washed with $H_2O$ several times. The organic layer was dried over $MgSO_4$ and concentrated under reduced pressure to give an oil which was purified by flash chromatography ($SiO_2$, 10-15% EtOAc-hexanes) to yield 9 g (97%) of **19** as an oil. ("THP" is tetrahydropyran-4-yl).

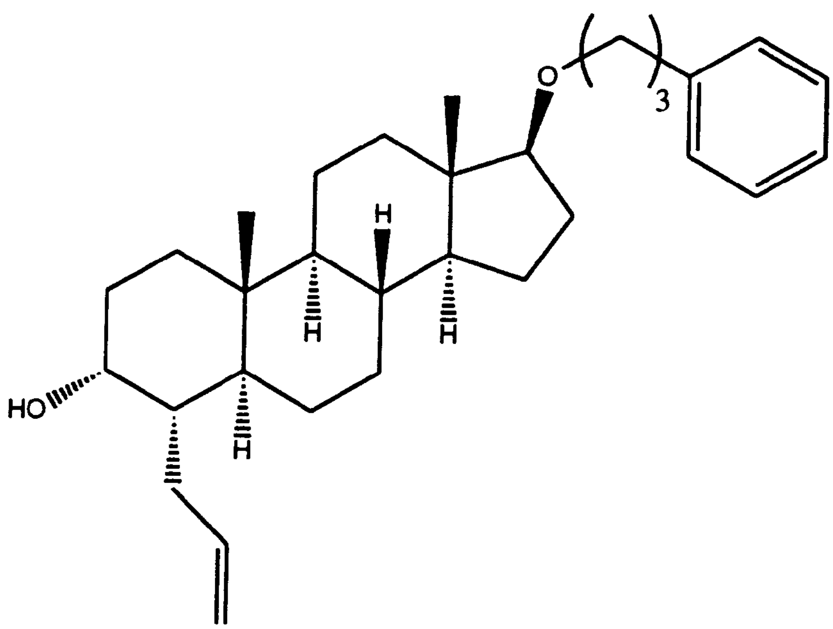

(20)

E.

To a flask containing potassium hydride (96 mg, 2.5 mmol) and dry THF (5 mL) under argon was added a solution of **19** in THF (10 mL). This mixture was cooled to 0°C and diluted with DMF (3 mL) before adding 1-bromo-3-phenyl propane (0.48 g, 2.5 mmol) as a neat liquid all at once. The cooling bath was removed. After stirring overnight at ambient temperature, the reaction was quenched with $H_2O$ and extracted with EtOAc. The extracts were washed with brine then concentrated under reduced pressure. The resulting oil was mixed with acetic acid (5 mL), $H_2O$ (2 mL) and THF (5 mL). This mixture was heated at 80°C overnight then concentrated under reduced pressure. The residue was purified by flash chromatography ($SiO_2$, 5% EtOAc-hexanes) to yield 150 mg (86%) of **20** as a solid, mp 108-109°C. $^1H$-NMR, IR, MS. ($C_{31}H_{46}O_2$)

Example 68

Preparation of (3α,4α)-17-(phenylmethoxy)-4-(2-propenyl)androstan-3-ol:

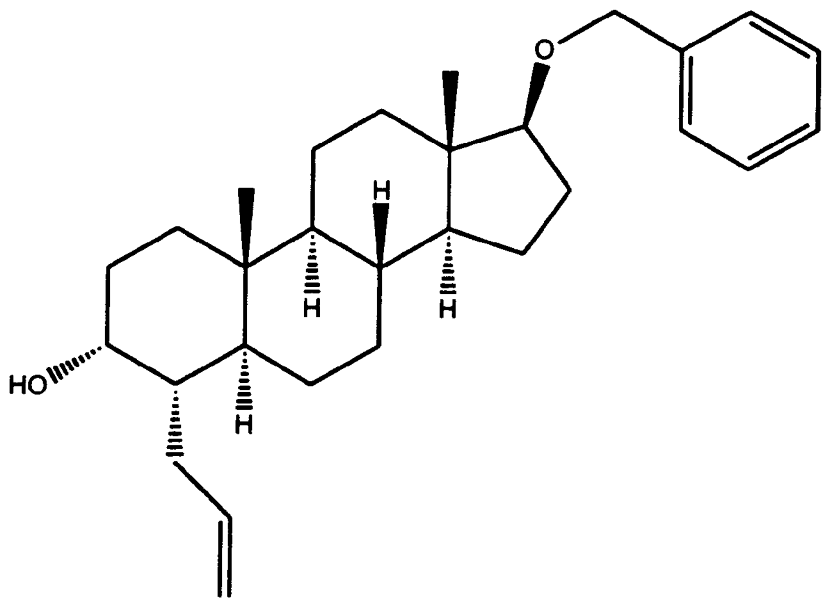

(21)

by substantially following the procedures of Example 67, the title compound was prepared. (yield: 62%), mp 146-147°C. $^1$H-NMR, IR, MS. Anal. ($C_{29}H_{42}O_2$) C, H, N

Example 69

Preparation of (3α,4α)-17-[(4,4-dimethylpentyl)oxy]-4-(2-propenyl)-androstan-3-ol

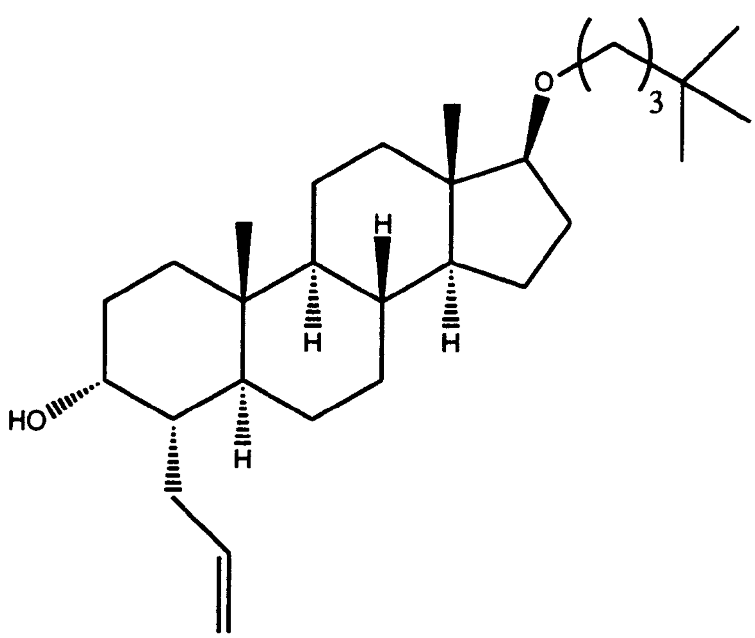

(22)

By substantially following the procedures of Example 67, the title compound was prepared (yield: 98%), mp 139-141°C. $^1$H-NMR, IR, MS. Anal. ($C_{29}H_{50}O_2$) C, H, N

Example 70

Preparation of (3α,4α)-17-(butoxymethyl)-4-(2-propenyl)androstan-3-ol

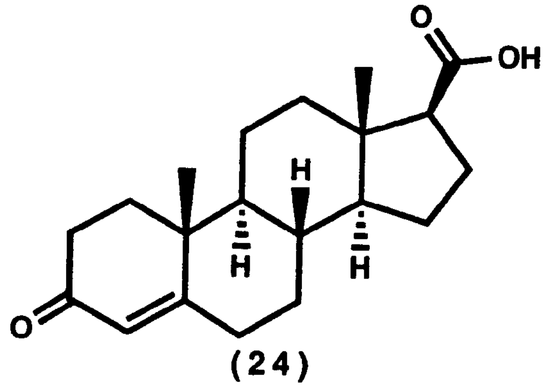

(24)

A.

Bromine (18 g, 113 mmol) was added dropwise to a solution of NaOH (33.3 g, 832 mmol) in $H_2O$ (300 mL) at -5°C. The mixture was stirred for 15 min at -5°C then diluted with cold (12°C) dioxane. The resulting solution was immediately added to a stirred mixture of progesterone (20 g, 64 mmol), dioxane (1.1 L) and $H_2O$ (310 mL) at 8°C. After stirring for 4 h while keeping the temperature below 10°C, the mixture was heated to reflux and a solution of $Na_2SO_3$ (10 g, 79 mmol) in $H_2O$ (100 mL) was added. The mixture was neutralized with conc. HCl (50 mL), cooled to room temperature, and extracted with EtOAc. The extracts were concentrated in vacuo and partitioned between 1.4 N NaOH (550 mL) and EtOAc. The aqueous layer was washed with EtOAc, acidified with conc HCl and extracted with EtOAc. The extracts were dried over $MgSO_4$ and concentrated under reduced pressure to afford 17.68 g of **24** as an off-white solid (88%). $^1$H-NMR

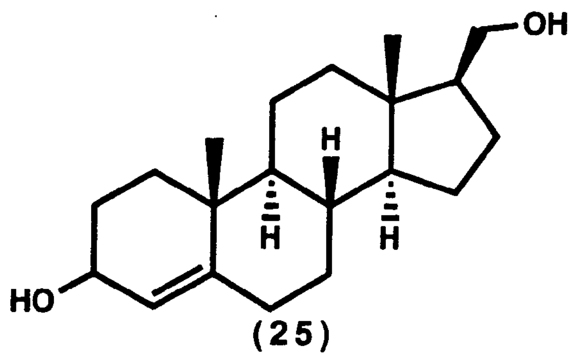

(25)

B.

Red-Al® (81 mL, 275 mmol, 3.4 M in toluene) was added dropwise to a solution of **24** (14.69 g, 46 mmol) in THF (500 mL) under argon at 0°C. The cooling bath was removed After stirring at ambient temperature for 2 h the reaction was quenched by the addition of 1 N HCl (600 mL) dropwise. It was then extracted with EtOAc several times. The combined organic layers were dried over $Na_2SO_4$ and concentrated under reduced pressure to yield 13.64 g (98%) of a solid. $^1$H-NMR

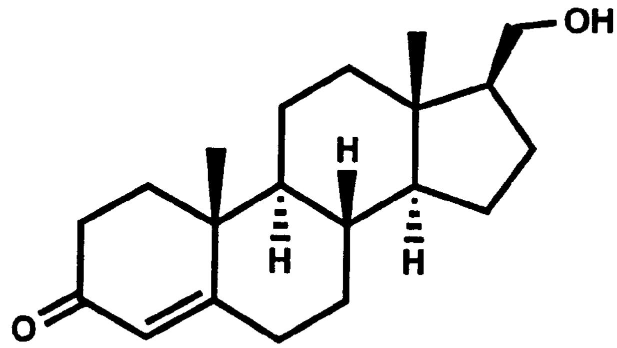

**(26)**

C.

To a mixture of **25** (13.3 g, 44 mmol) and $CHCl_3$ (500 mL) was added $MnO_2$ (66.5 g, 0.74 mol). The reaction was stirred overnight at room temperature then concentrated to half its original volume under reduced pressure using a 55°C water bath. The mixture was filtered through Celite and concentrated to yield 13.3 g (quantitative) of **26**. $^1$H-NMR

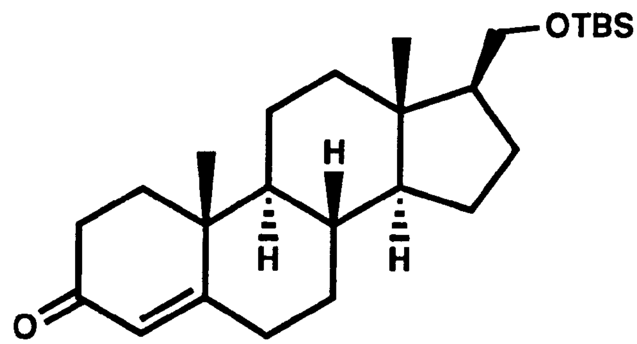

(27)

D.

The compound **26** (13.3 g, 44 mmol), imidazole (7.48 g, 110 mmol) and t-butyldimethylsilyl (TBS) chloride (10 g, 66 mmol) were combined with dry DMF (30 mL)and stirred at room temperature for 72 h. The reaction mixture was quenched with $H_2O$ and extracted with EtOAc. The organic layer was washed with $H_2O$ twice and brine 3 times, dried over $MgSO_4$ and concentrated under reduced pressure to yield 17 g of an oil. This material was purified by flash chromatography ($SiO_2$, 5-15% EtOAc-hexanes) to yield 10.42 g (57%) of **27** as an oil that crystallized on standing. $^1$H-NMR

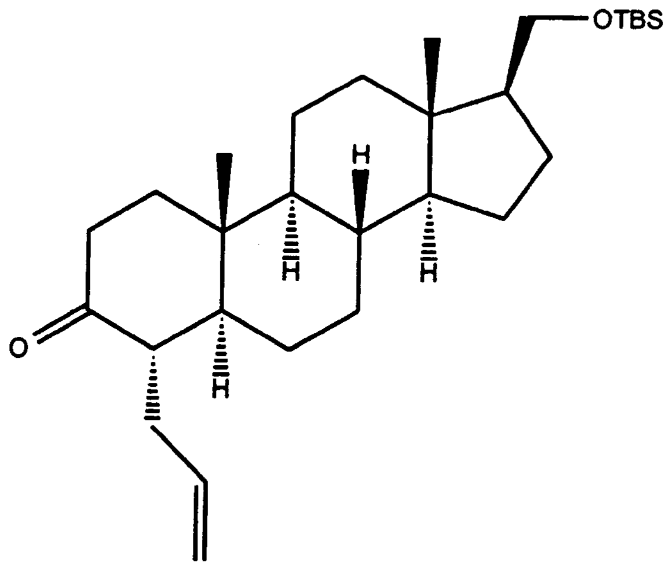

(28)

E.

Ammonia (25 mL) was condensed into a flask containing lithium metal (0.42 g, 61.3 mmol) under argon by immersing it in a dry ice/acetone bath. Cooling was continued with stirring for 10 min before adding dry THF (20 mL) followed by a solution of **27** (10.2 g, 24.5 mmol) and t-butanol (1.4 mL, 14.7 mmol) in THF (20 mL). The cooling bath was removed and the reaction mixture and stirred for 10 min. The reaction mixture was cooled to -78°C and isoprene (5 mL) added. The reaction mixture was stirred for 10 min before adding allyl bromide (8.9 g, 74 mmol). Stirring was continued for 15 min then quenched with $NH_4Cl$ (10 g, 187 mmol) and $H_2O$ and extracted with EtOAc. The organic layer was dried over $MgSO_4$ and concentrated under reduced pressure to an oil which was purified by flash chromatography ($SiO_2$, 2.5% EtOAc-hexanes) to yield 2.14 g (19%) of **28** as an oil. $^1$H-NMR

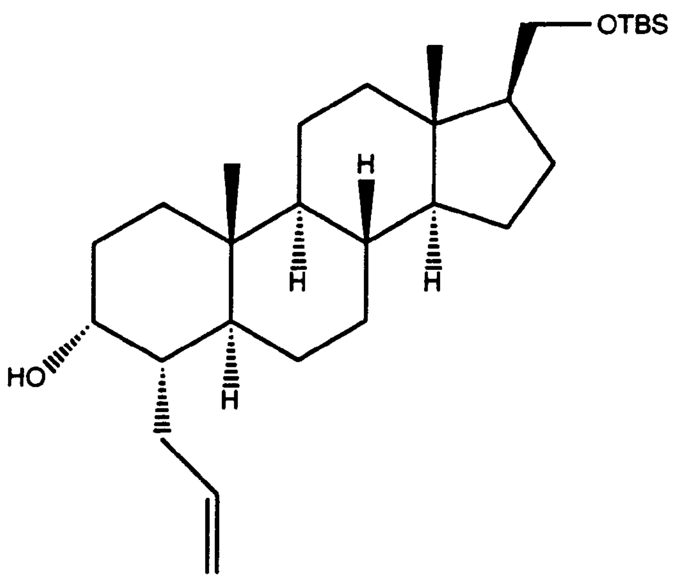

(29)

F.

(3α,4α)-17-[[[(1,1-dimethylethyl)dimethylsilyl]oxy]methyl]-4-(2-propenyl)androstan-3-ol

To a solution of **28** (2 g, 4.4 mmol) in dry THF (20 mL) under argon at -78°C was added K-Selectride® (2 mL, 2 mmol, 1 M in THF) dropwise. The cooling bath was removed and the reaction mixture stirred at ambient temperature for 36 h. After cooling in an ice bath, the reaction mixture was quenched with 5 N NaOH (3 mL) and 30% $H_2O_2$ (3 mL). The cooling bath was removed and the reaction mixture stirred at ambient temperature for 1 h. The reaction mixture was concentrated under reduced pressure, mixed with brine and extracted with EtOAc. The organic layer was dried over $MgSO_4$ and concentrated under reduced pressure to an oil that was purified by flash chromatography ($SiO_2$, 5% EtOAc-hexanes) to yield 1.09 g (54%) of **29** as a white solid, mp 160-161°C.

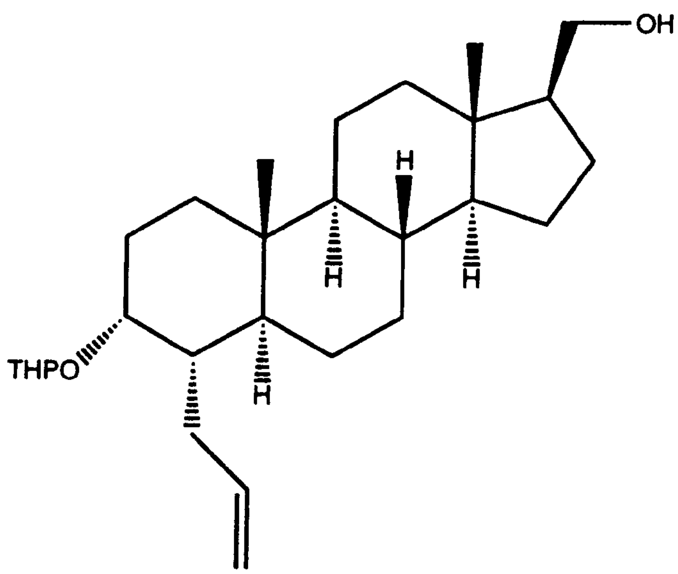

(30)

G.

To a solution of **29** (1 g, 2.2 mmol) in $CH_2Cl_2$ (30 mL) was added 3,4-dihydro-2H-pyran (0.6 g, 6.6 mmol) and pyridinium p-toluenesulfonate (55 mg, 0.22 mmol). The reaction was stirred at room temperature overnight, concentrated under reduced pressure, and partitioned between EtOAc and $H_2O$. The organic layer was washed with $H_2O$ and brine then dried over $MgSO_4$ and concentrated under reduced pressure. The residue was mixed with THF (25 mL) and treated with tetra-n-butyl ammonium fluoride (11 mL, 11 mmol, 1 M in THF) at room temperature for 2 h. The reaction was concentrated under reduced pressure, mixed with hexanes/EtOAc (1/1) and washed with $H_2O$ several times. The organic layer was dried over $MgSO_4$ and concentrated under reduced pressure to give an oil that was purified by flash chromatography ($SiO_2$, 10-15% EtOAc-hexanes) to yield 0.9 g (95%) of **30** as an oil. (THP is tetrahydropyran-4-yl) $^1$H-NMR

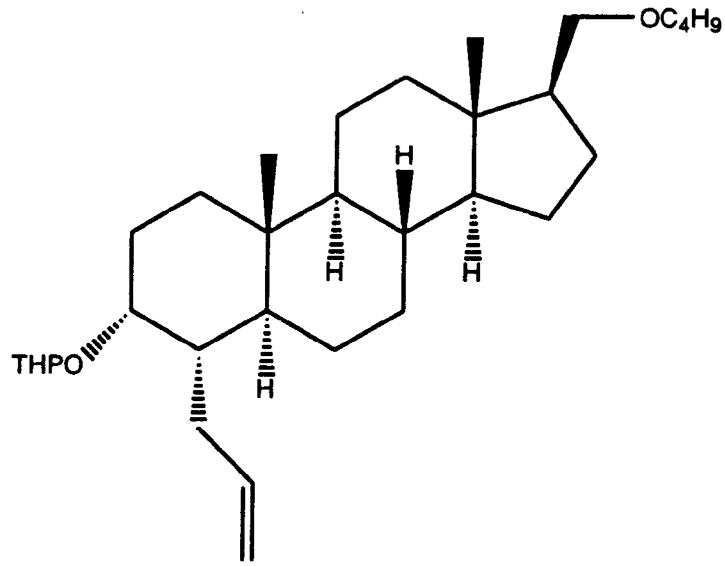

(31)

H.

To a flask containing potassium hydride (0.19 g, 4.75 mmol) and dry THF (5 mL) under argon was added a solution of **30** in THF (5 mL). This mixture was cooled to 0°C before adding butyl bromide (0.64 g, 4.6 mmol) as a neat liquid all at once and removed from the cooling bath. After stirring for 1 h at ambient temperature, the reaction mixture was quenched with brine and extracted with EtOAc. The extracts were washed with brine, dried over $MgSO_4$, then concentrated under reduced pressure. The resulting oil was purified by flash chromatography ($SiO_2$, 2.5% EtOAc-hexanes) to yield 355 mg (81%) of **31**.

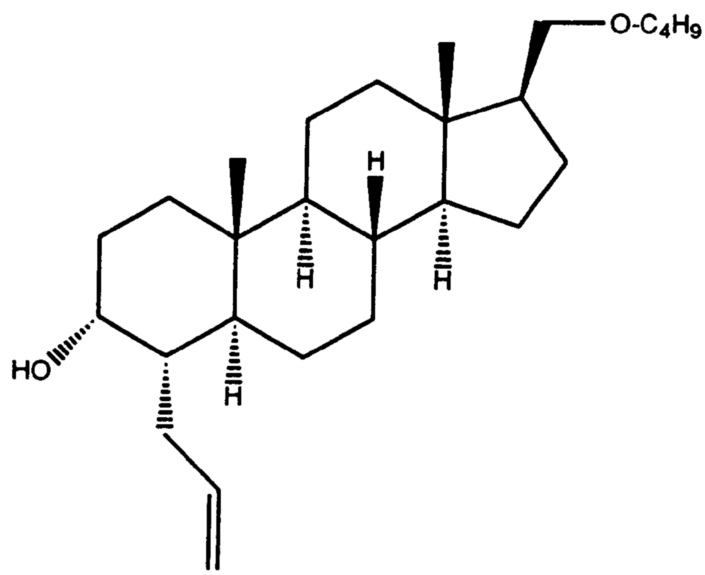

(33)

I.

(3α,4α)-17-(butoxymethyl)-4-(2-propenyl)androstan-3-ol

Intermediate **31** (300 mg, 0.6 mmol) was mixed with acetic acid (4 mL), $H_2O$ (1 mL) and THF (1 mL). The mixture was heated at 60°C for 4 h then concentrated under reduced pressure. The residue was purified by flash chromatography ($SiO_2$, 1% EtOAc-hexanes) to yield 198 mg (82%) of **33** as a solid, mp 96-98°C. $^1$H-NMR, IR, MS. Anal. ($C_{27}H_{46}O_2$ . 0.22 $H_2O$) C, H, N

Example 71

Preparation of (3α,4α)-17-[(heptyloxy)methyl]-4-(2-propenyl)androstan-3-ol:

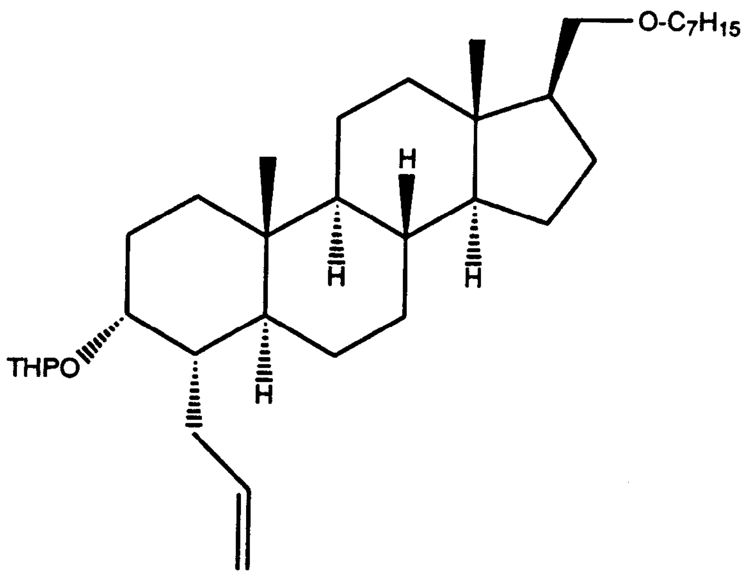

(32)

A.

To a flask containing potassium hydride (0.19 g, 4.75 mmol) and dry THF (10 mL) under argon was added a solution of **30** prepared according to the procedures described in Example 70, A through G, in THF (10 mL). This mixture was cooled to 0°C and diluted with DMF (5 mL) before adding heptyl bromide (0.83 g, 4.6 mmol) as a neat liquid all at once. The cooling bath was removed. After stirring for 48 h at ambient temperature, the reaction mixture was quenched with brine and extracted with EtOAc. The extracts were washed with brine, dried over $MgSO_4$, then concentrated under reduced pressure. The resulting oil was purified by flash chromatography ($SiO_2$, 2.5% EtOAc-hexanes) to yield 436 mg (92%) of **32**.

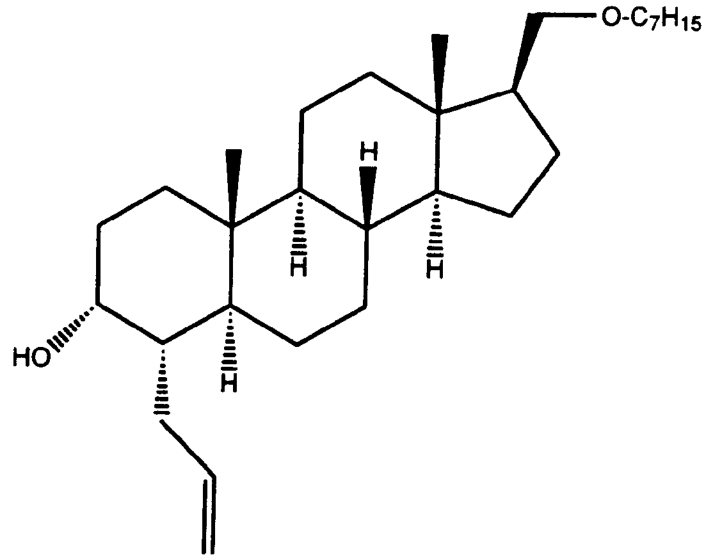

(34)

B.

(3α,4α)-17-[(heptyloxy)methyl]-4-(2-propenyl)androstan-3-ol

Intermediate **32** (330 mg, 0.63 mmol) was mixed with acetic acid (4 mL), $H_2O$ (1 mL) and THF (2 mL). The mixture was heated at 80°C overnight then concentrated under reduced pressure. The residue was purified by flash chromatography ($SiO_2$, 1-5% EtOAc-hexanes) to yield 238 mg (86%) of **34** as a solid, mp 62-64°C. $^1H$-NMR, IR, MS. ($C_{30}H_{52}O_2$)

Example 72

Preparation of 4α-4-(2-propenyl)cholestan-3-one

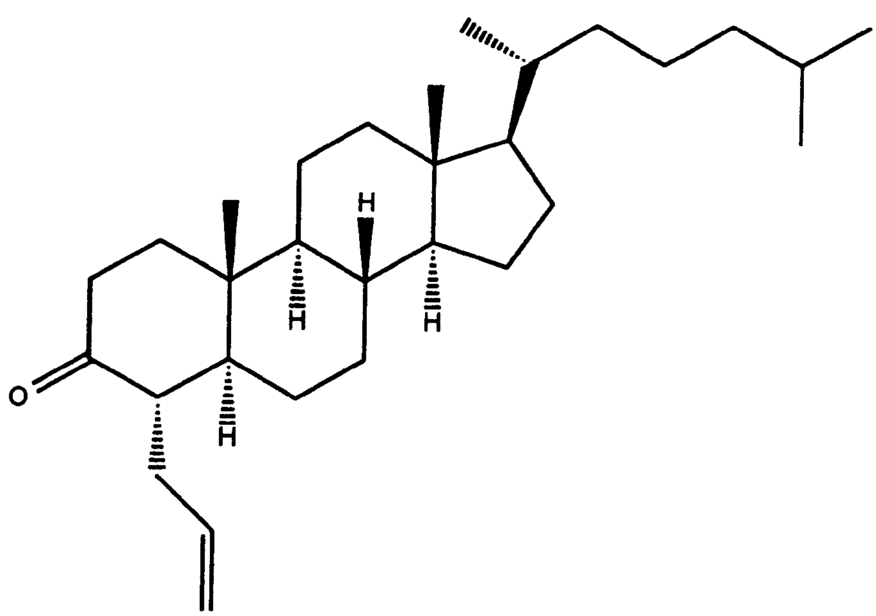

(36)

By substantially following the procedures described in Example 67, step B and starting from 4-cholesten-3-one, the title compound was prepared. (Yield: 16%). $^1$H-NMR, IR, MS. ($C_{30}H_{50}O$)

Example 73

Preparation of 4α-2-(hydroxymethylene)-4-(2-propenyl)cholestan-3-one

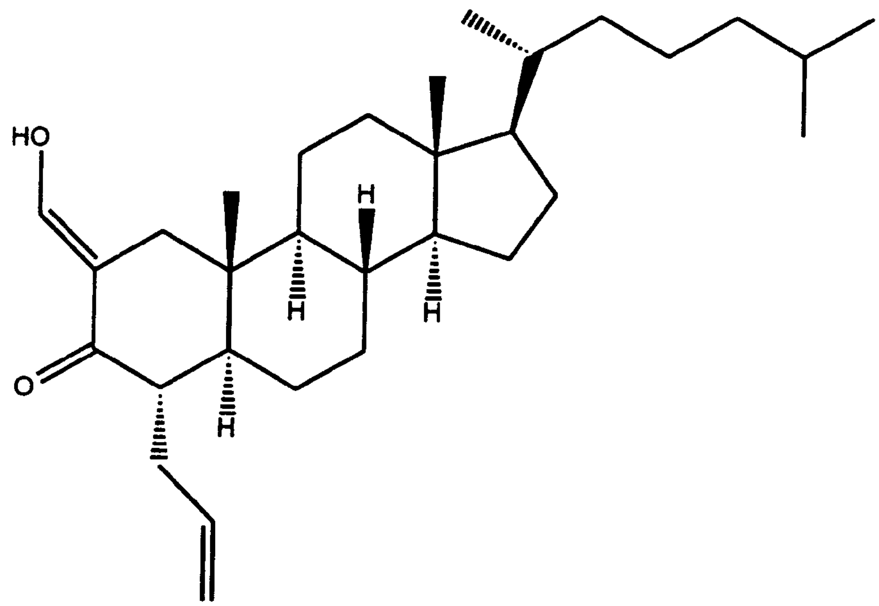

(37)

Sodium hydride (1.6 g, 41 mmol, 60% oil dispersion) was washed with hexanes under argon and mixed with toluene (50 mL). To the mixture was added a solution of the compound of Example 72 (3.5 g, 8.2 mmol) in toluene (50 mL) followed by ethyl formate (5.6 mL). The reaction mixture was stirred overnight at ambient temperature then quenched with $H_2O$ (5 mL). To the reaction mixture was added 1 N HCl (50 mL) and then the mixture extracted with EtOAc. The extracts were washed with 1 N HCl and brine and dried over $MgSO_4$. This material was concentrated under reduced pressure to give 3.6 g (97%) of **37** as an oil that crystallized on standing, mp 84-94°C. $^1$H-NMR

Example 74

Preparation of (4α)-2-(4-morpholinylmethylene)-4-(2-propenyl)cholestan-3-one

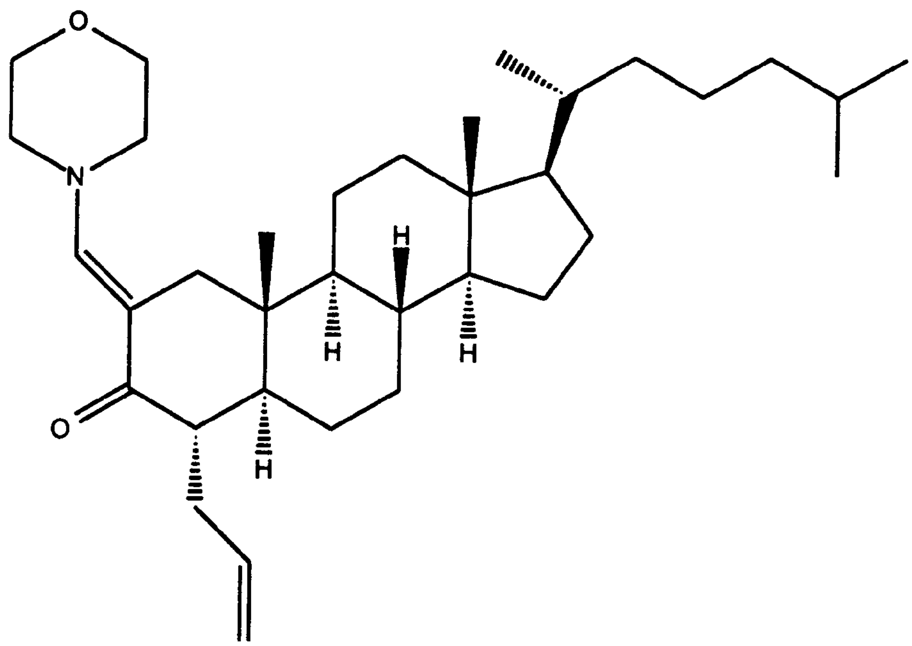

(38)

Morpholine (1.8 g, 20.5 mmol) was added to a solution of the compound of Example 73 (3.1 g, 6.8 mmol) in EtOH (30 mL) and refluxed for 2 h. The reaction was concentrated under reduced pressure and purified by flash chromatography ($SiO_2$, 1-5% MeOH-$CHCl_3$) to yield 3 g (84%) of **38** as a foam. $^1$H-NMR, IR, MS

Example 75

Preparation of (2α)-2-(2-propenyl)cholest-4-en-3-one.

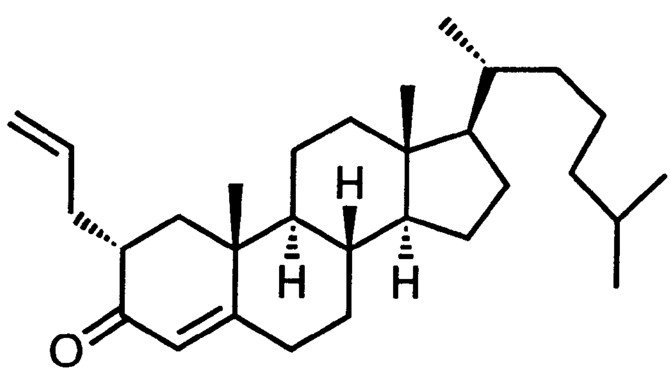

A ten ml solution of 4-cholesten-3-one (500 mg,1.30 mmol) in dry THF was added dropwise to a ten milliliter solution of $NaN(TMS)_2$ (1.69 mL, 1M in THF) at -78°C under argon, and the resultant golden solution was stirred for 45 min before it was treated with allyl iodide (238 mL, 2.60 mmol). The mixture was stirred at -78°C for 6 hr, and then warmed up slowly to ambient temperature. Acetic acid (0.5 mL) and EtOAc (30 mL) were added to the mixture, followed by saturated aq. NaCl (10 mL). The organic layer was separated, dried over $MgSO_4$, filtered and concentrated in vacuo to give an oily residue, which was subject to flash chromatography on silica (gradient EtOAc/hexane: 2% to 10%) to provide 422 mg (76%) of the title compound as an oil. IR ($CHCl_3$) 2933 and 1673 cm$^{-1}$; $^1$H NMR ($CDCl_3$, 300 MHz) δ 0.72 (3H, s), 0.87 (3H, d, J = 6.6Hz), 0.88 (3H, d, J = 6.6Hz), 0.92

(3H, d, J = 6.5Hz), 1.20 (3H, s), 0.75-1.70 (20H, m), 1.78-1.91 (2H, m), 1.98-2.14 (3H, m), 2.20-2.48 (3H, m), 2.68-2.77 (1H, m), 5.02-5.10 (2H, m), 5.72 (1H, d, J = 1.0Hz), and 5.75-5.88 (1H, m); FDMS: 424 ($M^+$); Anal. Calcd for $C_{30}H_{48}O$: C, 84.84; H, 11.39. Found: C, 84.64; H, 11.53.

Example 76

Preparation of (2α, 5α)-2-(2-propenyl)cholestan-3-one.

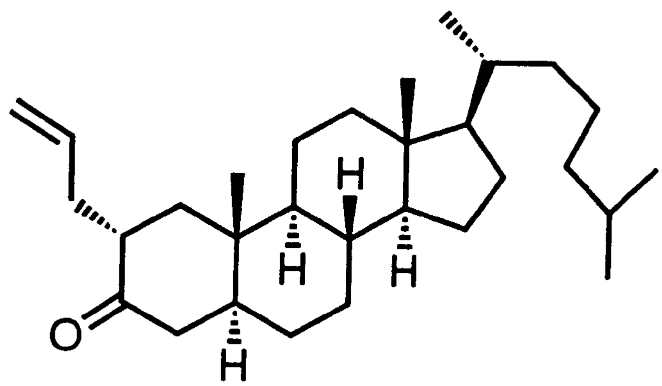

Lithium chip (24.6 mg, 3.54 mmol) and a glass-coated stir bar were placed in a flame-dried, three-necked, round-bottomed flask fitted with a dry ice condenser under argon. A thirty milliliter of liquid ammonia was collected in the flask at -78°C to form a deep blue solution, and then followed by the addition of dry THF (15 mL). A fifteen milliliter solution of (2α)-2-(2-propenyl)cholest-4-en-3-one (430 mg, 1.01 mmol) and t-BuOH (95.2 mL, 1.01 mmol) in dry THF was added dropwise to the deep blue solution. Upon completion of the addition, the resultant blue solution was stirred for 5 min before it was decolorized with a few drops of 1,3-pentadiene. Ten milliliter of saturated aq. $NH_4Cl$ was carefully added to the white suspension, then the cold bath was removed and the mixture was allowed to warm up to ambient temperature. Solid NaCl was added to saturate the aq. layer before it was extracted with EtOAc (30 mL); the organic layer was washed with saturated aq. NaCl (10 mL), dried over $MgSO_4$, filtered and concentrated to give an oily residue. After flash chromatography on silica (gradient toluene/hexane: 50% to 100%), 407 mg (94%) of the title compound was obtained as an oil. IR (neat) 2929 and 1711 $cm^{-1}$; $^1H$ NMR ($CDCl_3$, 300MHz) δ 0.69 (3H, s), 0.88 (6H, d, J = 6.6Hz), 0.91 (3H, d, J = 6.5Hz), 1.06 (3H, s), 0.70-2.15 (29H, m), 2.29-2.50 (2H, m), 2.52-2.62 (1H, m), 4.99-5.06 (2H, m) and 5.71-5.86 (1H, m); FDMS: 427 ($M^+$+1); Anal. Calcd for $C_{30}H_{50}O$: C, 84.44; H, 11.81. Found: C, 84.72; H, 11.68.

Example 77

This Example illustrates the preparation of compounds of the invention having a pharmaceutically active substituent at the 2 position of the sterol nucleus.

Preparation of (2α, 3α, 5α)-2-(2-propenyl)cholestan-3-ol.

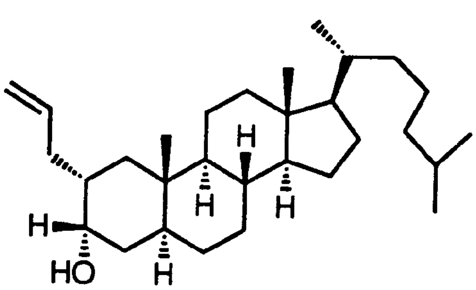

This Example illustrates the preparation of compounds of the invention having pharmaceutically active substituents at the 2 position of the sterol nucleus.

K-Selectride (23.6 mL, 1M in THF) was added to a stirred solution of (2α, 5α)-2-(2-propenyl)cholestan-3-one (5.03 g, 11.8 mmol) in dry THF (100 mL) at -78°C under argon, and the resultant yellowish solution was then stirred at 0°C for 1h. Excess K-selectride was carefully quenched with methanol (5 mL) and the solution was sequentially treated with 5N NaOH (14.1 mL, 70.8 mmol) and 30% $H_2O_2$ (7.20 mL, 70.8 mmol). The cold bath was removed and the mixture was stirred for 4h. Acetic acid (5 mL) and and EtOAc (100 mL) were added the mixture and then it was washed with saturated aq. NaCl (20 mL x 2), dried over $MgSO_4$, filtered and concentrated. The residue was subject to flash chromatography on silica (gradient toluene/hexane: 40% to 100%) to provide 3.92 g (77%) of the title compound, which was recrystallized from $Et_2O/CH_3CN$. mp: 72.0-73.5°C; IR (KBr) 3376 and 2931 $cm^{-1}$; $^1H$ NMR ($CDCl_3$, 300MHz) δ 0.66 (3H, s), 0.80 (3H, s), 0.87 (3H, d, J = 6.6Hz), 0.88 (3H, d, J = 6.6Hz), 0.91 (3H, d, J = 6.5Hz), 0.65-1.70 (29H, m), 1.75-1.90 (1H, m), 1.95-2.06 (2H, m), 2.09-2.21 (1H, m), 3.89 (1H, br s), 5.00-5.12 (2H, m) and 5.76-5.91 (1H, m); FDMS: 429 ($M^+$+1); Anal. Calcd for $C_{30}H_{52}O$: C, 84.04; H, 12.22. Found: C, 83.97; H, 12.40.

Example 78

Preparation of (2α, 5α)-2-[(4-fluorophenyl)methyl]cholestan-3-one.

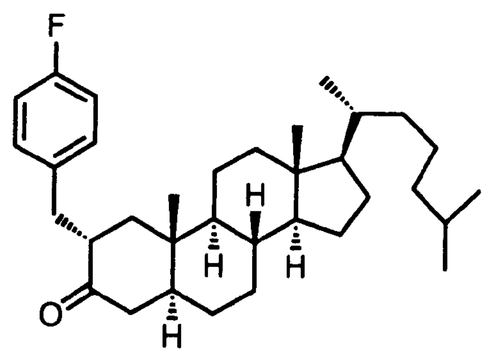

NaN(TMS)$_2$ (1.83 mL, 1M in THF) was added dropwise to a stirred solution of 5α-cholestan-3-one (545 mg, 1.41 mmol) in 10 mL of dry THF at -78C under argon, and the resultant suspension was stirred for 1h before it was treated with a 5 mL of THF solution containing 4-fluorobenzyl iodide (499 mg, 2.11 mmol). The suspension was stirred at -78C for 12h, and then allowed to warm up to ambient temperature. Acetic acid (0.5 mL) and EtOAc (30 mL) were added to the mixture before it was washed with saturated aq. NaCl (10 mL x 2), dried over $MgSO_4$, filtered and concentrated. After flash chromatography on silica (gradient toluene/hexane: 50% to 100%), 424 mg (61%) of the title compound was obtained as a white solid, which was crystallized from $Et_2O/CH_3CN$. mp: 131.0-132.5°C; IR ($CHCl_3$) 2928, 1712, 1509 and 1217 $cm^{-1}$; $^1H$ NMR ($CDCl_3$, 300MHz) δ 0.65 (3H, s), 0.88 (6H, d, J = 6.6Hz), 0.89 (3H, d, J = 7.0Hz), 0.99 (3H, s), 0.64-2.02 (27H, m), 2.10 (1H, dd, J = 14.0 and 3.6Hz), 2.28-2.41 (2H, m), 2.50-2.64 (1H, m), 3.22 (1H, dd, J = 14.0 and 5.0Hz), 6.90-7.00 (2H, m) and 7.07-7.15 (2H, m); FDMS: 495 ($M^+$+1); Anal. Calcd for $C_{34}H_{51}FO$: C, 82.54; H, 10.39. Found: C, 82.80; H, 10.65.

By following the procedures described above in Example 77, the compound of Example 79 was prepared).

Example 79

Preparation of (2α, 3α, 5α)-2-[(4-fluorophenyl)methyl]cholestan-3-ol.

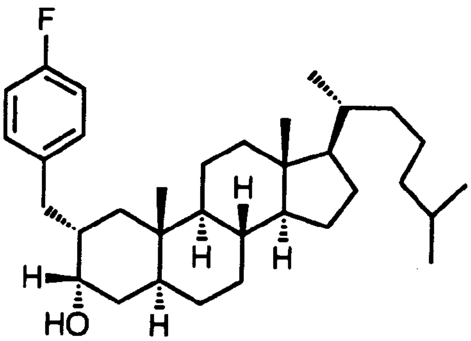

This Example illustrates the preparation of compounds of the invention having pharmaceutically active substituents at the 2 position of the sterol nucleus. (2α, 5α)-2-[(4-Fluorophenyl)methyl]cholestan-3-one (213 mg, 0.431 mmol), K-selectride (647 mL, 1M in THF), 5N NaOH (388 m L, 1.94 mmol) and 30% $H_2O_2$ (198 mL, 1.94 mmol) provided 156 mg (73%) of the title compound as a white solid, which was recrystallized from $Et_2O/CH_3CN$. mp: 148.5-150.5°C; IR (KBr) 3622, 2931, 2869 and 1509 $cm^{-1}$; $^1H$ NMR ($CDCl_3$, 300MHz) δ 0.65 (3H, s), 0.76 (3H, s), 0.88 (6H, d, J = 6.6Hz), 0.91 (3H, d, J = 7.0Hz), 0.90-1.90 (30H, m), 1.97 (1H, br d, J = 11.9Hz), 2.49 (1H, dd, J = 13.5 and 6.6Hz), 2.68 (1H, dd, J = 13.5 and 8.6Hz), 3.71 (1H, s), 6.90-7.02 (2H, m) and 7.10-7.20 (2H, m); FDMS: 497 ($M^+$+1); Anal. Calcd for $C_{34}H_{53}FO$: C, 82.20; H, 10.75. Found: C, 82.33; H, 10.93.

Example 80

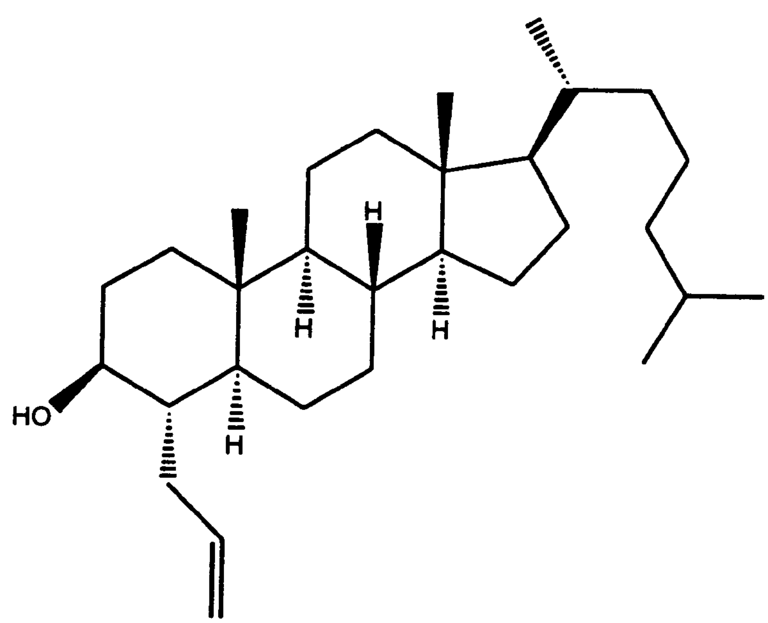

Preparation of (3β,4α,5α,20β)-4-(2-propenyl)cholestan-3-ol

A mixture of 10g (0.0235 mole) of 4α-4-(2-propenyl)cholestan-3-one, 4.4g (0.117mole) ofsodium borohydride, 25ml of methanol, and 50ml of tetrahydrofuran was heated to reflux for 10hr's. The reaction mixture was cooled with an ice bath and quenched with 50% acetic acid(aq). The reaction was concentrated *in vacuo*, and taken up in ethyl acetate,washed with saturated sodium bicarbonate (aq.) solution. The organiclayer was dried with $MgSO_4$, filtered, and concentrated *in vacuo*. The crude isomeric mixture was separated and purified by preparative HPLC (gradient 0-10% ethyl acetate:hexanes). Two major products were formed, (3α,4α,5α)-4-(2-propenyl)cholestan-3-ol and (3β,4α,5α,20β)-4-(2-propenyl)cholestan-3-ol. The fractions containing (3β,4α,5α,20β)-4-(2-propenyl)cholestan-3-ol were combined and 7.2g of (3β,4α,5α,20β)-4-(2-propenyl)cholestan-3-ol was isolated.
M.S. (FD) $MH^+$ = 429

| Elem. Anal. $C_{30}H_{52}O$ | | |
|---|---|---|
| | Calc'd | Found |
| C | 84.04 | 84.20 |
| H | 12.23 | 12.04 |

$^1H$ NMR ($CDCl_3$) d 3.35(m, 1H, H-COH) 4.98-5.15 (m, 2H $H_2C$=CH) 5.79 -5.95 (m, 1H, -HC=$CH_2$)

Example 81

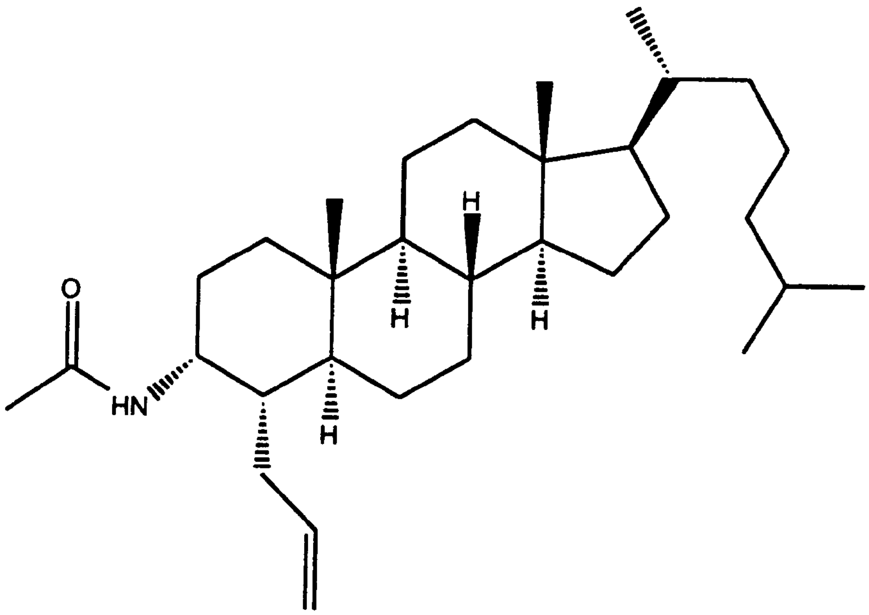

Preparation of N-[(3α,4α,5α,20β)-4-(2-propenyl)cholestan-3-6l]acetamide

A 3.0g (6.79 mmole) mixture of (3α, 4α, 5α, 20β)-4-(2-propenyl) cholestan-3-amine and (3β, 4α,5α,20β)-4-(2-propenyl)cholestan-3-amine was combined with 1.6 ml (17.0 mmole) of acetic anhydride, 3.5ml (33.9 mmole) of pyridine, and 40 ml of toluene. The reaction mixture was heated to refux for 1hr. The reaction mixture was concentrated *in vacuo*, and the residue was purified by preparative HPLC (gradient of 15-50% ethyl acetate:hexanes). The correct acetamide isomer (LY306873) was isolated to yield 1.38 g.
M.S. (FD) $MH^+$ = 470
$^1H$ NMR ($CDCl_3$) δ 2.01(s, 3H, $H_3C$-CON), 4.18(br s, 1H, H-CNH) 4.92-5.01(m, 2H, $H_2C$=CH) 5.65(br s, 1H, HN-CO) 5.70-5.85(m, 1H, HC=$CH_2$)

Example 82

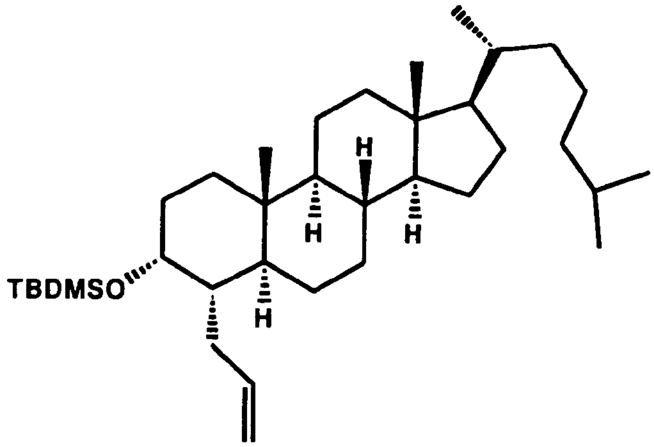

Preparation of (3α, 4α, 5α)-3-[[(1,1-dimethylethyl)dimethylsilyl]oxy]-4-(2-propenyl) cholestane

A mixture of 3.0g of (3α,4α,5α)-4-(2-propenyl)cholestan-3-ol 7.0 mmole), 1.3g *tert*-Butyldimethysilyl chloride (8.5 mmole), and 0.578 g of imidizole (8.5 mmole) in 20 ml of dimethylformamide was stirred for 10 hours at room temperature. The reaction product was then poured into 75 ml of water, and product extracted with diethyl ether, dried over $Na_2S0_4$, filtered, and concentrated *in vacuo* to yield an oil. The oil was then dissolved in 100 ml of hexane and filtered though a silica gel pad and concentrated *in vacuo* to yield 3.34g (88.8%) of (3α, 4α, 5α)-3-[[1,1dimethylethyl)dimethylsilyl]oxy]-4-(2-propenyl) cholestane.
M.S. (FD) $MH^+$ = 543

| Elem. Anal. $C_{36}H_{66}OSi$ | | |
|---|---|---|
| | Calc'd | Found |
| C | 79.90 | 79.63 |
| H | 12.53 | 12.25 |

$^1$H NMR ($CDCl_3$) δ 0.0 (m, 6H, (CH3)2-Si), 0.89 (s, 9H, $(CH_3)_3$C-Si)

Example 83

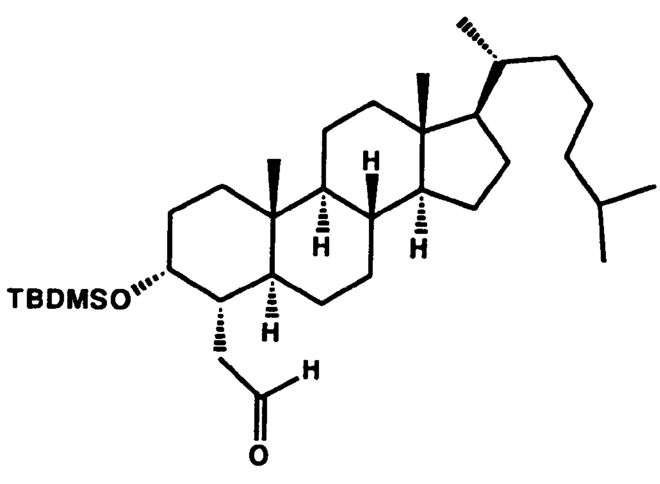

Preparation of (3α, 4α, 5α)-3-[[(1,1-dimethylethyl)dimethylsilyl]oxy]cholestane-4-acetaldehyde

Ozone was bubbled through a solution of 20g of (3α, 4α, 5α)-3-[[(1,1dimethylethyl)dimethylsilyl]oxy]-4-(2-propenyl) cholestane (36.6 mmole) in $CH_2Cl_2$ at -78°C until the reaction became dark purple. The reaction was stirred for 30 minutes at -78°C. $N_2$ was bubbled through the reaction until the color dissipated, and 24 g of triphenyl phosphine (92.2 mmole) was added to the reaction. The mixture was stirred overnight with the cooling bath removed. The eaction was concentrated *in vacuo*, and the crude product was purified by preparative HPLC (5% ethyl acetate: hexanes) yielding 15.5g (77.5%) of (3α, 4α, 5α)-3-[[(1,1-dimethylethyl)dimethylsilyl]oxy]cholestane-4-acetaldehyde.
M.S. (FD) $MH^+$ = 545

| Elem. Anal. | $C_{35}H_{64}O_2Si$ | |
|---|---|---|
| | Calc'd | Found |
| C | 77.14 | 77.34 |
| H | 11.84 | 11.91 |

$^1H$ NMR ($CDCl_3$) δ 9.81 (s, 1H, H-C=O)

Example 84

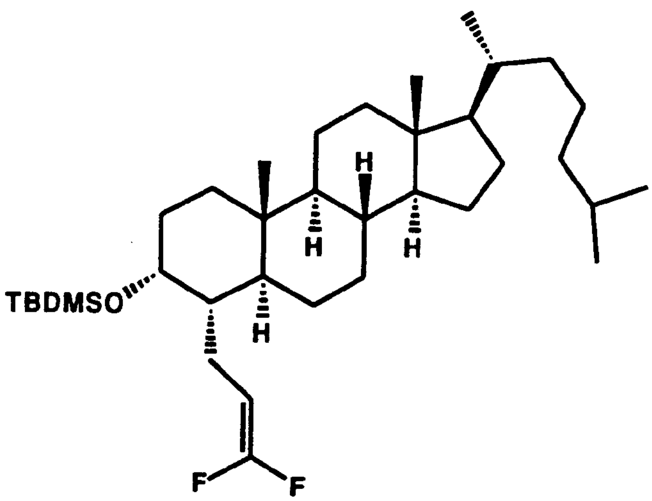

Preparation of (3α, 4α, 5α)-4-(3,3-difluoro-2-propenyl)-3-[[(1,1-dimethylethyl)dimethylsilys]oxy]cholestane

To a chilled solution (-78°C) of 0.70g of difluromethyldiphenyl phosphine oxide (3.50 mmole) in THF, 2.4 ml of a 1.6M solution of butyl lithium in hexanes was added dropwise. The reaction was stirred for 15 minutes. To the reaction, a solution of 1.71 g of (3α, 4α, 5α)-3-[[(1,1-dimethylethyl)dimethylsilyl]oxy]cholestane-4-acetaldehyde (3.15 mmole) in THF was added dropwise and the cooling bath was removed. The reaction was stirred for 3 hr's and quenched with saturated ammonium chloride (aqueous). The product was extracted with diethyl ether and the organic solution was dried over $MgSO_4$, filtered, and concentrated *in vacuo* to yield an oil. The crude product was purified by flash chromatography (4% ethyl acetate:hexanes) to yield 120mg of (3α, 4α, 5α)-4-(3,3-difluoro-2-propenyl)-3-[[(1,1-dimethylethyl)dimethylsilys]oxy]cholestane
M.S. (FD) $MH^+$ 576
$^1H$ NMR (CDCl3) δ 3.93(s, 1H, H-COTBS) 4.08-4.21(m,1H, H-C=CF2)

Example 85

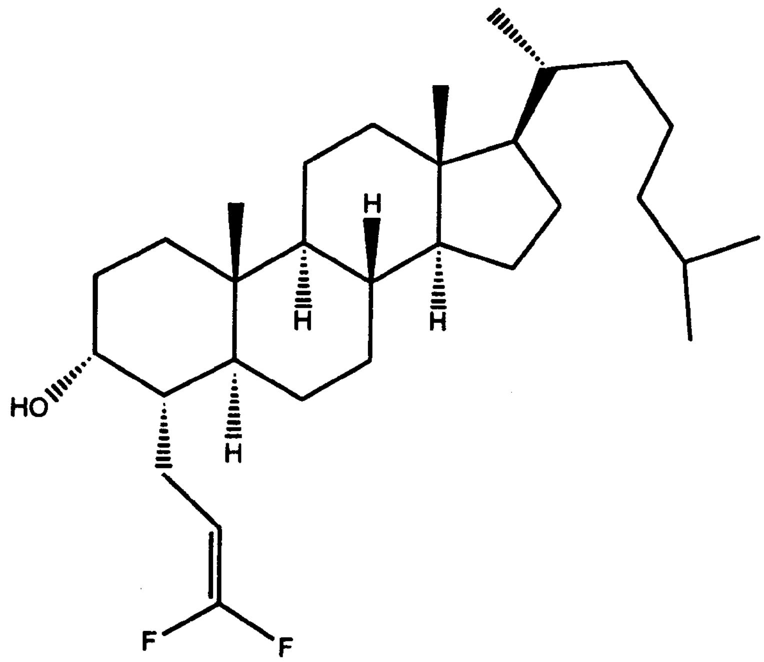

Preparation of (3α, 4α, 5α, 20β)-4-(3,3-difluoro-2-propenyl) cholestan-3-ol

To a solution of 100mg of (3α, 4α, 5α)-4-(3,3-difluoro-2-propenyl)-3-[[(1,1-dimethylethyl)dimethylsilys]oxy]cholestane in 10 ml of $CH_2Cl_2$, 1 ml of boron trifluoride etherate was added. The solution was stirred for 10 hr's at room temperature. The reaction was concentrated *in vacuo*, and residue dissolved in diethyl ether and washed with saturated sodium bicarbonate solution (aqueous). The organic layer was dried over $MgSO_4$, filtered, and concentrated *in vacuo*. The crude product was purified by chromatography (10% ethyl acetate:hexanes) to yield 81 mg of (3α, 4α, 5α, 20β)-4-(3,3-difluoro-2-propenyl) cholestan-3-ol.
M.S. (FD) $MH^+$ = 465

| Elem. Anal. $C_{30}H_{50}F_2O$ | | |
|---|---|---|
| | Calc'd | found |
| C | 77.53 | 77.77 |
| H | 10.85 | 11.07 |

$^1H$ NMR ($CDCl_3$) δ 3.91 (s, 1H, H-COH) 4.12-3.21 (m, 1H, H-C=$CF_2$)

Exmaple 86

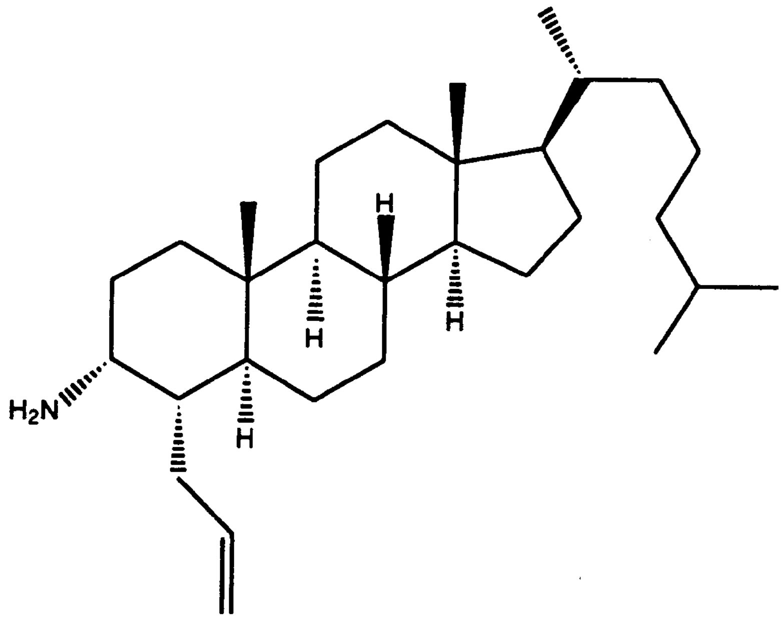

Preparation of (3α,4α,5α,20β)-4-(2-propenyl) cholestan-3-amine.

A mixture of 4.0 g of (4α, 5α)-4-(2-propenyl)cholestan-3-one (9.37 mmole), 7.2 g of ammonium acetate (93.7mmole), 3.3g of sodium cyanoborohydride (52.6 mmole) 3A° molecular sieves, 25 ml of methanol, and 50 ml of THF was stirred for 10 hours at room temperature. The reaction mixture was then poured over fuller's earth and the filtrate was concentrated *in vacuo* , the residue was taken up in 5% NaOH and the product was extracted with diethyl ether. The organic layer was dried over $MgSO_4$, filtered, and concentrated *in vacuo*, the product was purified by chromatography (9:1 Dichloromethane:Methanol) to yield (3α,4α,5α,20β)-4-(2-propenyl) cholestan-3-amine.
M.S. (FD) $MH^+$ 428

| Elem. Anal. $C_{30}H_{53}N$ | | |
|---|---|---|
| | Calc'd | Found |
| C | 84.24 | 84.02 |
| H | 12.49 | 12.48 |
| N | 3.27 | 3.14 |

$^1H$ NMR ($CDCl_3$) δ 3.62 (br s, 1H, H-$CNH_2$)

Example 87

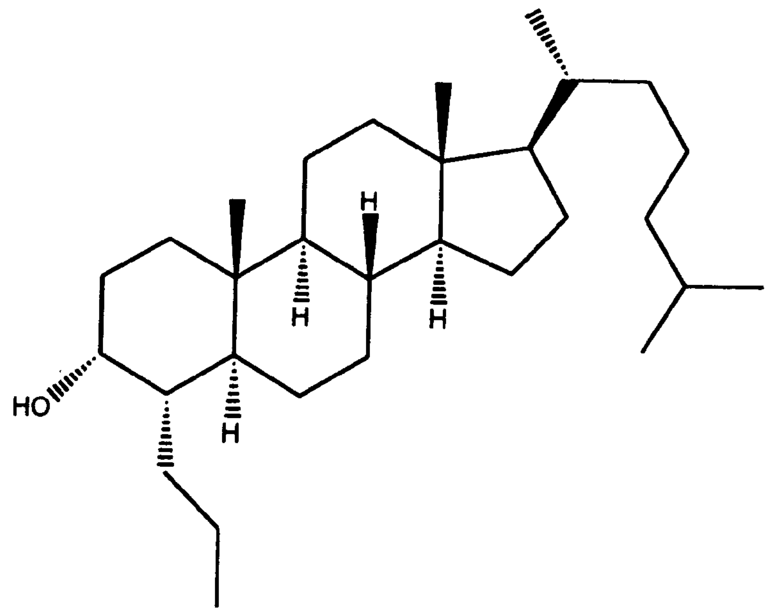

Preparation of (3α, 4α, 5α, 20β)-4-propylcholestan-3-ol

A mixture of 2 g of (3α,4α,5α)-4-(2-propenyl)cholestan-3-ol (4.67mmole) and 0.320 g of 5%Pd/C in 100ml of ethyl acetate was subjected to 60 psi of hydrogen at room temperature for 8 hours. Filtration of the reaction mixture over Fuller's earth followed by evaporation gave 1.54 g of (3α, 4α, 5α, 20β)-4-propylcholestan-3-ol. M.S. (FD) $MH^+$ = 430

| Elem. Anal. $C_{30}H_{54}O$ | | |
|---|---|---|
| | Calc'd | found |
| C | 83.65 | 83.80 |
| H | 12.64 | 12.62 |

$^1H$ NMR ($CDCL_3$) δ 3.92(s, 1H, H-COH)

Example 88

Preparation of (4α,5α)-4-(2-methyl-2-propenyl)cholestan-3-one.

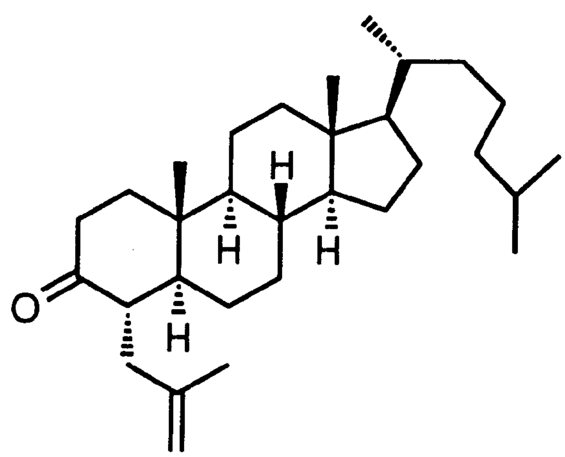

This example illustrates the preparation of a 3- position ketone useful as an intermediate for preparing the compounds of the invention.

Lithium chip (32.5 mg, 4.68 mmol) and a glass-coated stir bar were placed in a flame-dried, three-necked, round-bottomed flask fitted with a dry ice condenser under argon. Forty milliliters of liquid ammonia was collected in the flask at -78°C to form a deep blue solution, then followed by the addition of dry THF (25 ml). A fifteen-milliliter solution of (+)-4-cholesten-3-one (600 mg, 1.56 mmol) and t-BuOH (0.147 mL, 1.56 mmol) in dry THF was added dropwise to the deep blue solution. Upon completion of the addition, the resultant blue solution was stirred for 5 min before it was decolorized with a few drops of 1,3-pentadiene. Methallyl iodide (0.480 mL, 4.67 mmol) was added to the white suspension and the resultant mixture was stirred at -78°C for 3h. Methanol (1 mL) and saturated aq. $NH_4Cl$ (10 mL) were carefully added to the white suspension. The cold bath was removed and the mixture, with the evaporation of ammonia, was allowed to warm up to ambient temperature. Ten milliliters of sat'd aq. NaCl was added to the mixture before it was extracted with EtOAc (30 mL x 2); the combined organic layers were washed with sat'd aq. NaCl (10 mL), dried over $MgSO_4$, filtered and concentrated. After flash chromatographic separation on silica (gradient ethyl acetate/hexane 4% to 6%), 212 mg (31%) of the title compound was obtained as a white solid, which was recrystallized from $Et_2O/CH_3CN$. mp: 104.0-105.5°C; IR (KBr) 2950 and 1712 $cm^{-1}$; $^1H$ NMR ($CDCl_3$, 300MHz) δ 0.69 (3H, s), 0.88 (6H, d, J = 6.6Hz), 0.92 (3H, d, J = 6.5Hz), 1.08 (3H, s), 1.71 (3H, s), 0.65-1.90 (24H, m), 1.95-2.20 (4H, m), 2.30-2.60 (4H, m), 4.61 (1H, s) and 4.73 (1H, s); FDMS: 440 ($M^+$); Anal. Calcd for $C_{31}H_{52}O$: C, 84.48; H, 11.89. Found: C, 84.43; H, 11.68.

Example 89

Preparation of (3α,4α,5α)-4-(2-methyl-2-propenyl)cholestan-3-ol.

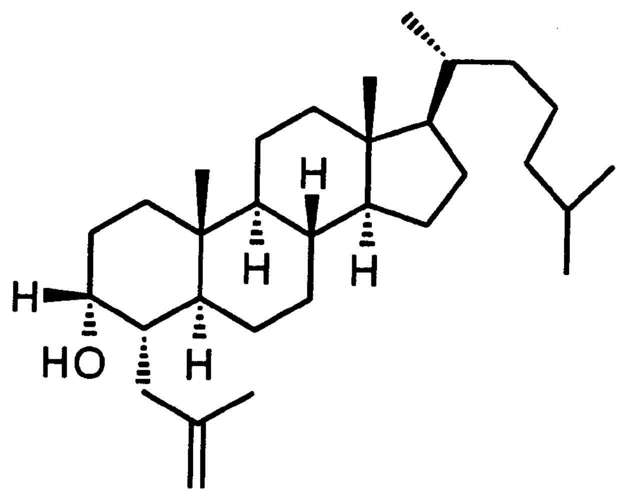

K-Selectride (0.368 mL, 1M in THF) was added to a stirred solution of (4a)-4-(2-methyl-2-propenyl)cholestan-3-one (108 mg, 0.245 mmol) in dry THF (4 mL) at -78°C under argon, and the resultant yellowish solution was then stirred at 0°C for 1h. Excess K-selectride was carefully quenched with methanol (0.5 mL) and the solution was sequentially treated with 5N NaOH (0.220 mL, 1.10 mmol) and 30% $H_2O_2$ (0.113 mL, 1.10 mmol). The cold bath was removed and the mixture was stirred for 2h. Half-sat'd aq. NaCl (10 mL) and EtOAc (30 mL) were added to the mixture, the organic layer was separated, washed with half-sat'd aq. NaCl (10 mL x 2), dried over $MgSO_4$, filtered and concentrated. The residue was subject to flash chromatography on silica (gradient ethyl acetate/hexane 4% to 8%) to provide 85.5 mg (79%) of the title compound, which was recrystallized from $Et_2O/CH_3CN$. mp: 129.0-130.5°C; IR (KBr) 3581, 3472 and 2937 $cm^{-1}$; $^1H$ NMR ($CDCl_3$, 300MHz) δ 0.66 (3H, s), 0.84 (3H, s), 0.88 (6H, d, J = 6.6Hz), 0.91 (3H, d, J = 6.5Hz), 1.73 (3H, s), 0.65-2.05 (32H, m), 2.20 (1H, dd, J = 13.3 and 4.3Hz), 3.79 (1H, br s), 4.78 (1H, s) and 4.81 (1H, s); FDMS: 443 ($M^+$+1); Anal. Calcd for $C_{31}H_{54}O$: C, 84.09; H, 12.29. Found: C, 84.35; H, 12.06.

By following the procedures described above in Example 88, the compound of Example 90 was prepared.

Example 90

Preparation of (4α,5α)-4-(2-chloro-2-propenyl)cholestan-3-one.

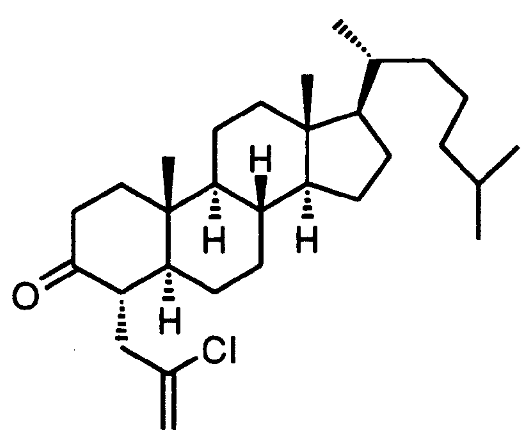

This example illustrates the preparation of a 3-position ketone useful as an intermediate for preparing the compounds of the invention.

Lithium chip (32.5 mg, 4.68 mmol), liquid $NH_3$ (30 mL)/THF (30 mL), (+)-4-cholesten-3-one (600 mg, 1.56

mmol), t-BuOH (0.147 mL, 1.56 mmol), 1,3-pentadiene (a few drops) and 2-chloroallyl iodide (0.500 mL) provided 377 mg (52%) of the title compound as a white solid after stirring at -78°C for 6.5h and flash chromatographic separation on silica (gradient ethyl acetate/hexane 4% to 6%), which was recrystallized from $Et_2O/CH_3CN$. mp: 107.0-109.0°C; IR (KBr) 2945, 1714, 1639 and 1467 $cm^{-1}$; $^1H$ NMR ($CDCl_3$, 300MHz) δ 0.69 (3H, s), 0.88 (6H, d, J = 6.6Hz), 0.92 (3H, d, J = 6.5Hz), 1.11 (3H, s), 0.70-1.65 (22H, m), 1.65-1.90 (3H, m), 1.95-2.12 (2H, m), 2.32-2.68 (4H, m), 2.92 (1H, dd, J = 15.0 and 7.0Hz), 5.19 (1H, s) and 5.22 (1H, s); FDMS: 460 ($M^+$, $^{35}Cl$) and 462 ($M^+$, $^{37}Cl$); Anal. Calcd for $C_{30}H_{49}ClO$: C, 78.13; H, 10.71. Found: C, 77.99; H, 10.42.

By following the procedures described above in Example 89, the compound of Example 91 was prepared.

Example 91

Preparation of (3α,4α,5α)-4-(2-chloro-2-propenyl)cholestan-3-ol.

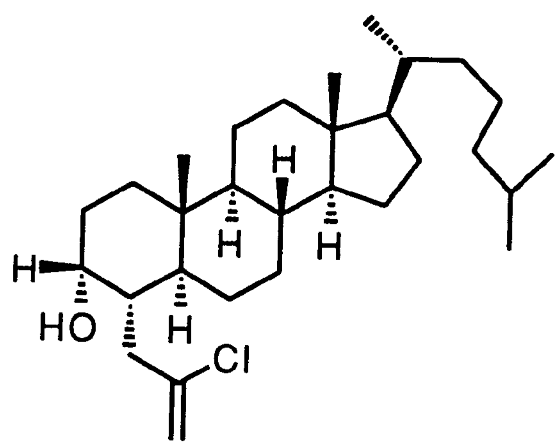

K-Selectride (0.586 mL, 1M in THF), (4α)-4-(2-chloro-2-propenyl)cholestan-3-one (180 mg, 0.390 mmol), THF (5 mL), methanol (0.15 mL), 5N NaOH (0.352 mL, 1.76 mmol) and 30% $H_2O_2$ (0.179 mL, 1.76 mmol) provided 124 mg (69%) of the title compound as a white solid after flash chromatographic separation on silica (gradient toluene/hexane 60% to 100%), which was recrystallized from $Et_2O/CH_3CN$. mp: 130.0-131.5°C; IR (KBr) 3599, 3497 and 2933 $cm^{-1}$; $^1H$ NMR ($CDCl_3$, 300MHz) δ 0.67 (3H, s), 0.86 (3H, s), 0.87 (3H, d, J = 6.7Hz), 0.88 (3H, d, J = 6.6Hz), 0.92 (3H, d, J = 6.5Hz), 0.70-1.90 (30H, m), 1.98 (1H, br d, J = 12.2Hz), 2.33 (1H, dd, J = 13.9 and 10.6Hz), 2.47 (1H, dd, J = 13.9 and 4.7Hz), 3.91-3.93 (1H, m) and 5.23 (2H, s); FDMS: 462 ($M^+$, $^{35}Cl$) and 464 ($M^+$, $^{37}Cl$).

By following the procedures described above in Example 88, the compound of Example 92 was prepared.

Example 92

Preparation of (4α,5α)-4-(2-bromo-2-propenyl)cholestan-3-one.

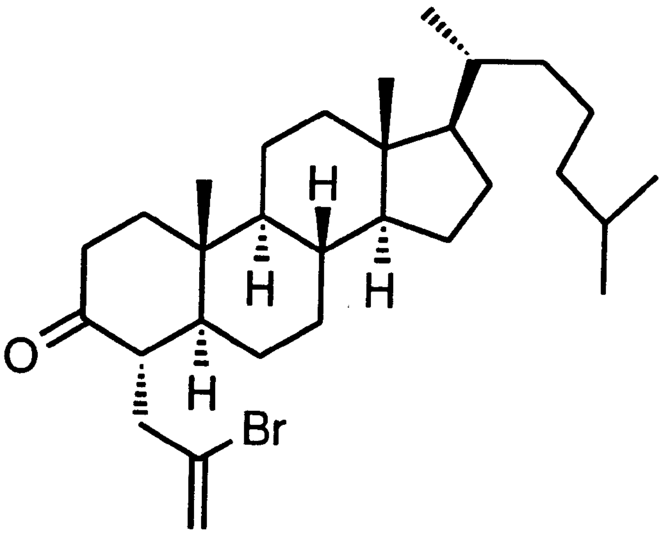

This example illustrates the preparation of a 3- position ketone useful as an intermediate for preparing the compounds of the invention.

Lithium chip (32.5 mg, 4.68 mmol), liquid $NH_3$ (35 mL)/THF (35 mL), (+)-4-cholesten-3-one (600 mg, 1.56 mmol), t-BuOH (0.147 mL, 1.56 mmol), 1,3-pentadiene (a few drops) and 2-bromoallyl bromide (0.484 mL, 4.68 mmol) provided 70.0 mg (8.8%) of the title compound as a white solid after stirring at -78°C for 6h and flash chromatographic separation on silica (gradient ethyl acetate/hexane 4% to 6%). $^1$H NMR ($CDCl_3$, 300MHz) δ 0.69 (3H, s), 0.87 (3H, d, J = 6.6Hz), 0.88 (3H, d, J = 6.6Hz), 0.91 (3H, d, J = 6.5Hz), 1.12 (3H, s), 0.65-1.90 (25H, m), 1.95-2.10 (2H, m), 2.30-2.70 (4H, m), 3.01 (1H, dd, J = 15.0 and 7.0Hz), 5.43 (1H, s) and 5.66 (1H, s).

By following the procedures described above in Example 89, the compound of Example 93 was prepared.

Example 93

Preparation of (3α,4α,5α)-4-(2-bromo-2-propenyl)cholestan-3-ol.

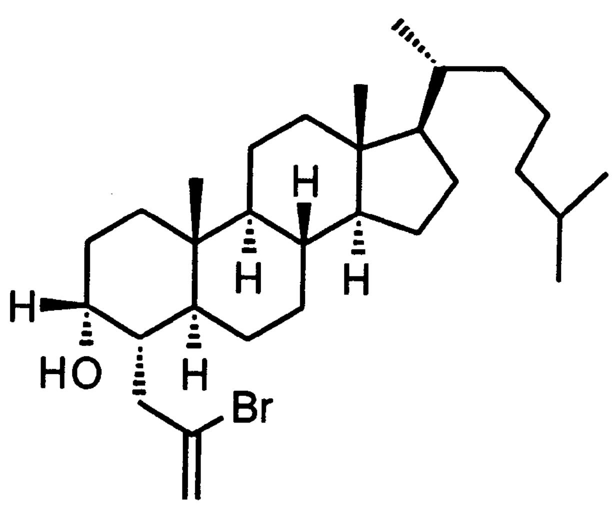

K-Selectride (0.278 mL, 1M in THF), (4a)-4-(2-bromo-2-propenyl)cholestan-3-one (70.0 mg, 0.139 mmol), THF (3 mL), methanol (0.15 mL), 5N NaOH (0.167 mL, 0.834 mmol) and 30% $H_2O_2$ (0.085 mL, 0.834 mmol)

provided 47.0 mg (67%) of the title compound as a white solid after flash chromatographic separation on silica (gradient toluene/hexane 60% to 100%). mp: 144.0-147.0°C; IR (KBr) 3601, 3478 and 2931 $cm^{-1}$; $^1H$ NMR ($CDCl_3$, 300MHz) δ 0.67 (3H, s), 0.87 (3H, s), 0.88 (6H, d, J = 6.8Hz), 0.92 (3H, d, J = 6.5Hz), 0.70-1.90 (30H, m), 1.99 (1H, br d, J = 12.3Hz), 2.40 (1H, dd, J = 14.0 and 10.5Hz), 2.55 (1H, dd, J = 14.0 and 4.5Hz), 3.91-3.94 (1H, m), 5.48 (1H, s) and 5.68 (1H, s); FDMS: 506 ($M^+$, $^{79}Br$) and 508 ($M^+$, $^{81}Br$).

Example 94

Preparation of (14α)-stigmast-4-en-3-one.

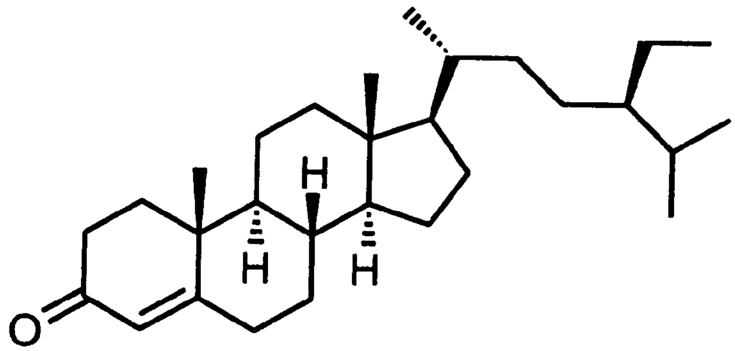

This example illustrates the preparation of a 3- position ketone useful as an intermediate for preparing the compounds of the invention.

A suspension of β-sitosterol (30.0 g, 72.5 mmol), aluminum isopropoxide (7.40 g, 36.2 mmol) and methyl ethyl ketone (97.0 mL, 1.09 mol) in dry toluene (180 mL) was heated to reflux with stirring for 21 hours under argon. The mixture was cooled in an ice bath, treated with 2.5N HCl (120 mL) and stirred for 30 min. The organic layer was separated, washed with sat'd aq. NaCl (100 mL), dried over $MgSO_4$, filtered and concentrated; the oily residue was chromatographed on silica (gradient 50% toluene/hexane to 10% ethyl acetate/toluene) to provide 23.9 g (80%) of the title compound, which was crystallized from $CH_2Cl_2/CH_3CN$. mp: 73.0-75.0°C; $^1H$ NMR ($CDCl_3$, 300MHz) δ 0.73 (3H, s), 0.83 (3H, d, J = 6.7Hz), 0.85 (3H, d, J = 6.6Hz), 0.86 (3H, t, J = 6.8Hz), 0.93 (3H, d, J = 6.5Hz), 1.20 (3H, s), 0.65-1.78 (21H, m), 1.80-1.95 (2H, m), 1.98-2.10 (2H, m), 2.24-2.53 (4H, m) and 5.75 (1H, s)

By following the procedures described above in Example 88, the compound of Example 95 was prepared.

Example 95

Preparation of (4α,5α,14α)-4-(2-propenyl)stigmastan-3-one.

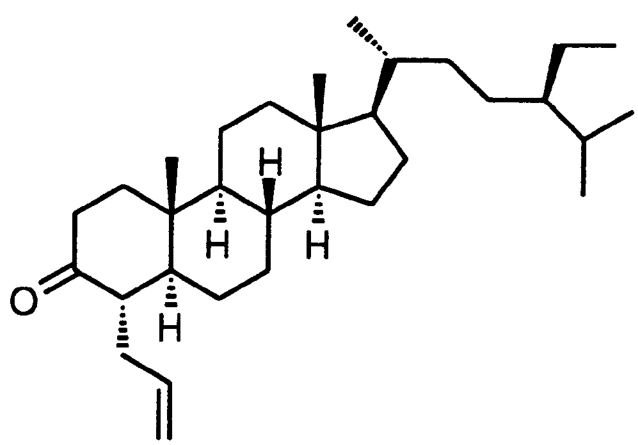

This example illustrates the preparation of a 3- position ketone useful as an intermediate for preparing the compounds of the invention.

Lithium chip (135 mg, 19.4 mmol), liquid $NH_3$ (100 mL)/THF (150 mL), (24R)-24-(ethyl)cholest-4-en-3-one

(2.00 g, 4.85 mmol), t-BuOH (0.366 mL, 3.88 mmol), isoprene (2 mL) and allyl iodide (0.886 mL, 9.70 mmol) provided 235 mg (11%) of the title compound as a white solid after stirring at -78°C for 3h and flash chromatographic separation on silica (gradient 70% toluene/hexane to 5% ethyl acetate/toluene), which was recrystallized from $CH_2Cl_2/CH_3CN$ mp: 81.0-82.0°C; $^1H$ NMR ($CDCl_3$, 300MHz) $\delta$ 0.69 (3H, s), 0.83 (3H, d, J = 6.7Hz), 0.85 (3H, d, J = 6.4Hz), 0.86 (3H, t, J = 6.7Hz), 0.92 (3H, d, J = 6.4Hz), 1.07 (3H, s), 0.65-1.94 (26H, m), 1.95-2.10 (2H, m), 2.20-2.55 (5H, m), 4.93-5.08 (2H, m) and 5.70-5.88 (1H, m).

By following the procedures described above in Example 89, the compound of Example 96 was prepared.

Example 96

Preparation of (3α, 4α, 5α)-4-(2-propenyl)-stigmastan-3-ol.

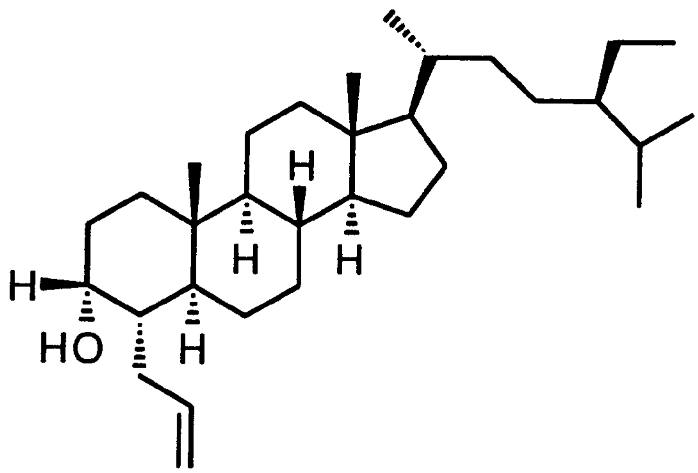

K-Selectride (0.440 mL, 1M in THF), (4α, 24R)-4-(2-propenyl)-24-(ethyl)cholestan-3-one (100 mg, 0.220 mmol), THF (3 mL), methanol (0.20 mL), 5N NaOH (0.264 mL, 1.32 mmol) and 30% $H_2O_2$ (0.135 mL, 1.32 mmol) provided 95.0 mg (95%) of the title compound as a white solid after flash chromatographic separation on silica (gradient 70% toluene/hexane to 5% ethyl acetate/toluene). mp: 118.0-120.5°C; $^1H$ NMR ($CDCl_3$, 300MHz) $\delta$ 0.66 (3H, s), 0.83 (3H, d, J = 6.5Hz), 0.84 (3H, s), 0.85 (3H, d, J = 6.4Hz), 0.86 (3H, t, J = 6.9Hz), 0.92 (3H, d, J = 6.4Hz), 0.65-2.07 (33H, m), 2.24-2.35 (1H, m), 3.91-3.93 (1H, m), 5.00-5.15 (2H, m) and 5.80-5.98 (1H, m).

By following the procedures described above in Example 88, the compound of Example 97 was prepared.

Example 97

Preparation of (4α,5α,14α,22E)-4-(2-propenyl)-stigmast-22-en-3-one.

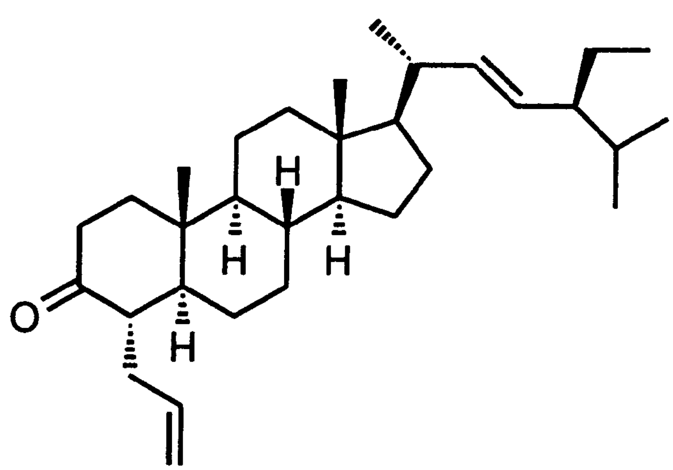

This example illustrates the preparation of a 3-position ketone useful as an intermediate for preparing the compounds of the invention.

Lithium chip (67.0 mg, 9.76 mmol), liquid $NH_3$ (60 mL)/THF (100 mL), (22E, 24R)-24-(ethyl)cholesta-4,22-dien-3-one (1.00 g, 2.44 mmol), t-BuOH (0.230 mL, 2.44 mmol), 1,3-pentadiene (2 mL) and allyl iodide (0.670 mL, 7.32 mmol) provided 526 mg (48%) of the title compound as a white solid after stirring at -78°C for 6h and flash chromatographic separation on silica (gradient toluene/hexane 60% to 100%), which was recrystallized from $CH_2Cl_2/CH_3CN$

. mp: 109.0-111.0°C; $^1$H NMR ($CDCl_3$, 300MHz) δ 0.71 (3H, s), 0.81 (3H, d, J = 6.5Hz), 0.82 (3H, t, J = 6.5Hz), 0.86 (3H, d, J = 6.3Hz), 1.02 (3H, d, J = 6.6Hz), 1.07 (3H, s), 0.65-1.60 (18H, m), 1.62-1.80 (3H, m), 1.94-2.09 (3H, m), 2.20-2.54 (5H, m), 4.95-5.08 (3H, m), 5.16 (1H, dd, J = 15.1 and 8.5Hz) and 5.72-5.86 (1H, m).

By following the procedures described above in Example 89, the compound of Example 98 was prepared.

Example 98

Preparation of (3α, 4α, 5α, 22E)-4-(2-propenyl)-stigmast-22-en-3-ol.

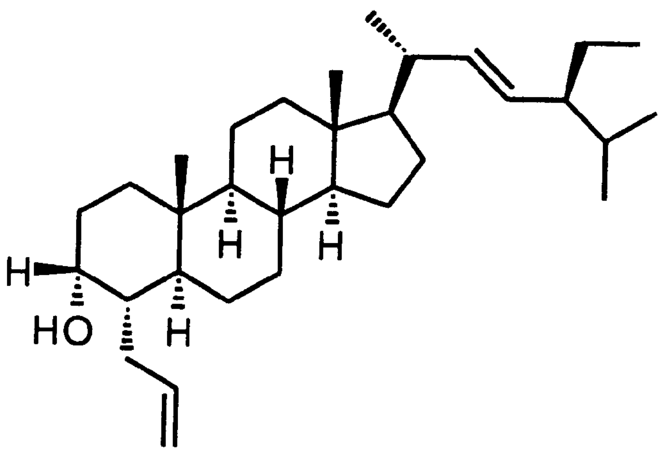

K-Selectride (0.880 mL, 1M in THF), (4α, 22E, 24R)-4-(2-propenyl)-24-(ethyl)cholest-22-en-3-one (200 mg, 0.440 mmol), THF (5 mL), methanol (0.20 mL), 5N NaOH (0.572 mL, 2.86 mmol) and 30% $H_2O_2$ (0.292 mL, 2.86 mmol) provided 186 mg (93%) of the title compound as a white solid after flash chromatographic separation on silica (gradient 60% toluene/hexane to 5% ethyl acetate/ toluene). mp: 141.0-142.0°C; $^1$H NMR ($CDCl_3$, 300MHz) δ 0.68 (3H, s), 0.81 (3H, d, J = 6.3Hz), 0.82 (3H, t, J = 6.7Hz), 0.83 (3H, s), 0.86 (3H, d, J = 6.5Hz), 1.02 (3H, d, J = 6.5Hz), 0.65-1.78 (26H, m), 1.93-2.10 (3H, m), 2.23-2.34 (1H, m), 3.90-3.93 (1H, m), 4.97-5.21 (4H, m) and 5.82-5.96 (1H, m).

Example 99

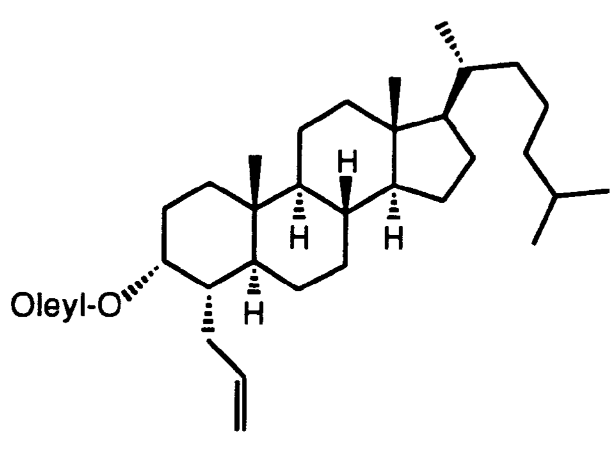

Preparation of (3α,4α,5α)-4-(2-propenyl)cholestan-3-ol octadecanoate (ester):

An acid chloride of Oleic acid was prepared by a mixture of 4.0g of oleic acid (14.0 mmoles), 1.8g of oxayl chloride (14.0 mmoles), 1.1g of pyridine (14.0 mmoles), a catalytic amount of DMF, and 50ml 0f $CH_2Cl_2$, which was stirred at room temperature for 1 hr. The reaction was concentrated *in vacuo* and 50ml of $CH_2Cl_2$ was added to the crude acid chloride. The reaction mixture was chilled to 0°C and a solution of 3.0g [4α,5α]-4-(2-propenyl)cholestan-3α-ol (7.0 mmoles) in 25ml of $CH_2Cl_2$ was added dropwise. The reaction stirred overnight with the ice bath removed. The reaction was concentrated in vacuo and the crude product was purified by preparative H.P.L.C. (gradient 0-5%, ethyl Acetate: hexanes) yielding 2.25g (46%) of an oil.
M.S. (FD) $MH^+$=693

| Elem. Anal. $C_{48}H_{86}O_2$ | | |
|---|---|---|
| | Calc'd | Found |
| C | 82.93 | 82.91 |
| H | 12.47 | 12.55 |

$^1H$ NMR ($CDCl_3$) d 4.88 (s, 1H, HC-$OCOCH_2$), 4.98 (m, 2H, $H_2$=CH), 5.36 (m, 2H, -HC=CH-), 5.62-5.79 (m, 1H, HC=$CH_2$)

Example 100

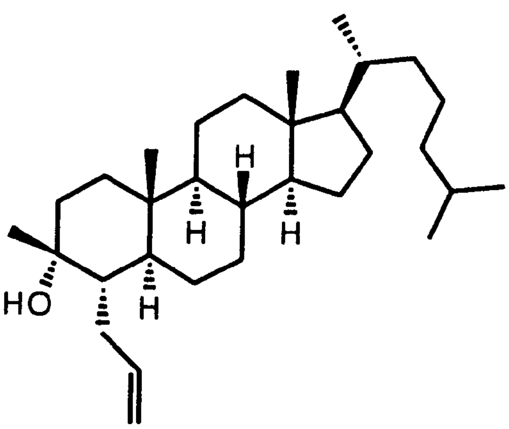

Preparation of (3β,4α,5α)-3-methyl-4-(2-propenyl)cholestan-3-ol:

A solution of 2.0 g of 4α-4-(2-propenyl)cholestan-3-one (4.7mmoles) in 10ml of diethyl ether was chilled to -78°C. To the solution, a 2.0M solution of methyl magnesium bromide in diethyl ether was added slowly. The reaction was stirred overnight slowly coming to room temperature. A saturated ammonium chloride solution (aq.) was added and the organic layer was isolated and dried over $MgSO_4$, and concentrated *in vacuo*. The crude product was purified via flash chromatography (8% ethyl acetate: hexanes) yielding 620 mg of product.
M.S. (FD) $MH^+$=442

| Elem. Anal. $C_{31}H_{54}O$ | | |
|---|---|---|
| | calc'd | found |
| C | 84.09 | 84.13 |
| H | 12.29 | 12.39 |

$^1H$ NMR (CDCl3) d 4.98-5.14(m, 2H, $H_2C$=CH), 5.91-6.08 (m, 1H, HC=$CH_2$)

EXAMPLE T-1

The following experiments were carried out to demonstrate the ability of the compounds of the present invention to upregulate LDL receptor synthesis.

Method

A 1546 base pair sequence of the human LDL receptor promoter was amplified using the polymerase chain reaction. A reaction mixture containing 20 pMoles each of the synthetic oligonucleotides 5'-GCGCCATATGAGTCTTAACTGCCAAAAATTCTTATCATCAAT-3' (Seq. I.D. No: 1) and 5'-AAG-CAAGCTTTCGCAGCCTCTGCCAGGCAGTGTCCCGACCCGGA-3', (Seq. I.D. No:2) 1 μg human genomic DNA purified from the adenocarcinoma cell line P3UCLA, 200 μM each of dATP, dGTP, dCTP, and TTP, 2.5 units of Taq DNA polymerase, 10mM Tris-HCl pH 8.3, 50 mM KCl, 15 mM MgC12, 0.1% gelatin in a final volume of 100 μl was subjected to 30 cycles of 15 sec at 96°C, 30 sec at 55°C, and 1 min at 72°C. The material was subject to gel electrophoresis on a 1% agarose gel and the 1546 base pair band was isolated and restriction enzyme digested with Hind III and Nde I. This fragment was ligated into the plasmid pSP72 (ProMega Biotech), which had previously been restriction enzyme nuclease digested with Hind III and Nde I. The resulting vector, pNLDLRP, was restriction endonulease digested with NdeI and Hind III and the material was again run on a 1% agarose gel to reisolate the 1546 bp LDL receptor sequence.

Plasmid vector PSv2 was constructed by digesting plasmid pSv2-globin with Hind III and Bgl II then ligating an NruI-XhoI linker into the vector. Plasmid pSv2 globin is disclosed in U.S. Patent No. 4,775,624, the entire teaching of which is herein incorporated by reference. The linker contained the following sequences:

5'-AGCTTCGCGACTCGAGA-3' (Seq. I.D. No: 3), and
5'GATCTCTCGAGTCGCGA-3' (Seq. I.D. No:4).

The resulting vector was designated pSv2-H NXB because it contained a BamH I site, an NruI site, an XhoI site and a Bgl II site. The Hind III-Bgl II fragment of plasmid pAlc4(NRRL B-18783), which contains the firefly luciferase gene, was then ligated into the Hin III-Bgl II site of plasmid pSv2-HNXB. A neomycin resistance-conforming gene was then ligated into the Bam HI site of the resultant plasmid to form plasmid pSv2.

A 1546 base pair fragment was isolated and cloned into a NdeI and Hind III (partial) restriction digested vector pSv2 containing the firefly luciferase reporter gene and a gene for neomycin resistance. The resulting vector, pLDL-Luc1Neo-10, contains the human LDL receptor promoter driving expression of the firefly luciferase gene, a gene coding for neomycin resistance, ampicillin resistant marker and an origin of replication.

Plasmid pLDL-Luc 1 Neo-10 was used to stably transfect Chinese Hamster Ovary cells using a Lipofection Reagent kit and procedure from Gibco BRL Research Products Division Life Technologies Inc. Transformants were selected by picking individual colonies grown in Dulbecco's modified Eagle's medium supplimented with 10% fetal bovine serum and containing 500 μg per ml 25 hydroxycholesterol. A cell colony (S27/B30) was selected that showed the highest luciferase production and at least 50% repression in the presence of Geneticin (G-418). Clone S27/B30 was used for subsequent screening.

In developing a cell based screen it is important to have a negative control. The viral SV40 promoter is ideal for this purpose in that it has a number of SP1 elements that enhance gene transcription. These elements are not under the control of end product repression; but they do have a high degree of sequence homology with the 16 base pair long sterol response element found in the LDL receptor promoter. The SV40 promoter should therefore be a sensitive mechanism to screen out agents that induce nonspecific transcription.

Chinese Hamster Ovary cells were stably cotransfected with a vector pSV2AL-A 5' containing the SV40 promoter driving expression of the firefly luciferase gene and a second vector pSV2 NeoBam containing a neomycin resistant marker. Transfections were carried out using the Lipofectin Reagent kit and procedure from Gibco BRL Research Products Division Life Technologies Inc. Transformants were selected by picking individual colonies grown in Dulbecco's modified Eagle's medium supplemented with 10% fetal bovine serum and containing 500 μg per ml of geneticin. Individual clones were selected, lysed and assayed for luciferase production. Clone SV40 LN 9 was found to have the highest luciferase expression and was used as a negative control for the LDL receptor screen.

Assay

A. Media

Both the "growth medium" and the "assay medium" are prepared from the same basal medium which consists of three parts Dulbecco's modified Eagle's medium and one part (by volume) of Ham's F-12 medium with the addition of $10^{-8}$M selenium, 50 μM ethanolamine, and 20 mM hydroxyethylpiperazine ethanesulfonic acid. Growth medium is basal medium supplemented with 5% v/v fetal bovine serum. Assay medium is basal medium supplemented with 0.5% Bovuminar Cohn fraction V powder (bovine albumin) and 0.5 μg/ml 25-hydroxycholesterol.

B. Cell Culture

The recombinant CHO cell lines (clone 8, clone 527B30 or clone SV40-LN9) are seeded at 5 x $10^4$ cells/well into 24-well plates in 0.5 ml growth medium and incubated at 37°C in a humidified air atmosphere containing 5% $CO_2$. At confluence (2-3 days after seeding), the growth medium is removed, the monolayers rinsed 1X with media (0.5 ml/well) and either vehicle or candidate test compound is added to triplicate wells. The plates are incubated for an additional 24 hr and then assayed for luciferase activity.

C. Luciferase Activity

Each well is washed 1X in phosphate buffered saline without $Ca^{+2}$ or $Mg^{+2}$ (500 μl/well), and the cell monolayer is lysed by addition of 100 μl/well assay buffer containing 1% Triton X-100, 25 mM glycylglycine (pH 7.8), 15 mM $MgSO_4$, 1 mM dithiothreitol, and 1 mM ATP. A 50 μl aliquot of each lysate is diluted to 425 μl in the above assay buffer and placed in LKB luminometer and the reaction is initiated by the injection of 25 μl 1mM luciferin. Light output from the luciferase reaction is expressed as peak luminescence and is proportional to luciferase concentration. Total protein is determined in each lysate (~5 μl) by Coomassie brilliant blue G250 binding with protein assay kit (Bio Rad Laboratories)

Luciferase Activity is expressed as light units/ug of protein (specific activity). A minimum effective dose is defined as the molar concentration of agent required to increase the luciferase specific activity by 30% relative to control. Relative effective dose is the product of the luciferase specific activity of a compound divided by the specific activity of compound [4α(E),5α]-4-(2-butenyl)cholestan-3α-ol. A number less than one represents a compound more active than [4α(E),5α]-4-(2-butenyl)cholestan-3α-ol in de-repressing the LDL receptor promoter transcription.

The results of evaluating compounds of the present invention are set forth below in Table I.

Table I

(in vitro Luciferase Activity Test)

| Example No. | MED | RED | MA% |
|---|---|---|---|
| 3[1] | 2.2 | 0.97 | 144 |
| 4[1] | 2.2 | 1.00 | 80 |
| 5[2] | 5.8 | 1.02 | 121 |
| 5[2] | 2.3 | 0.20 | 120 |
| 5[2] | 2.3 | 1.00 | 135 |
| 6[2] | 21.7 | 0.96 | 72 |
| 6[2] | 21.7 | 1.90 | 67 |
| 7[2] | 11.2 | 1.97 | 46 |
| 7[2] | 45 | 45 | 30 |
| 8[1] | 5.6 | 2.50 | 51 |
| 9[1] | 2.0 | 0.88 | 60 |
| 10[1] | 10.3 | 4.5 | 55 |
| 11[1] | | | NA |
| 12[1] | | | NA |
| 13[1] | | | NA |
| 14[1] | | | NA |
| 15[1] | | | NA |
| 16[1] | | | NA |
| 17[1] | | | NA |
| 18[1] | | | NA |
| 19[1] | | | NA |
| 20[1] | | | NA |
| 21[1] | | | NA |
| 22[1] | | | 18% (NA) |
| 23[2] | 5.02 | 0.44 | 49 |
| 23[2] | 10.12 | 1.8 | 37 |

| Example No. | MED | RED | MA% |
|---|---|---|---|
| 24[2] | 8.9 | 1.57 | 37 |
| 24[2] | 8.9 | 0.78 | 48 |
| 24[2] | 1.8 | 0.32 | 87 |
| 25[2] | 4.1 | 0.72 | 89 |
| 25[2] | 4.1 | 0.36 | 64 |
| 25[2] | 8.3 | 1.46 | 79 |
| 26[2] | 9.1 | 0.79 | 59 |
| 26[2] | 9.1 | 1.6 | 34 |
| 27[2] | 9.1 | 4.0 | 32 |
| 29[2] | 14 | 2.5 | 37 |
| 30[2] | 8.9 | 3.2 | 76 |
| 30[1] | 17.3 | 3.2 | 65 |
| 30[1] | 8.9 | 0.8 | 92 |
| 31[2] | 9.7 | 4.3 | 46 |
| 32[2] | 8.6 | 3.8 | 36 |
| 33[2] | 9.8 | 4.3 | 28 |
| 34[2] | 4.8 | 2.1 | 38 |
| 35[1] | | | NA |
| 36[2] | 40 | 17.6 | 20 |
| 36[1] | | | NA |
| 37[1] | | | NA |
| 38[2] | 84 | 7.37 | 33 |
| 38[2] | 42 | 3.7 | 58 |
| 39[1] | | | NA |
| 40[2] | 2.3 | 1.0 | 99 |
| 40[1] | 2.9 | 0.51 | 110 |
| 40[1] | 5.9 | 0.52 | 71 |
| 41[1] | | | NA |
| 42[1] | | | NA |
| 43[1] | | | NA |
| 44[2] | 5.8 | 2.5 | 62 |
| 44[2] | 1.16 | 1.05 | 78 |
| 44[1] | 11.6 | 2.04 | 77 |
| 44[1] | 2.9 | 0.5 | 56 |
| 45[1] | | | NA |
| 46[1] | | | NA |
| 47[1] | | | NA |
| 48[1] | | | NA |
| 49[1] | | | NA |
| 50[1] | 6.2 | 1.09 | 81 |
| 50[1] | 6.2 | 0.54 | 92 |

| Example No. | MED | RED | MA% |
|---|---|---|---|
| 51[2] | 1.28 | 1.16 | 173 |
| 51[1] | 6.4 | 1.13 | 112 |
| 52[1] | 1.5 | 0.13 | 121 |
| 52[1] | 12.3 | 1.08 | 85 |
| 52[1] | 1.5 | 0.26 | 136 |
| 52[1] | 3 | 0.53 | 86 |
| 52[1] | 6.2 | 0.54 | 84 |
| 53[1] | 11.9 | 2.1 | 57 |
| 53[1] | 5.9 | 0.52 | 99 |
| 54[1] | 12.3 | 1.08 | 102 |
| 54[1] | 12.3 | 4.3 | 92 |
| 55[1] | | | NA |
| 56[1] | | | NA |
| 58[1] | | | NA |
| 62[1] | | | NA |
| 64[2] | 6.2 | 2.73 | 141 |
| 64[2] | 12.4 | 1.09 | 49 |
| 64[2] | 6.2 | 1.10 | 70 |
| 64[2] | 6.2 | 5.46 | 77 |
| 64[2] | 24.9 | 8.8 | 119 |
| 65[2] | 11.3 | 5 | 100 |
| 65[2] | 22.5 | 3.96 | 71 |
| 65[2] | 11.3 | 9.9 | 38 |
| 66[1] | 12 | 1.06 | 73 |
| 66[1] | 6 | 0.53 | 73 |
| 66[1] | 24 | 21.8 | 100 |
| 66[1] | 6 | 5.45 | 105 |
| 66[1] | 3 | 1.06 | 118 |
| 67[2] | 5.5 | 0.48 | 116 |
| 67[2] | 5.0 | 5.0 | 118 |
| 67[2] | 22.2 | 20.2 | 74 |
| 67[1] | 11.1 | 3.9 | 111 |
| 67[1] | 22.2 | 1.9 | 76 |
| 68[1] | 2.4 | 0.2 | 42 |
| 68[1] | | | NA |
| 68[1] | 11.8 | 10.7 | 33 |
| 68[1] | 47.4 | 16.7 | 39 |
| 68[1] | | | NA |
| 69[2] | 46.5 | 20.5 | 48 |
| 69[1] | 46.5 | 16.4 | 39 |
| 69[1] | 23.2 | 8.2 | 47 |
| 70[1] | | | NA |

| Example No. | MED | RED | MA% |
|---|---|---|---|
| 71[1] | | | NA |
| 73[2] | 11.0 | 0.97 | 27 |
| 77[1] | 40.8 | 4.6 | 60 (n=4) |
| 79[1] | 40.3 | 5.4 | 29 (n=2) |
| 80[1] | | | NA |
| 81[1] | 44.9 | 7.9 | 32 |
| 85[1] | 2.7 | 0.36 | 138 (n=2) |
| 86[1] | 5.6 | 2.0 | 89 |
| 87[1] | 23.2 | 8.3 | 14 (n=2) |
| 89[1] | 2.8 | 0.25 | 102 (n=2) |
| 91[1] | 2.7 | 0.24 | 96 (n=2) |
| 93[1] | 4.9 | 0.43 | 131 (n=2) |
| 96[1] | 8.3 | 0.73 | 93 (n=3) |
| 98[1] | 22 | 2.0 | 66 |

[1] Tests using 30% luciferase activity cutoff point (viz., compound tested must have 30% greater light emission than control compound of Example 4)

[2] Tests using 15% luciferase activity cutoff point (viz., compound tested must have 15% greater light emission than control compound of Example 4)

MED is minimum effective dose

RED is relative effective dose (compared to compound of Example 4)

MA is maximum activity (%) over control

NA is not active

number of samples in test is one (n=1), unless otherwise indicated

The following section describes animal tests which illustrate the cholesterol lowering efficacy of the compounds and method of this invention.

EXAMPLE T-2

*In vivo* testing of the compounds of the invention was done as described below:

Protocol:

Syrian Golden hamsters were fed a cholesterol test diet made of 10% coconut oil and 0.12% cholesterol (by weight) in Purina 5001 Rodent Chow to induce hypercholesteriolemia.

After two weeks on the cholesterol test diet the hamsters were bled from the orbital sinium under light $CO_2$ anesthesia. Serum was prepared and analyzed for cholesterol using a commercial test kit (Cholesterol High Performance, Single Vial™, product of Boehringer Mannheim Corp., Indianapolis, IN, USA). The hamsters were separated into groups of six animals, such that each group had approximately the same mean serum cholesterol levels. Selected compounds of the invention were incorporated into the Cholesterol Test Diet at 0.2% (w/w) and fed to the hamsters for one week. A control group of hamsters continued on the Cholesterol Test Diet during the same one-week period. The dose of test compounds of the invention was equivalent to 100 mg/kg/day based on the weight of the hamsters and their diet consumption. Upon completion of the test period, the animals were bled and serum cholesterol determined as above.

Test Results:

Mean serum cholesterol values from the test hamsters were compared to values from the control group and tested for significance at the ≦.05 level using Dunnett's test. The results are displayed in Table I below:

**TABLE 1**

| Test Compound | Serum Cholesterol (mg/dL Mean | ± SEM | Percent Lowering of Cholesterol from Control Value* Mean | ± SEM |
|---|---|---|---|---|
| | 337 | 11 | 0.0 | 3.1 |
| (1) | 163 | 9 | 51.8 | 2.7** |
| (2) | 171 | 5 | 49.2 | 1.6** |
| (3) | 181 | 15 | 46.3 | 4.4** |
| (4) | 198 | 11 | 41.4 | 3.4** |
| (5) | 202 | 13 | 40.2 | 3.8** |
| (6) | 209 | 13 | 38.0 | 4.0** |
| (7) | 209 | 15 | 38.0 | 4.4** |
| (8) | 210 | 14 | 37.8 | 4.2** |
| (9) | 222 | 13 | 31.2 | 4.0** |
| (10) | 234 | 12 | 30.7 | 3.4** |
| (11) | 240 | 30 | 28.8 | 8.8** |
| (12) | 245 | 14 | 27.4 | 4.0** |
| (13) | 253 | 24 | 25.1 | 7.0** |
| (14) | 259 | 47 | 23.2 | 5.6 |
| (15) | 261 | 6 | 22.6 | 1.9 |
| (16) | 267 | 22 | 20.8 | 6.4 |
| (17) | 269 | 23 | 20.2 | 6.8 |
| (18) | 309 | 17 | 8.6 | 5.1 |
| (19) | 312 | 23 | 7.5 | 6.7 |
| (20) | 328 | 37 | 2.7 | 11.1 |
| (21) | 330 | 19 | 2.0 | 5.7 |
| (22) | 345 | 28 | -2.4 | 8.2 |
| (23) | 364 | 26 | -7.8 | 7.7 |
| (24) | 422 | 42 | -25.0 | 12.3 |
| (25) | 464 | 22 | -37.4 | 6.4 |

*negative values indicate serum cholesterol increased relative to control.

**differ from control value by Dennett Test at p≤0.05

Compound Identification for Table 2:

4α-alkyl-5-cholestan-3α-ol
[4α,5α]-4-(2-propenyl)cholestan-3α-ol
4α-(4-fluorobenzyl)cholestan-3α-ol
(3α,4α,5α,20β)-4-(2-propenyl)cholestan-3-ol
4α-benzylcholestan-3-one
(2α,3α,5α)-2-(2-propenyl)cholestan-3-ol
4α(4-trifluoromethoxybenzyl) cholestan-3α-ol
[4α(E),5α]-4-92-butenyl)cholestan-3α-ol
(3α,4α,5α,20β)-4-propylcholestan-3-ol
(3α,4α)-17-[4-(methylpentyl)oxy]-4-(2-propenyl)androstan-3-ol
4α-(4-iodobenzyl)cholestan-3α-ol
(3β,4α,5α)-4-(2-butenyl)cholestan-3-ol

(3α,4α)-4-(2-propenyl)cholestane-3,20-diol
(3α,4α,5α)-4-(3,3-dibromo-2-propenyl)cholestan-3-ol
4α-(4-hydroxybenzyl)cholestan-3α-ol
4α-4-(2-propenyl)cholestan-3-one
(3β,4α,5α)-4-(2-propenyl)cholestan-3-ol
(3α,4α)-17-(phenylmethoxy)-4-(2-propenyl)androstan-3-ol
4α-benzylcholestan-3-one
(3α,4α,5α)-4-(2-propenyl)cholestan-3-ol octadecanoate 4-(2-propenyl)cholestan-3-one
(3β,4α,5α,20β)-4-(2-propenyl)cholestan-3-amine
N-[(3α,4α,5α,20β)-4-(2-propenyl)cholestan-3-yl]acetamide 5α-cholestan-3α-ol
(3α,4α,5α,20β)-4-(2-propenyl)cholestan-3-amine

EXAMPLE T-3

Monkey Pilot Study of the Compound of Example 5 for Changing Plasma Cholesterol Levels

Objective:

To study the effect in short term lowering of plasma cholesterol concentrations in African green monkeys.

Method:

Six African green monkeys were given a hypercholesterolemic diet containing 16.4% lard and 0.33% cholesterol for two weeks to establish a baseline cholesterol level. Thereafter, two daily doses of [14α,5α]-4-(2-propenyl)cholestan-3α-ol, the compound of Example 5, were administered in their feed at 25 mg/kg/day total daily dosage. After ten days the drug dose was increased to 50 mg/kg/day for an additional 3 weeks, followed by a washout period when no drug was administered and the hypercholesterolemic diet was maintained. Blood samples were drawn on the days indicated and plasma was prepared for analysis. Cholesterol was determined by standard enzymatic techniques(method of Allain et al., using diagnostics high performance Cholesterol Reagent No. 236691, product of Boehringer Mannheim Co., Indianapolis, Indiana USA).

Results:

Test results as shown in Tables 3(a) and 3(b) below:

Table 3(a)

| Dose[1] / Animal No. | Day -12 none | Day -5 none | Day 0 none | Day 5 25 | Day 8 25 | Day 4 50 |
|---|---|---|---|---|---|---|
| 1 | 270 | 280 | 275 | 268 | 208 | 184 |
| 2 | 363 | 400 | 382 | 411 | 386 | 878 |
| 3 | 182 | 185 | 183 | 175 | 172 | 155 |
| 4 | 197 | 190 | 194 | 182 | 184 | 159 |
| 5 | 208 | 202 | 205 | 189 | 174 | 148 |
| 6 | 254 | 295 | 275 | 252 | 249 | 211 |
| Mean | 246 | 258 | 252 | 244 | 226 | 205 |
| SEM[2] | 27 | 34 | 31 | 36 | 35 | 36 |

Table 3(b)

| Dose[1] / Animal No. | Day 12 | Day 20 | Day 7 Washout | Day 14 Washout | Day 21 Washout |
|---|---|---|---|---|---|
| 1 | 172 | 188 | 209 | 226 | 270 |
| 2 | 276 | 314 | 251 | 311 | 344 |
| 3 | 159 | 143 | 165 | 176 | 193 |
| 4 | 169 | 161 | 192 | 159 | 168 |
| 5 | 157 | 142 | 174 | 173 | 216 |
| 6 | 206 | 206 | 200 | 213 | 246 |
| Mean | 190 | 192 | 199 | 210 | 240 |
| SEM[2] | 19 | 26 | 12 | 23 | 26 |

[1]Dose in mg/kg/day

[2]SEM=Standard Error of the Mean

Conclusions:

The responses in this pilot study showed that [4α,5α]-4-(2-propenyl)cholestan-3α-ol was effective in lowering plasma cholesterol levels in the animal model used.

As noted above, the compounds of the present invention are useful for upregulating LDL receptor synthesis at the chromosomal level and lowering serum cholesterol levels. The term "upregulating" means the presence of a compound of Formula I in a mammal increases the rate at which RNA polymerese can bind to the beginning of the gene that codes for LDL receptors and initiate the synthesis of the mRNA for said LDL receptors. The higher amounts of LDL receptor mRNA leads, in turn, to correspondingly more LDL receptors. It is not clear whether this upregulation occurs by derepressing LDL receptor synthesis, inducing LDL receptor synthesis,

relieving attenuation, removing a ligand responsible for end product repression or by some combination of these means. A further embodiment of the present invention is a method of upregulating LDL receptor synthesis comprising administering to a mammal in need of treatment an LDL receptor synthesis upregulating dose of a compound of Formula I or a pharmaceutically acceptable salt or solvate thereof. Another embodiment of the present invention is a method for lowering serum LDL cholesterol levels comprising administering to a mammal in need of treatment, a serum LDL cholesterol lowering dose of a compound of Formula I or a pharmaceutically acceptable salt or solvate thereof. A further embodiment of the present invention comprises administering to a mammal in need of treatment, an atherosclerosis inhibiting dose of a compound of Formula I or a pharmaceutically acceptable salt or solvate thereof. A still further embodiment of the invention is the use of the compounds of the invention to effect.transfer of cholesterol from HDL particles to LDL particles.

The term "effective amount" as used herein, means an amount of a compound of the present invention which is capable of upregulating LDL receptor synthesis and lowering serum LDL cholesterol levels and/or inhibiting atherosclerosis. The upregulation of LDL receptor synthesis, serum LDL/cholesterol lowering and atherosclerosis inhibiting methods contemplated by the present invention includes both medical therapeutic and/or prophylactic treatment, as appropriate. A specific dose of a compound administered according to this invention to obtain therapeutic and/or prophylactic effects will, of course, be determined by the particular circumstances surrounding the case, including, for example, the compound administered, the route of administration and the condition being treated. A typical daily dose will contain a non-toxic dosage level of from about 0.01 mg/kg to about 50 mg/kg of body weight of an active compound of this invention. Preferred daily doses generally will be from about 0.05 mg/kg to about 20 mg/kg and ideally from about 0.1 mg/kg to about 10 mg/kg.

The compounds can be administered by a variety of routes including oral, rectal, transdermal, subcutaneous, intravenous, intramuscular, and intranasal. The compounds of the present invention are preferably formulated prior to administration. Therefore, another embodiment of the present invention is a pharmaceutical formulation comprising an effective amount of a compound of Formula I or a pharmaceutically acceptable salt thereof and a pharmaceutically acceptable carrier, diluent or excipient therefor.

The active ingredient in such formulations comprises from 0.1% to 99.9% by weight of the formulation. By "pharmaceutically acceptable" it is meant the carrier, diluent or excipient must be compatible with the other ingredients of the formulation and not deleterious to the recipient thereof.

The present pharmaceutical formulations are prepared by known procedures using well known and readily available ingredients. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), ointments containing, for example, up to 10% by weight of the active compound, soft and hard gelatin capsules, suppositories, sterile injectable solutions, sterile packaged powders, and the like.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared according to any method known to the art for the manufactuere of pharmaceutical compositions and such compositions may contain one or more agents selected from the group consisting of sweweetning, flavoring, coloring and preserving agents in order to provide pharmaceutically platable preparations. Tablets containing the active ingredients in admixture with non-toxic pharmaceutically aceceptable excipients which are suitable for the manufacture of tablets. These excipients may be, for example, inert diluents, such as calcium carbonate, sodium carbonate, lactose, calcium phosphate or sodium phosphate; graulating and disintegrating agents, for example, maize, starch, or alginic acid; binding agents, for example starch, gelatin or acacia, and lubricating agents, for example, magnesium stearage, stearic acid, or talc. The tablets may be uncoated or they may be coated by known techniques to delay disintegration and absorption in the gastointestinal tract and thereby provide a sustained action over a longer period.

The compounds of formula (I) may also be administered in the form of suppositories for rectal administration of the drug. These compositions can be prepared by mixing the drug with a suitable non-irritating excipient which is solid at ordinary temperatures but liquid at the rectal temperature. Such materials are cocoa buter and polyethylene glycols.

The following formulation examples are illustrative only and are not intended to limit the scope of the invention in any way. "Active ingredient," of course, means a compound according to Formula I or a pharmaceutically acceptable salt thereof.

Formulation 1

Hard gelatin capsules are prepared using the following ingredients:

| | Quantity (mg/capsule) |
|---|---|
| Active ingredient | 250 |
| Starch, dried | 200 |
| Magnesium stearate | 10 |
| Total | 460 mg |

Formulation 2

A tablet is prepared using the ingredients below:

| | Quantity (mg/capsule) |
|---|---|
| Active ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

Formulation 3

An aerosol solution is prepared containing the following components:

| | Weight |
|---|---|
| Active ingredient | 0.25 |
| Ethanol | 25.75 |
| Propellant 22 (Chlorodifluoromethane) | 70.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | |
|---|---|
| Active ingredient | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinyl-pyrrolidone is mixed with the resultant powder, and the mixture then is passed through a No. 14 mesh U.S. sieve. The granules so produced are dried at 50°C and passed through a No. 18 mesh U.S. Sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| Active ingredient | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| Active ingredient | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, each containing 50 mg of active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| Active ingredient | 100 mg |
| Isotonic saline | 1,000 ml |

The solution of the above ingredients generally is administered intravenously to a subject at a rate of 1 ml per minute.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description, and all changes which come within the meaning and range of equivalency of the chaims are therefore intended to be embodied therein.

**Claims**

1. A compound having the formula (I) or a pharmaceutically acceptable salt thereof;

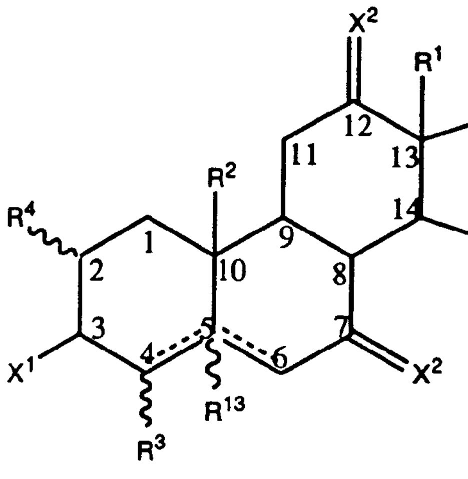

I

wherein:

$R^1$ is a straight chain $C_1$-$C_4$ alkyl or $C_1$-$C_4$ halo alkyl;
$R^2$ is hydrogen, methyl, or halomethyl;
$R^3$ is hydrogen, $C_1$-$C_6$ alkyl, $C_1$-$C_6$ haloalkyl, or a group

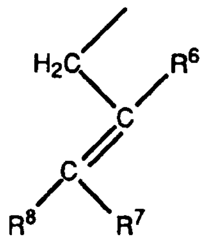

where $R^6$ is hydrogen, halo, $C_1$-$C_4$ alkyl, $C_1$-$C_4$ haloalkyl $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

$R^4$ is hydrogen, $=C-X^4$, $-CH_2CH=C(X^4)_2$, substituted benzyl, or $-(CH_2)_n-X^4$ where n = 1 to 6, and $X^4$ is independently hydrogen, -OH, $C_1$-$C_6$ alkyl, $C_1$-$C_6$ haloalkyl, $C_1$-$C_6$ alkoxy, $C_1$-$C_6$ haloalkoxy, substituted or unsubstituted phenyl, substituted or unsubstituted phenoxy, substituted or unsubstituted benzyloxy,halo -SH, or $-S(C_1$-$C_4$ alkyl), or monocyclic heterocyclic ring;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

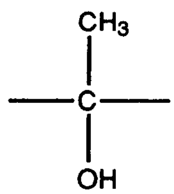 ;

and

Z is a bond, -O-, $-CH_2-$, $-CH(CH_3)-$, $-CH(CH_2CH_3)-$, -CH(halo)-, $-C(halo)_2-$, or

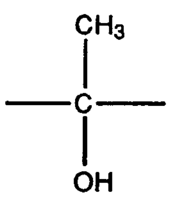 ;

provided that only one of A and Z are -O-, $-CH(CH_3)-$, $-CH(CH_2CH_3)-$,-CH(halo)-, $-C(halo)_2-$, or

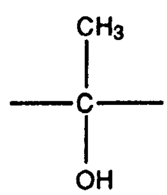 ;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from

a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl;

$R^{13}$ is hydrogen, provided the steroid nucelus is saturated, or $R^{13}$ is absent when the nucleus is unsaturated at the 4,5 or 5,6 position;

$X^1$ is hydroxy, acyloxy, amino, acetamido, substituted amino, mercapto, =O, or ($C_1$-$C_4$ alkoxy)carbonyloxy; and

each $X^2$ is independently oxygen; hydrogen, hydrogen; hydrogen, hydroxy; hydrogen, mercapto; halo, hydrogen; or halo, halo.

2. The compound as claimed in claim 1 or a pharmaceutically acceptable salt thereof wherein;

$R^1$ is a straight chain $C_1$-$C_4$ alkyl;

$R^2$ is hydrogen or methyl;

$X^1$ is hydroxy, amino, mercapto, =O, acetamido, or ($C_1$-$C_4$ alkoxy)carbonyloxy;

each $X^2$ is independently oxygen; hydrogen, hydrogen; hydrogen, hydroxy; or hydrogen, mercapto;

$R^3$ is hydrogen, $C_1$-$C_6$ alkyl or a group

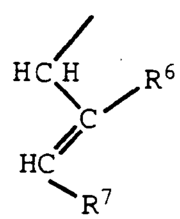

$R^6$ is hydrogen, $C_1$-$C_4$ alkyl or $C_2$-$C_4$ alkenyl;

$R^7$ is hydrogen or methyl; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^4$ is hydrogen or $=CH-X^4$ where $X^4$ is hydrogen, OH, $C_1$-$C_6$ alkyl, $C_1$-$C_6$ alkoxy, substituted or unsubstituted phenyl, substituted or unsubstituted phenoxy, substituted or unsubstituted benzyloxy or a nitrogen containing heterocyclic ring.

$R^5$ is a group

$$-A-Z-R^{10}-X^3$$

where

A is a bond, -O-, $-CH_2$-, $-CH(CH_3)$-, or $-CH(CH_2CH_3)$-;

Z is a bond, -O-, $-CH_2$-, $-CH(CH_3)$-, or $-CH(CH_2CH_3)$-;

$R^{10}$ is unsubstituted or substituted $C_1$-$C_{12}$ alkyl where the substituents are 1 or 2 of the same or different hydroxy, amino, mercapto, halo or two adjacent carbon atoms may each be bonded to the same oxygen atom to afford an epoxide; unsubstituted or substituted $C_2$-$C_{12}$ alkenyl where the substituents are 1 or 2 of the same or different hydroxy, amino, mercapto or halo;

$X^3$ is hydrogen, halo, OH, $-CF_3$, -CN, $C_2$-$C_6$ alkenyl, $-OCF_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl, unsubstituted or substituted phenyl, unsubstituted or substituted phenoxy or unsubstituted or substituted benzyloxy; and pharmaceutically acceptable salts thereof; provided that only one of A and Z are -O-, -C(O)-, $-CH(CH_3)$- or $-CH(CH_2CH_3)$-.

3. A compound as claimed in claim 1 or its pharmaceutically acceptable salt represented by having the formula IV

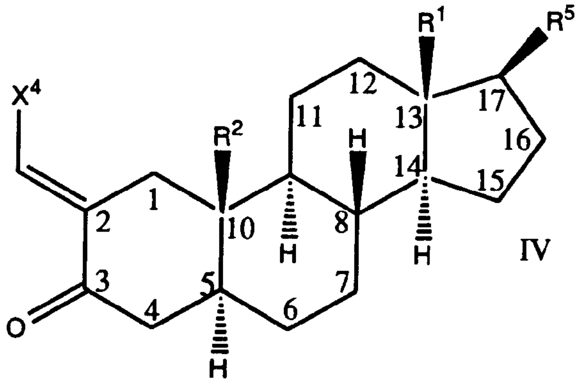

IV

wherein:

$R^1$ is a straight chain $C_1$-$C_4$ alkyl or $C_1$-$C_4$ halo alkyl;

$R^2$ is hydrogen, methyl, or halomethyl;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

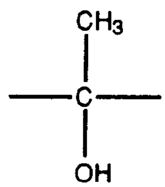

;

and

Z is a bond, -O-, $-CH_2-$, $-CH(CH_3)-$, $-CH(CH_2CH_3)-$, -CH(halo)-, $-C(halo)_2-$, or

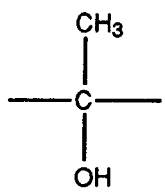

;

provided that only one of A and Z are -O-, $-CH(CH_3)-$, $-CH(CH_2CH_3)-$,-CH(halo)-, $-C(halo)_2$ -, or

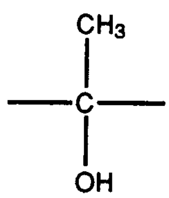

;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$- $C_{12}$ alkene,

where the substituents of (i) and (ii) are one or more of the same or different hydroxy, $-CH_2N(alkyl)_2$,

substituted amino, amino, mercapto, $-S(C_1-C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, $-S(C_1-C_6$ alkyl), $-CF_3$, -CN, $C_2-C_6$ alkenyl, $-OC(F)_3$, $C_1-C_4$ alkoxy, $-C(O)C_1-C_4$ alkyl, $-C(O)(C_2-C_6$ alkenyl) -CHO, -COOH, $-COO(C_1-C_4$ alkyl), $-NR^{11}R^{12}$, $-C(O)NR^{11}R^{12}$ where $R^{11}$ and $R^{12}$ are independently hydrogen or $C_1-C_4$ alkyl;

$X^4$ is hydrogen, -OH, $C_1-C_6$ alkoxy, substituted or unsubstituted phenoxy, substituted or unsubstituted benzyloxy or $-NR^8R^9$ where $R^8$ and $R^9$ are independently hydrogen or $C_1-C_4$ alkyl or combine with the nitrogen atom to which they are attached to form a substituted or unsubstituted nitrogen containing heterocyclic ring.

**4.** A compound as claimed in claim 1 having the formula (II) or a pharmaceutically acceptable salt thereof;

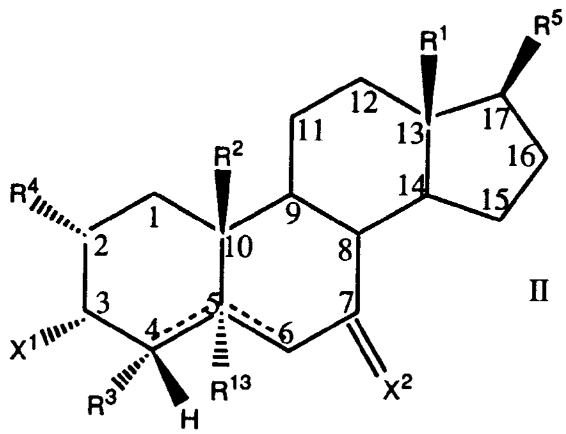

II

wherein:

$R^1$ is a straight chain $C_1-C_4$ alkyl or $C_1-C_4$ halo alkyl;

$R^2$ is hydrogen, methyl, or halomethyl;

$R^3$ is hydrogen, $C_1-C_6$ alkyl, $C_1-C_6$ haloalkyl, or a group

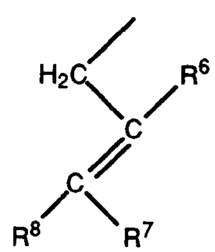

where, $R^6$ is hydrogen, halo, $C_1-C_4$ alkyl, $C_1-C_4$ haloalkyl, $C_2-C_4$ alkenyl, or $C_2-C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5-C_6$ cycloalkenyl, substituted or unsubstituted $C_5-C_6$ cycloalkadienyl, substituted phenyl or unsubstituted or substituted heterocyclic ring;

$R^8$ is hydrogen, methyl, halomethyl, or halo;

$R^4$ is hydrogen, $-CH_2CH=C(X^4)_2$, substituted benzyl, or $-(CH_2)_n-X^4$ where n = 1 to 6, and $X^4$ is independently hydrogen, -OH, $C_1-C_6$ alkyl, $C_1-C_6$ haloalkyl, $C_1-C_6$ alkoxy, $C_1-C_6$ haloalkoxy, substituted or unsubstituted phenyl, substituted or unsubstituted phenoxy, substituted or unsubstituted benzyloxy, -OH, -SH, or $-S(C_1-C_4$ alkyl),or monocyclic heterocyclic ring;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

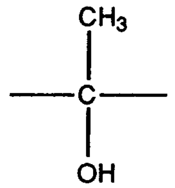 ;

and

Z is a bond, -O-, $-CH_2-$, $-CH(CH_3)-$, $-CH(CH_2CH_3)-$, -CH(halo)-, $-C(halo)_2-$, or

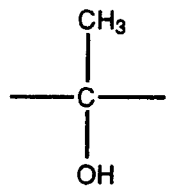 ;

provided that only one of A and Z are -O-, $-CH(CH_3)-$, $-CH(CH_2CH_3)-$,-CH(halo)-, $-C(halo)_2-$, or

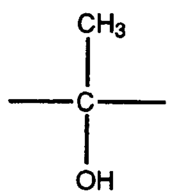 ;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy. halo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl;

$R^{13}$ is hydrogen, provided the steroid nucelus is saturated, or $R^{13}$ is vacant when the nucleus is unsaturated at the 4,5 or 5,6 position;

$X^1$ is hydroxy, acyloxy, amino, acetamido, substituted amino, mercapto, or ($C_1$-$C_4$ alkoxy)carbonyloxy; and

$X^2$ is independently oxygen; hydrogen, hydrogen; hydrogen, hydroxy; hydrogen, mercapto; halo, hydrogen; or halo, halo.

**5.** A compound as claimed in claim 1 and its pharmaceutically acceptable salts, wherein said compound is selected from the group consisting of:

[4α(E),5α]-4-(2-butenyl)cholestan-3αol,
[4α,5α]-4-(2-propenyl)cholestan-3α-ol ,
[4α(E),5α]-4-(2-butenyl)-25-hydroxycholestan-3α- ol,
[4α,5α]-4-butylcholestan-3α-ol,
[4α(E),5α]-4-(2-butenyl)-3α aminocholaetane,
[4α(E),5α]-4-(2-butenyl)-3α acetamidocholestane,

[4α(E),5α]-4-(2-butenyl)-3β acetamidocholestane, 4α-(4-fluorobenzyl)cholestan-3α-ol,
4α-(4-bromobenzyl)cholestan-3α-ol,
4α-(4-iodobenzyl)cholestan-3α-ol,
4α-(4-trifluoromethylbenzyl)cholestan-3α-ol,
4α-(4-dichlorobenzyl)cholestan-3α-ol,
4α-(4-cyanobenzyl)cholestan-3α-ol,
4α-(4-methoxycarbonylbenzyl)cholestan-3α-ol,
4α-(4-trifluoromethoxybenzyl)cholestan-3α-ol,
4α-(4-chlorobenzyl)cholestan-3α-ol,
4α-(4-benzyloxybenzyl)cholestan-3α-ol,
4α-(4-hydroxymethylbenzyl)cholestan-3α-ol,
4α-(4-carboxybenzyl)cholestan-3α-ol,
4α-(4-hydroxybenzyl)cholestan-3α-ol,
4α-benzyl-4-cholestan-3α-ol,
4α-(2-propenyl)-5-cholestan-3α-ol,
4α-(2-propenyl)-cholan-24-N,N-dimethylamino-3α-ol,
3α, 12α-dihydroxy-25-azacoprostane,
3α-hydroxy-25-azacoprostane,
3α, 7α-dihydroxy-25-azacoprostane,
3α,7α, 12α-trihydroxy-25-azacoprostane,
3α,7α, 12α-dihydroxy-25-azacoprostane,
(3α,4α, 5α)-17-(pentyloxy)-4-(2-propenyl) androstan-3-ol ,
(3α,4α, 5α)-17-(octyloxy)-4-(2-propenyl) androstan-3-ol ,
(3α,4α)-17-[4-methylpentyl(oxy]-4-(2-propenyl) androstan-3-ol ,
(3α,4α)-17-(3-phenylpropoxy(oxy]-4-(2-propenyl) androstan-3-ol ,
(3α,4α)-17-(phenylmethoxy)-4-(2-propenyl) androstan-3-ol ,
(3α,4α)-17-[(4,4-dimethylpentyl)oxy]-4-(2-propenyl) androstan-3-ol ,
2-(hydroxymethylene)-4α-(2-propenyl)cholestan -3-one,
(2α,3α,5α)-2-(2-propenyl)cholestan-3-ol
3β,4α,5α,20β)-4-(2-propenyl)cholestan-3-ol
N-[(3α,4α,5α 20β)-4-(2-propenyl)cholestan-3-61]acetamide,
(3α,4α,5α,20β)-4-(3,3-difluoro-2-propenyl) cholestan-3-ol
(3α,4α,5α,20β)-4-(2-propenyl)cholestan-3-amine
(3α,4α,5α,20β)-4-propylcholestan-3-ol
(3α,4α)-4-(2-methyl-2-propenyl)cholestan-3-ol
(3α,4α)-4-(2-chloro-2-propenyl)cholestan-3-ol
(3α,4α)-4-(2-bromo-2-propenyl)cholestan-3-ol,
(3α,4α, 24R)-4-(2-propenyl)-24-(ethyl)cholestan-3-ol, and
(3α, 4α, 22E, 24R)-4-(2-propenyl)-24-(ethyl)cholest-22-en-3-ol.

6. A pharmaceutical formulation comprising; a compound as claimed in any one of claims 1 to 7, together with a pharmaceutically acceptable carrier or diluent therefor.

7. A multi-mode pharmaceutical composition comprising:
   (1) a compound as claimed in any one of claims 1 to 7;
   (2) a cholesterol and/or lipid control agent selected from the group consisting of:
      (a) bile acid sequestrants,
      (b) nicotinic acid and its derivatives,
      (c) HMG-CoA reductase inhibitors,
      (d) gemfibrozil and fibric acids,
      (e) probucol,
      (f) raloxifene and its derivatives, and
      (g) mixtures thereof; and
   (3) optionally; diluents, carriers or excipients.

8. A compound of formula (I) as claimed in any one of claims 1 to 7, or a pharmaceutically acceptable salt thereof for use in lowering serum LDL cholesterol.

**9.** A process for preparing a compound having the formula (I) or a pharmaceutically acceptable salt thereof;

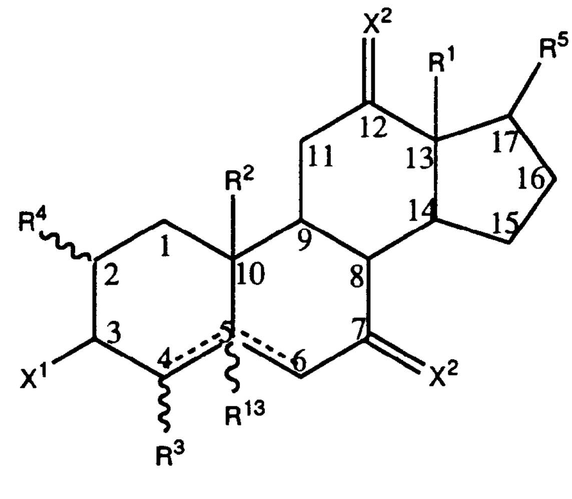

I

wherein:

$R^1$ is a straight chain $C_1$-$C_4$ alkyl or $C_1$-$C_4$ halo alkyl;

$R^2$ is hydrogen, methyl, or halomethyl;

$R^3$ is hydrogen, $C_1$-$C_6$ alkyl, $C_1$-$C_6$ haloalkyl, or a group

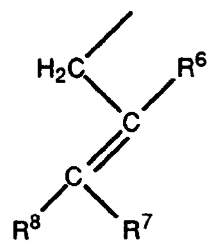

where $R^6$ is hydrogen, halo, $C_1$-$C_4$ alkyl, $C_1$-$C_4$ haloalkyl, $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

$R^4$ is hydrogen, $=C-X^4$, $-CH_2CH=C(X^4)_2$, substituted benzyl, or $-(CH_2)_n-X^4$ where n = 1 to 6, and $X^4$ is independently hydrogen, -OH, $C_1$-$C_6$ alkyl, $C_1$-$C_6$ haloalkyl, $C_1$-$C_6$ alkoxy, $C_1$-$C_6$ haloalkoxy; substituted or unsubstituted phenyl, substituted or unsubstituted phenoxy, substituted or unsubstituted benzyloxy,halo -SH, or -S($C_1$-$C_4$ alkyl), or monocyclic heterocyclic ring;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

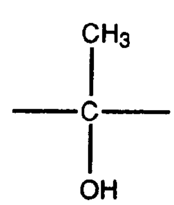 ;

and

Z is a bond, -O-, $-CH_2-$, $-CH(CH_3)-$, $-CH(CH_2CH_3)-$, -CH(halo)-, $-C(halo)_2-$, or

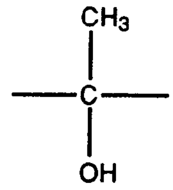 ;

provided that only one of A and Z are -O-, $-CH(CH_3)-$, $-CH(CH_2CH_3)-$,-CH(halo)-, $-C(halo)_2-$, or

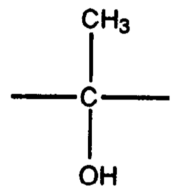 ;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl;

$R^{13}$ is hydrogen, provided the steroid nucelus is saturated, or $R^{13}$ is absent when the nucleus is unsaturated at the 4,5 or 5,6 position;

$X^1$ is hydroxy, acyloxy, amino, acetamido, substituted amino, mercapto, =O, or ($C_1$-$C_4$ alkoxy)carbonyloxy; and

each $X^2$ is independently oxygen; hydrogen, hydrogen; hydrogen, hydroxy; hydrogen, mercapto; halo, hydrogen; or halo, halo.

which process comprises sequential steps (i), (ii) and (iii), wherein;
step (i) is the reaction of a 4-cholesten-3-one with a secondary amine,
step (ii) is alkylation and hydrolysis of the reaction product of step (i), and
step (iii) is reduction of the reaction product of step (ii).

**10.** A process for preparing a compound having the formula (III) or pharmaceutically acceptable salt thereof;

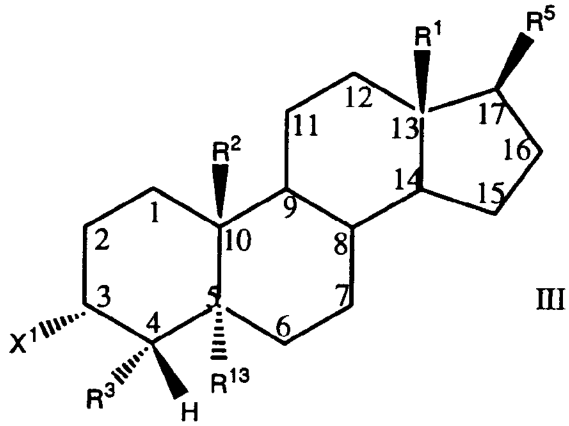

III

wherein;

$R^1$ is a straight chain $C_1$-$C_4$ alkyl or $C_1$-$C_4$ halo alkyl;

$R^2$ is hydrogen, methyl, or halomethyl;

$R^3$ is hydrogen, $C_1$-$C_6$ alkyl, $C_1$-$C_6$ haloalkyl, a group

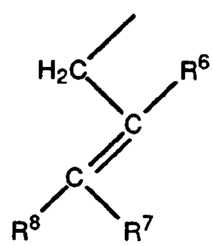

$R^6$ is hydrogen, halo, $C_1$-$C_4$ alkyl, $C_1$-$C_4$ haloalkyl, $C_2$-$C_4$ alkenyl, or $C_2$-$C_4$ haloalkenyl;

$R^7$ is hydrogen, methyl, halomethyl, or halo; or

$R^6$ and $R^7$ are combined with the carbon atoms to which they are attached to form a substituted or unsubstituted $C_5$-$C_6$ cycloalkenyl, substituted or unsubstituted $C_5$-$C_6$ cycloalkadienyl, substituted phenyl or unsubstituted or substituted heterocyclic ring;

$R^8$ is hydrogen, methyl, halomethyl, or halo;

$R^5$ is the group

$$-A-Z-R^{10}-X^3$$

where

A is a bond, -O-, $-CH_2-$, $-CH(CH_3)-$, $-CH(CH_2CH_3)-$, -CH(halo)-, $-C(halo)_2-$, or

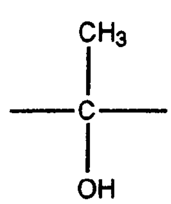 ;

and

Z is a bond, -O-, $-CH_2-$, $-CH(CH_3)-$, $-CH(CH_2CH_3)-$, -CH(halo)-, $-C(halo)_2-$, or

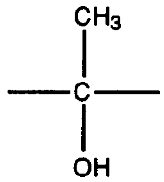 ;

provided that only one of A and Z are -O-, $-CH(CH_3)-$, $-CH(CH_2CH_3)-$,-CH(halo)-, $-C(halo)_2-$, or

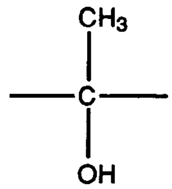 ;

$R^{10}$ is

(i) a divalent unsubstituted or substituted, branched or unbranched radical derived from a $C_1$-$C_{12}$ alkane, or

(ii) a divalent unsubstituted or substituted, branched or unbranched, unsaturated radical derived from a $C_2$-$C_{12}$ alkene,

where the substituents of (i) and (ii) are one or more of the same or different hydroxy, $-CH_2N(alkyl)_2$, acetamido, substituted amino, amino, mercapto, -S($C_1$-$C_6$ alkyl), halo, or two adjacent carbon atoms may each be bonded to the same oxygen atom to afford an epoxide;

$X^3$ is hydrogen, unsubstituted or substituted phenyl, unsubstituted or substituted phenoxy or unsubstituted or substituted benzyloxy, halo, haloalkyl, OH, -SH, -S($C_1$-$C_6$ alkyl), $-CF_3$, -CN, $C_2$-$C_6$ alkenyl, $-OC(F)_3$, $C_1$-$C_4$ alkoxy, -C(O)$C_1$-$C_4$ alkyl, -C(O)($C_2$-$C_6$ alkenyl) -CHO, -COOH, -COO($C_1$-$C_4$ alkyl), $-NR^{11}R^{12}$, $-C(O)NR^{11}R^{12}$ where $R^{11}$ and $R^{12}$ are independently hydrogen or $C_1$-$C_4$ alkyl;

$X^1$ is hydroxy, and

$R^{13}$ is hydrogen,

which process comprises sequential steps (i) and (ii) wherein;

step (i) is a reductive alkylating of a 4-cholesten-3-one,as shown by the reaction sequence IX, and;

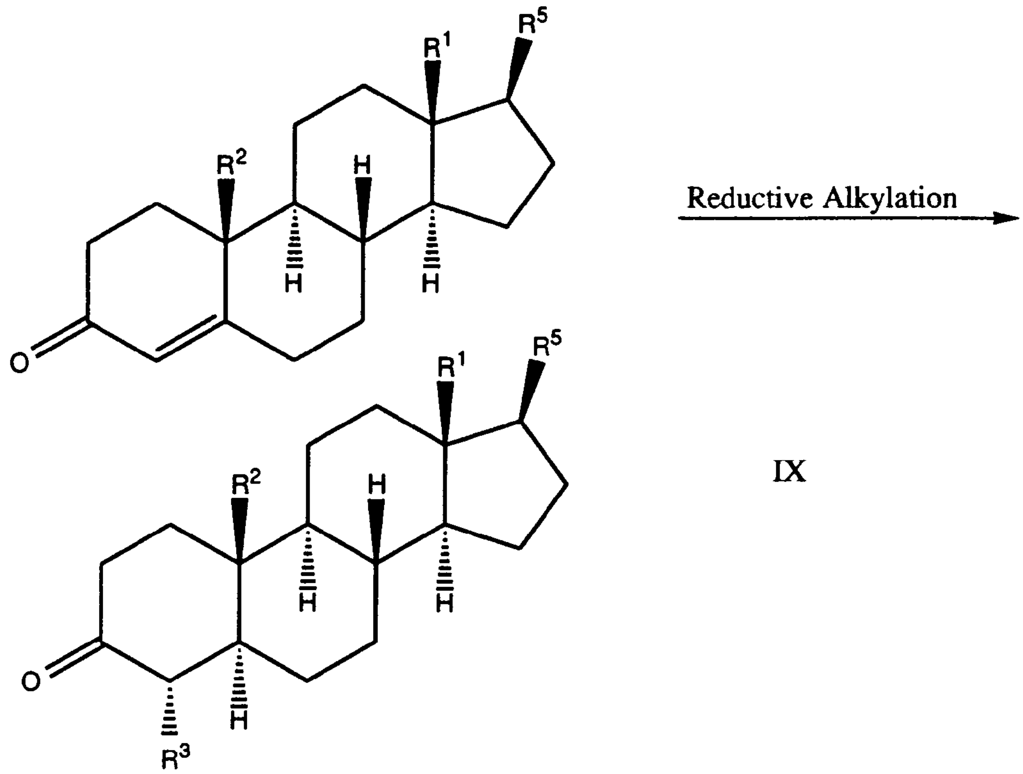


IX

step (ii) is a reduction of the reaction product of step (i) as shown by the reaction sequence X.

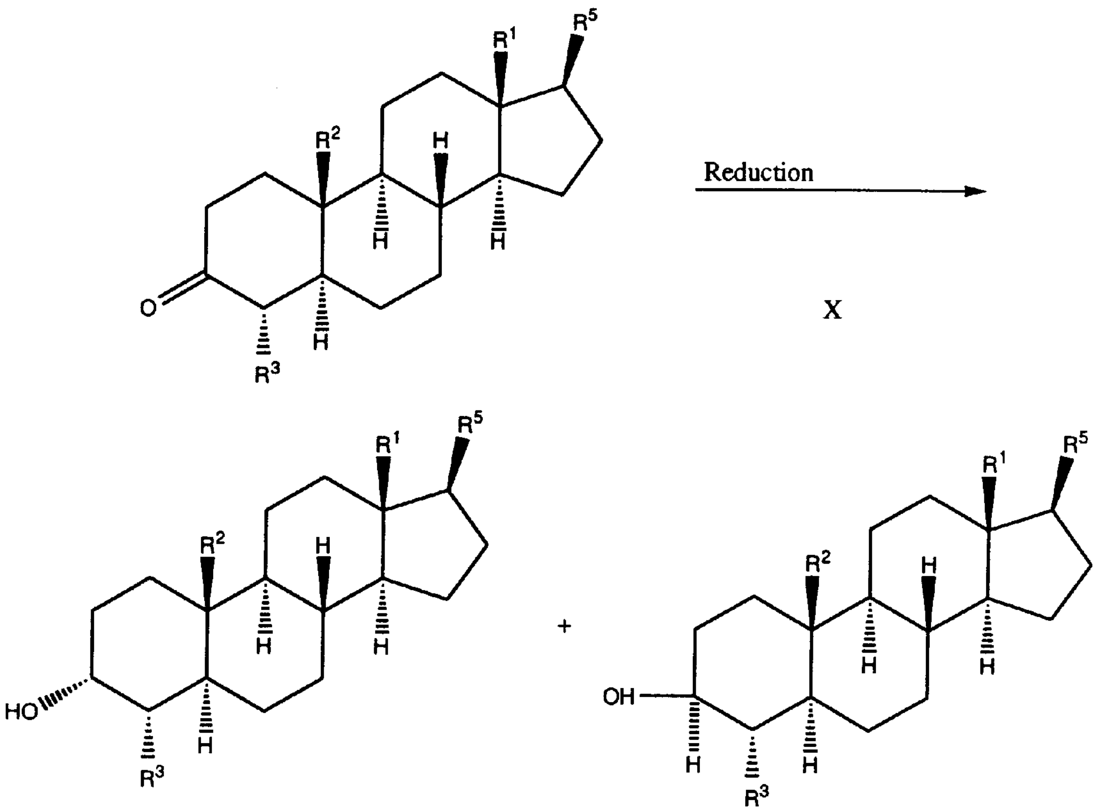
Reduction
X
+